(19) Europäisches Patentamt
European Patent Office
Office européen des brevets

(11) EP 1 745 783 A1

(12) # EUROPEAN PATENT APPLICATION

(43) Date of publication:
**24.01.2007 Bulletin 2007/04**

(21) Application number: **05384028.6**

(22) Date of filing: **15.07.2005**

(51) Int Cl.:
*A61K 31/4155* (2006.01) *A61K 31/496* (2006.01)
*A61K 31/454* (2006.01)

(84) Designated Contracting States:
**AT BE BG CH CY CZ DE DK EE ES FI FR GB GR HU IE IS IT LI LT LU LV MC NL PL PT RO SE SI SK TR**
Designated Extension States:
**AL BA HR MK YU**

(71) Applicant: **Laboratorios del Dr. Esteve S.A.**
**08041 Barcelona (ES)**

(72) Inventor: **Buschmann, Helmut, Heinrich**
**08960 Sant Just Desvern (Barcelona) (ES)**

(54) **Substituted pyrazoline compounds, their preparation and use as medicaments**

(57) The present invention relates to substituted pyrazoline compounds, methods for their preparation, medicaments comprising these compounds as well as their use for the preparation of a medicament for the treatment of humans and animals.

**Figure 1**

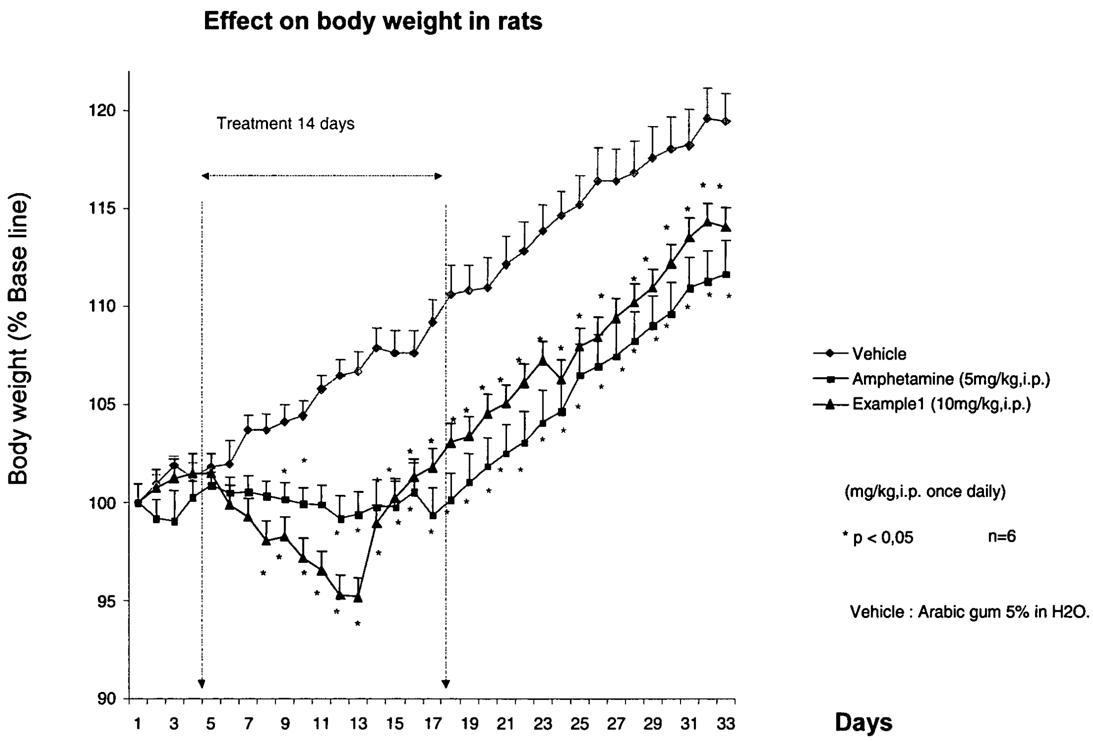


EP 1 745 783 A1

Printed by Jouve, 75001 PARIS (FR)

## Description

The present invention relates to substituted pyrazoline compounds, methods for their preparation, medicaments comprising these compounds as well as their use for the preparation of a medicament for the treatment of humans and animals.

Cannabinoids are compounds, which are derived from the cannabis sativa plant which is commonly known as marijuana. The most active chemical compound of the naturally occurring cannabinoids is tetrahydrocannabinol (THC), particularly $\Delta^9$-THC.

These naturally occuring cannabinoids as well as their synthetic analogues promote their physiological effects via binding to specific G-coupled receptors, the so-called cannabinoid-receptors.

At present, two distinct types of receptors that bind both the naturally occurring and synthetic cannabinoids have been identified and cloned. These receptors, which are designated $CB_1$ and $CB_2$ are involved in a variety of physiological or pathophysiological processes in humans and animals, e.g. processes related to the central nervous system, immune system, cardiovascular system, endocrinous system, respiratory system, the gastrointestinal tract or to reproduction, as described for example, in Hollister, Pharm. Rev. 38, 1986, 1-20; Reny and Singha, Prog. Drug. Res., 36, 71-114, 1991; Consroe and Sandyk, in Marijuana/Cannabinoids, Neurobiology and Neurophysiology, 459, Murphy L. and Barthe A. Eds., CRC Press, 1992.

Therefore, compounds, which have a high binding affinity for these cannabinoid receptors and which are suitable for modulating these receptors are useful in the prevention and/or treatment of cannabinoid-receptor related disorders.

In particular, the $CB_1$-Receptor is involved in many different food-intake related disorders such as bulimia or obesity, including obesity associated with type II diabetes (non-insulin-dependent diabetes) and thus, compounds suitable for regulating this receptor may be used in the prophylaxis and/or treatment of these disorders.

Thus, it was an object of the present invention to provide novel compounds for use as active substances in medicaments. In particular, these active substances should be suitable for the modulation of Cannabinoid receptors, more particularly for the modulation of Cannabinoid 1 ($CB_1$) receptors.

Said object was achieved by providing the substituted pyrazoline compounds of general formula I given below, their stereoisomers, corresponding salts and corresponding solvates thereof.

It has been found that these compounds have a high affinity for cannabinoid receptors, particularly for the $CB_1$-receptor, and that they act as modulators e.g. antagonists, inverse agonists or agonists on these receptors. They are therefore suitable for the prophylaxis and/or treatment of various disorders related to the central nervous system, the immune system, the cardiovascular system, the endocrinous system, the respiratory system, the gastrointestinal tract or reproduction in humans and/or animals, preferably humans including infants, children and grown-ups.

Thus, in one of its aspects the present invention relates to substituted pyrazoline compounds of general formula I,

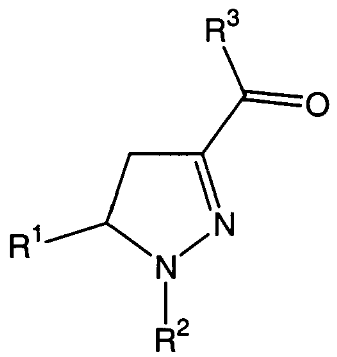

I

wherein

$R^1$ represents an optionally at least mono-substituted phenyl group,

$R^2$ represents an optionally at least mono-substituted phenyl group,

$R^3$ represents a saturated or unsaturated, optionally at least mono-substituted, optionally at least one heteroatom as ring member containing cycloaliphatic group, which may be condensed with an optionally at least mono-substituted mono- or polycyclic ring system, or $R^3$ represents an optionally at least mono-substituted aryl or heteroaryl group, which may be condensed with an optionally at least mono-substituted mono- or polycyclic ring system, or $R^3$ represents an —$NR^4R^5$-moiety,

$R^4$ and $R^5$, identical or different, represent a hydrogen atom, an unbranched or branched, saturated or unsaturated,

optionally at least mono-substituted aliphatic radical, a saturated or unsaturated, optionally at least mono-substituted, optionally at least one heteroatom as ring member containing cycloaliphatic group, which may be condensed with an optionally at least mono-substituted mono- or polycyclic ring system, or an optionally at least mono-substituted aryl or heteroaryl group, which may be condensed with an optionally at least mono-substituted mono- or polycyclic ring system and/or bonded via a linear or branched alkylene group, an —$SO_2$-$R^6$-moiety, or an -$NR^7R^8$-moiety,
$R^6$ represents a linear or branched, saturated or unsaturated, optionally at least mono-substituted aliphatic group, a saturated or unsaturated, optionally at least mono-substituted, optionally at least one heteroatom as ring member containing cycloaliphatic group, which may be condensed with a mono- or polycyclic ring-system, or an optionally at least mono-substituted aryl or heteroaryl group, which may be condensed with a mono- or polycyclic ring system and/or bonded via a linear or branched alkylene group,
$R^7$ and $R^8$, identical or different, represent a hydrogen atom, an unbranched or branched, saturated or unsaturated, optionally at least mono-substituted aliphatic radical, a saturated or unsaturated, optionally at least mono-substituted, optionally at least one heteroatom as ring member containing cycloaliphatic group, which may be condensed with an optionally at least mono-substituted mono- or polycyclic ring system, or an optionally at least mono-substituted aryl or heteroaryl group, which may be condensed with an optionally at least mono-substituted mono- or polycyclic ring system and/or bonded via a linear or branched alkylene group,
optionally in form of one of the stereoisomers, preferably enantiomers or diastereomers, a racemate or in form of a mixture of at least two of the stereoisomers, preferably enantiomers and/or diastereomers, in any mixing ratio, or a corresponding N-oxide thereof, or a corresponding salt thereof, or a corresponding solvate thereof.

Particularly preferably the following provisos (disclaimers) apply for the pyrazoline compounds of general formula I given above:

that $R^4$ and $R^5$ do not both represent a hydrogen atom, and

that if one of the residues $R^4$ and $R^5$ represents a hydrogen atom or an alkyl group, which is optionally at least mono-substituted with an alkoxy group, an alkoxyalkoxy group, a halogen atom or a phenyl group, the other one of these residues $R^4$ and $R^5$ does not represent a pyrid-2-yl group, which is optionally mono-substituted in the 5-position, a pyrid-5-yl group, which is optionally mono-substituted in the 2-position, a pyrimid-5-yl group, which is optionally mono-substituted in the 2-position, a pyridaz-3-yl group, which is optionally mono-substituted in the 6-position, a pyrazin-5-yl group, which is optionally mono-substituted in the 2-position, a thien-2-yl group, which is optionally mono-substituted in the 5 position, a thien-2-yl group, which is optionally at least mono-substituted in the 4-position, a benzyl group, which is optionally mono-substituted in the 4-position of the ring, a phenethyl group, which is optionally mono-substituted in the 4-position of the ring, an optionally mono-, di- or tri-substituted phenyl group, a di-substituted phenyl group, wherein the two substituents together form an -$OCH_2O$-, -$OCH_2CH_2O$- or -$CH_2CH_2O$- chain, which is optionally substituted with one or more halogen atoms or one or two methyl groups, an -NH-phenyl-moiety, wherein the phenyl group may be mono-substituted in the 4-position, and

that if one of the residues $R^4$ and $R^5$ represents an alkynyl group, the other one of these residues $R^4$ and $R^5$ does not represent a phenyl group, which is optionally substituted in the 4-position, and

that if one of the residues $R^4$ and $R^5$ represents a hydrogen atom or a linear or branched, saturated or unsaturated, unsubstituted or substituted aliphatic radical, the other one of these residues $R^4$ and $R^5$ does not represent an unsubstituted or substituted thiazole group or an unsubstituted or substituted [1,3,4]thiadiazole group.

A mono- or polycyclic ring-system according to the present invention means a mono-or polycyclic hydrocarbon ring-system that may be saturated, unsaturated or aromatic. If the ring system is polycyclic, each of its different rings may show a different degree of saturation, i.e. it may be saturated, unsaturated or aromatic. Optionally each of the rings of the mono- or polycyclic ring system may contain one or more, e.g. 1, 2 or 3, heteroatoms as ring members, which may be identical or different and which can preferably be selected from the group consisting of N, O, S and P, more preferably be selected from the group consisting of N, O and S. Preferably the polycyclic ring-system may comprise two rings that are condensed. The rings of the mono- or polycyclic ring-sytem are preferably 5- or 6-membered.

The term "condensed" according to the present invention means that a ring or ring-system is attached to another ring or ring-system, whereby the terms "annulated" or "annelated" are also used by those skilled in the art to designate this kind of attachment.

If one or more of the residues $R^3$-$R^8$ represents or comprises a saturated or unsaturated, optionally at least one heteroatom as ring member containing cycloaliphatic group, which is substituted by one or more, e.g. 1, 2, 3 or 4, substituents, unless defined otherwise, each of the substituents may be independently selected from the group consisting of hydroxy, fluorine, chlorine, bromine, branched or unbranched $C_{1-6}$-alkoxy, branched or unbranched $C_{1-6}$-alkyl,

branched or unbranched $C_{1-4}$-perfluoroalkoxy, branched or unbranched $C_{1-4}$-perfluoroalkyl, oxo, amino, carboxy, amido, cyano, nitro, $-SO_2NH_2$, $-CO-C_{1-4}$-alkyl, - $SO-C_{1-4}$-alkyl, $-SO_2-C_{1-4}$-alkyl, $-NH-SO_2-C_{1-4}$-alkyl , wherein the $C_{1-4}$-alkyl may in each case be branched or unbranched, and a phenyl group, more preferably be selected from the group consisting of hydroxy, F, Cl, Br, methyl, ethyl, methoxy, ethoxy, oxo, $CF_3$ and a phenyl group.

If one or more of the residues $R^3$-$R^8$ represents or comprises a cycloaliphatic group, which contains one or more heteroatoms as ring members, unless defined otherwise, each of these heteroatoms may preferably be selected from the group consisting of of N, O and S. Preferably a cycloaliphatic group may contain 1, 2 or 3 heteratoms independently selected from the group consisting of N, O and S as ring members.

Suitable saturated or unsaturated, optionally at least one heteroatom as ring member containing, optionally at least mono-substituted cycloaliphatic groups may preferably be selected from the group consisting of Cyclopropyl, Cyclobutyl, Cyclopentyl, Cyclohexyl, Cycloheptyl, Cyclooctyl, Cyclopentenyl, Cyclohexenyl, Cycloheptenyl, Cyclooctenyl, Pyrrolidinyl, Piperidinyl, Piperazinyl, homo-Piperazinyl and Morpholinyl.

If one or more of the residues $R^3$-$R^8$ comprises a mono- or polycyclic ring system, which is substituted by one or more, e.g. 1, 2, 3, 4 or 5 substituents, unless defined otherwise, each of the substituents may be independently selected from the group consisting of hydroxy, fluorine, chlorine, bromine, branched or unbranched $C_{1-6}$-alkoxy, branched or unbranched $C_{1-6}$-alkyl, branched or unbranched $C_{1-4}$-perfluoroalkoxy, branched or unbranched $C_{1-4}$-perfluoroalkyl, amino, carboxy, oxo, amido, cyano, nitro, $-SO_2NH_2$, $-CO-C_{1-4}$-alkyl, $-SO-C_{1-4}$-alkyl, $-SO_2-C_{1-4}$-alkyl, $-NH-SO_2-C_{1-4}$-alkyl , wherein the $C_{1-4}$-alkyl may in each case be branched or unbranched, and a phenyl group, more preferably be selected from the group consisting of hydroxy, F, Cl, Br, methyl, ethyl, methoxy, ethoxy, $CF_3$, oxo and a phenyl group.

If one or more of the residues $R^1$-$R^8$ represents or comprises an aryl group, including a phenyl group, which is substituted by one or more, e.g. 1, 2, 3, 4 or 5 substituents, unless defined otherwise, each of the substituents may be independently selected from the group consisting of a halogen atom (e.g. F, Cl, Br, I), a linear or branched $C_{1-6}$-alkyl group, a linear or branched $C_{1-6}$ alcoxy group, a formyl group, a hydroxy group, a trifluoromethyl group, a trifluoromethoxy group, a $-CO-C_{1-6}$-alkyl group, a cyano group, a nitro group, a carboxy group, a $-CO-O-C_{1-6}$-alkyl group, a $—CO-NR^AR^B$-moiety, a $-CO-NH-NR^CR^D$-moiety, an —SH, an $-S-C_{1-6}$-alkyl group, an $-SO-C_{1-6}$-alkyl group, an $-SO_2-C_{1-6}$-alkyl group, a $-C_{1-6}$-alkylene-$S-C_{1-6}$-alkyl group, a $-C_{1-6}$-alkylene-$SO-C_{1-6}$-alkyl group, a $-C_{1-6}$-alkylene-$SO_2-C_{1-6}$-alkyl group, an $—NH_2$-moiety, an NHR'-moiety or an NR'R''-moiety, wherein R' and R'' independently represent a linear or branched $C_{1-6}$-alkyl group, a $C_{1-6}$-alkyl group substituted by one or more hydroxy groups and a $—C_{1-6}$-alkylene-$NR^ER^F$ group,
whereby $R^A$, $R^B$, identical or different, represent hydrogen or a $C_{1-6}$-alkyl group, or $R^A$ and $R^B$ together with the bridging nitrogen atom form a saturated, mono- or bicyclic, 3-10 membered heterocyclic ring system, which may be at least mono-substituted by one or more, identical or different, $C_{1-6}$ alkyl groups and/or which may contain at least one further heteroatom selected from the group consisting of nitrogen, oxygen and sulphur as a ring member,
$R^C$, $R^D$, identical or different, represent a hydrogen atom, a $C_{1-6}$-alkyl group, a $-CO-O-C_{1-6}$-alkyl group, a $C_{3-8}$-cycloalkyl group, a $C_{1-6}$-alkylene-$C_{3-8}$-cydoalkyl group, $C_{1-6}$-alkylene-$O-C_{1-6}$-alkyl group or a $C_{1-6}$-alkyl group substituted with one or more hydroxy groups, or $R^C$, $R^D$ together with the bridging nitrogen atom form a saturated, mono- or bicyclic, 3-10 membered heterocyclic ring system, which may be at least mono-substituted by one or more substituents independently selected from the group consisting of $C_{1-6}$ alkyl group, a $-CO-C_{1-6}$-alkyl group, a $—CO-O-C_{1-6}$-alkyl group, a - $CO-NH-C_{1-6}$-alkyl group, a $-CS-NH-C_{1-6}$-alkyl group, an oxo group, a $C_{1-6}$-alkyl group substituted with one or more hydroxy groups, a $C_{1-6}$-alkylene-$O-C_{1-6}$-alkyl group and a $—CO-NH_2$ group and/or which may contain at least one further heteroatom selected from the group consisting of nitrogen, oxygen and sulphur as a ring member, and
wherein $R^E$, $R^F$, identical or different, represent hydrogen or a $C_{1-6}$-alkyl group, or $R^E$ and $R^F$ together with the bridging nitrogen atom form a saturated, mono- or bicyclic, 3-10 membered heterocyclic ring system, which may be at least mono-substituted by one or more, identical or different $C_{1-6}$ alkyl groups and/or which may contain at least one further heteroatom selected from the group consisting of nitrogen, oxygen and sulphur as a ring member.

Preferred aryl groups, which may optionally be at least mono-substituted, are phenyl and naphthyl.

If one or more of the residues $R^3$-$R^8$ represents or comprises a heteroaryl group, which is substituted by one or more, e.g. 1, 2, 3, 4 or 5 substituents, unless defined otherwise, each of the substituents may be independently selected from the group consisting of a halogen atom (e.g. F, Cl, Br, I), a linear or branched $C_{1-6}$-alkyl group, a linear or branched $C_{1-6}$ alcoxy group, a formyl group, a hydroxy group, a trifluoromethyl group, a trifluoromethoxy group, a-$CO-C_{1-6}$-alkyl group, a cyano group, a carboxy group, a $—CO-O-C_{1-6}$-alkyl group, a $—CO-NR^AR^B$- moiety, a $-CO-NH-NR^CR^D$-moiety, an $-S-C_{1-6}$-alkyl group, an $-SO-C_{1-6}$-alkyl group, an $-SO_2-C_{1-6}$-alkyl group, a $-C_{1-6}$-alkylene-$S-C_{1-6}$-alkyl group, a $-C_{1-6}$-alkylene-$SO-C_{1-6}$-alkyl group, a $-C_{1-6}$-alkylene-$SO_2-C_{1-6}$-alkyl group, a $C_{1-6}$-alkyl group substituted by one or more hydroxy groups and a $—C_{1-6}$-alkylene-$NR^ER^F$ group,
whereby $R^A$, $R^B$, identical or different, represent hydrogen or a $C_{1-6}$-alkyl group, or $R^A$ and $R^B$ together with the bridging nitrogen atom form a saturated, mono- or bicyclic, 3-10 membered heterocyclic ring system, which may be at least mono-substituted by one or more, identical or different, $C_{1-6}$ alkyl groups and/or which may contain at least one further heteroatom selected from the group consisting of nitrogen, oxygen and sulphur as a ring member,
$R^C$, $R^D$, identical or different, represent a hydrogen atom, a $C_{1-6}$-alkyl group, a $-CO-O-C_{1-6}$-alkyl group, a $C_{3-8}$-cycloalkyl

group, a $C_{1-6}$-alkylene-$C_{3-8}$-cycloalkyl group, $C_{1-6}$-alkylene-O-$C_{1-6}$-alkyl group or a $C_{1-6}$-alkyl group substituted with one or more hydroxy groups, or $R^C$, $R^D$ together with the bridging nitrogen atom form a saturated, mono- or bicyclic, 3-10 membered heterocyclic ring system, which may be at least mono-substituted by one or more substituents independently selected from the group consisting of $C_{1-6}$ alkyl group, a -CO-$C_{1-6}$-alkyl group, a —CO-O- $C_{1-6}$-alkyl group, a - CO-NH-$C_{1-6}$-alkyl group, a -CS-NH- $C_{1-6}$-alkyl group, an oxo group, a $C_{1-6}$-alkyl group substituted with one or more hydroxy groups, a $C_{1-6}$-alkylene-O-$C_{1-6}$-alkyl group and a —CO-$NH_2$ group and/or which may contain at least one further heteroatom selected from the group consisting of nitrogen, oxygen and sulphur as a ring member, and

wherein $R^E$, $R^F$, identical or different, represent hydrogen or a $C_{1-6}$-alkyl group, or $R^E$ and $R^F$ together with the bridging nitrogen atom form a saturated, mono- or bicyclic, 3-10 membered heterocyclic ring system, which may be at least mono-substituted by one or more, identical or different $C_{1-6}$ alkyl groups and/or which may contain at least one further heteroatom selected from the group consisting of nitrogen, oxygen and sulphur as a ring member,

The heteroatoms, which are present as ring members in the heteroaryl radical, may, unless defined otherwise, independently be selected from the group consisting of nitrogen, oxygen and sulphur. Preferably a heteroaryl radical may comprise 1, 2 or 3 heteroatoms independently selected from the group consisting of N, O and S as ring members.

Suitable heteroaryl groups, which may optionally be at least mono-substituted, may preferably be selected from the group consisting of thienyl, furyl, pyrrolyl, pyridinyl, imidazolyl, pyrimidinyl, pyrazinyl, indolyl, chinolinyl, isochinolinyl, benzo[1,2,5]-thiodiazolyl, benzo[b]thiophenyl, benzo[b]furanyl, imidazo[2,1-b]thiazolyl, triazolyl, and pyrazolyl, more preferably be selected from the group consisting of thienyl-, benzo[1,2,5]-thiodiazolyl, benzo[b]thiophenyl, imidazo [2,1-b]thiazolyl, triazolyl and pyrazolyl.

If one or more of the residues $R^4$-$R^8$ represents or comprises a linear or branched, saturated or unsaturated aliphatic group such as an alkyl group, which is substituted by one or more, e.g. 1, 2, 3, 4 or 5 substituents, unless defined otherwise, each of the substituents may be independently selected from the group consisting of hydroxy, fluorine, chlorine, bromine, branched or unbranched $C_{1-4}$-alkoxy, branched or unbranched $C_{1-4}$-perfluoroalkoxy, branched or unbranched $C_{1-4}$-perfluoroalkyl, amino, carboxy, amido, cyano, nitro, -$SO_2NH_2$, -CO-$C_{1-4}$-alkyl, -SO-$C_{1-4}$-alkyl, -$SO_2$-$C_{1-4}$-alkyl, -NH-$SO_2$-$C_{1-4}$-alkyl, wherein the $C_{1-4}$-alkyl may in each case be branched or unbranched, and a phenyl group, more preferably be selected from the group consisting of hydroxy, F, Cl, Br, methoxy, ethoxy, $CF_3$ and a phenyl group.

Preferred linear or branched, saturated or unsaturated aliphatic groups, which may be substituted by one or more substituents, may preferably be selected from the group consisting of methyl, ethyl, n-propyl, isopropyl, n-butyl, iso-butyl, sec-butyl, tert-butyl, n-pentyl, n-hexyl, n-heptyl, n-octyl, n-nonyl, n-decyl, vinyl, ethinyl, propenyl, propinyl, butenyl and butinyl.

If any of the residues $R^4$-$R^8$ represents or comprises a linear or branched alkylene group, said alkylene group may preferably be selected from the group consisting of — methylene -($CH_2$)-, ethylene -($CH_2$-$CH_2$)-, n-propylene -($CH_2$-$CH_2$-$CH_2$)- or isopropylene —(-$C(CH_3)_2$)-.

Preferred are substituted pyrazoline compounds of general formula I given above, wherein

$R^1$ represents an optionally at least mono-substituted phenyl group,

$R^2$ represents an optionally at least mono-substituted phenyl group,

$R^3$ represents a saturated or unsaturated, optionally at least mono-substituted, optionally at least one heteroatom as ring member containing cycloaliphatic group, which may be condensed with an optionally at least mono-substituted mono- or polycyclic ring system, or $R^3$ represents an optionally at least mono-substituted aryl or heteroaryl group, which may be condensed with an optionally at least mono-substituted mono- or polycyclic ring system, or $R^3$ represents an -$NR^4R^5$-moiety,

$R^4$ and $R^5$, identical or different, represent a hydrogen atom, an unbranched or branched, saturated or unsaturated, optionally at least mono-substituted aliphatic radical, a saturated or unsaturated, optionally at least mono-substituted, optionally at least one heteroatom as ring member containing cycloaliphatic group, which may be condensed with an optionally at least mono-substituted mono- or polycyclic ring system, or an optionally at least mono-substituted aryl or heteroaryl group, which may be condensed with an optionally at least mono-substituted mono- or polycyclic ring system and/or bonded via a linear or branched alkylene group, an -$SO_2$-$R^6$-moiety, or an -$NR^7R^8$-moiety,

$R^6$ represents a linear or branched, saturated or unsaturated, optionally at least mono-substituted aliphatic group, a saturated or unsaturated, optionally at least mono-substituted, optionally at least one heteroatom as ring member containing cycloaliphatic group, which may be condensed with a mono- or polycyclic ring-system, or an optionally at least mono-substituted aryl or heteroaryl group, which may be condensed with a mono- or polycyclic ring system and/or bonded via a linear or branched alkylene group,

$R^7$ and $R^8$, identical or different, represent a hydrogen atom, an unbranched or branched, saturated or unsaturated, optionally at least mono-substituted aliphatic radical, a saturated or unsaturated, optionally at least mono-substituted, optionally at least one heteroatom as ring member containing cycloaliphatic group, which may be condensed with an optionally at least mono-substituted mono- or polycyclic ring system, or an optionally at least mono-substituted aryl or heteroaryl group, which may be condensed with an optionally at least mono-substituted mono- or polycyclic ring system

and/or bonded via a linear or branched alkylene group,
optionally in form of one of the stereoisomers, preferably enantiomers or diastereomers, a racemate or in form of a mixture of at least two of the stereoisomers, preferably enantiomers and/or diastereomers, in any mixing ratio, or a corresponding N-oxide thereof, or a corresponding salt thereof, or a corresponding solvate thereof,
whereby preferably the following provisos (disclaimers) apply:
that $R^4$ and $R^5$ do not both represent a hydrogen atom, and that if one of the residues $R^4$ and $R^5$ represents a hydrogen atom or a linear or branched, saturated or unsaturated, substituted or unsubstituted aliphatic group, the other one of these residues $R^4$ and $R^5$ does not represent a substituted or unsubstituted pyridyl group, a substituted or unsubstituted pyrimidyl group, a substituted or unsubstituted pyridazyl group, a substituted or unsubstituted pyrazinyl group, a substituted or unsubstituted thienyl group, a substituted or unsubstituted benzyl group, a substituted or unsubstituted phenethyl group, a substituted or unsubstituted phenyl group, a substituted or unsubstituted phenyl group, which is condensed (attached) to at least one, optionally substituted ring or ringsystem, an —NH-phenyl-moiety, wherein the phenyl group may be at least mono-substituted, an unsubstituted or substituted thiazole group, or an unsubstituted or substituted [1,3,4]thiadiazole group.

Preferred are also substituted pyrazoline compounds of general formula I given above, wherein $R^1$ represents a phenyl group, which is optionally substituted by one or more substituents independently selected from the group consisting of a linear or branched $C_{1-6}$-alkyl group, a linear or branched $C_{1-6}$-alkoxy group, a halogen atom, $CH_2F$, $CHF_2$, $CF_3$, CN, OH, $NO_2$, -(C=O)-R', SH, SR', SOR', $SO_2R'$, $NH_2$, NHR', NR'R", -(C=O)-$NH_2$, -(C=O)-NHR' and -(C=O)-NR'R", whereby R' and R" for each substituent independently represent linear or branched $C_{1-6}$ alkyl, preferably $R^1$ represents a phenyl group, which is optionally substituted by one or more substituents selected from the group consisting of methyl, ethyl, F, Cl, Br and $CF_3$, more preferably $R^1$ represents a phenyl group, which is substituted with a chlorine atom in the 4-position, and $R^2$-$R^8$ have the meaning given above, optionally in form of one of the stereoisomers, preferably enantiomers or diastereomers, a racemate or in form of a mixture of at least two of the stereoisomers, preferably enantiomers and/or diastereomers, in any mixing ratio, or a corresponding N-oxide thereof, or a corresponding salt thereof, or a corresponding solvate thereof.

Also preferred are substituted pyrazoline compounds of general formula I given above, wherein $R^2$ represents a phenyl group, which is optionally substituted by one or more substituents independently selected from the group consisting of a linear or branched $C_{1-6}$-alkyl group, a linear or branched $C_{1-6}$-alkoxy group, a halogen atom, $CH_2F$, $CHF_2$, $CF_3$, CN, OH, $NO_2$, -(C=O)-R', SH, SR', SOR', $SO_2R'$ $NH_2$, NHR', NR'R", -(C=O)-$NH_2$, -(C=O)-NHR' and -(C=O)-NR'R" whereby R' and R" for each substituent independently represent linear or branched $C_{1-6}$ alkyl, preferably $R^2$ represents a phenyl group, which is optionally substituted by one or more substituents selected from the group consisting of methyl, ethyl, F, Cl, Br and $CF_3$, more preferably $R^2$ a phenyl group, which is di-substituted with two chlorine atoms in the 2- and 4-position, and $R^1$ and $R^3$-$R^8$ have the meaning given above, optionally in form of one of the stereoisomers, preferably enantiomers or diastereomers, a racemate or in form of a mixture of at least two of the stereoisomers, preferably enantiomers and/or diastereomers, in any mixing ratio, or a corresponding N-oxide thereof, or a corresponding salt thereof, or a corresponding solvate thereof.

Preference is also given to substituted pyrazoline compounds of general formula I given above, wherein $R^3$ represents a saturated or unsaturated, optionally at least mono-substituted, optionally at least one heteroatom as ring member containing $C_{3-8}$ cycloaliphatic group, which may be condensed with an optionally at least mono-substituted mono- or polycyclic ring system, or $R^3$ represents an optionally at least mono-substituted, 5- or 6-membered aryl or heteroaryl group, which may be condensed with an optionally at least mono-substituted mono- or polycyclic ring system, or $R^3$ represents an —$NR^4R^5$-moiety, preferably $R^3$ represents a saturated, optionally at least mono-substituted, optionally one or more nitrogen-atoms as ring member containing $C_{3-8}$ cycloaliphatic group, which may be condensed with an optionally at least mono-substituted mono- or polycyclic ring system, or $R^3$ represents an —$NR^4R^5$-moiety, more preferably $R^3$ represents a pyrrolidinyl group, a piperidinyl group or a piperazinyl group, whereby each of these groups may be substituted with one or more $C_{1-6}$-alkyl groups, or $R^3$ represents an -$NR^4R^5$-moiety and $R^1$, $R^2$ and $R^4$-$R^8$ have the meaning given above, optionally in form of one of the stereoisomers, preferably enantiomers or diastereomers, a racemate or in form of a mixture of at least two of the stereoisomers, preferably enantiomers and/or diastereomers, in any mixing ratio, or a corresponding N-oxide thereof, or a corresponding salt thereof, or a corresponding solvate thereof.

Furthermore, substituted pyrazoline compounds of general formula I given above are preferred, wherein $R^4$ and $R^5$, identical or different, represent a hydrogen atom, an unbranched or branched, saturated or unsaturated, optionally at least mono-substituted $C_{1-6}$-aliphatic radical, a saturated or unsaturated, optionally at least mono-substituted, optionally at least one heteroatom as ring member containing $C_{3-8}$-cycloaliphatic group, which may be condensed with an optionally at least mono-substituted mono- or polycyclic ring system, or an optionally at least mono-substituted, 5- or 6-membered aryl or heteroaryl group, which may be condensed with an optionally at least mono-substituted mono- or polycyclic ring system and/or bonded via a methylene (-$CH_2$-) or ethylene (-$CH_2$-$CH_2$)-group, an —$SO_2$-$R^6$-moiety, or an -$NR^7R^8$-moiety, preferably one of these residues $R^4$ and $R^5$ represents a hydrogen atom and the other one of these residues $R^4$ and $R^5$ represents a saturated or unsaturated, optionally at least mono-substituted, optionally at least one heteroatom as ring

member containing $C_{3-8}$-cycloaliphatic group, which may be condensed with an optionally at least mono-substituted mono- or polycyclic ring system, or an optionally at least mono-substituted, 5- or 6-membered aryl or heteroaryl group, which may be condensed with an optionally at least mono-substituted mono- or polycyclic ring system, an —$SO_2$-$R^6$-moiety, or an -$NR^7R^8$-moiety, or $R^4$ and $R^5$, identical or different, each represent a $C_{1-6}$ alkyl group, more preferably one of these residues $R^4$ and $R^5$ represents a hydrogen atom and the other one of these residues $R^4$ and $R^5$ represents an optionally at least mono-substituted pyrrolidinyl group, an optionally at least mono-substituted piperidinyl group, an optionally at least mono-substituted piperazinyl group, an optionally at least mono-substituted triazolyl group, an -$SO_2$-$R^6$-moiety, or an -$NR^7R^8$-moiety, or $R^4$ and $R^5$, identical or different, represent a methyl group, an ethyl group, an n-propyl group, an isopropyl group, an n-butyl group, a sec-butyl group or a tert.-butyl group, and $R^1$-$R^3$ and $R^6$-$R^8$ have the meaning given above, optionally in form of one of the stereoisomers, preferably enantiomers or diastereomers, a racemate or in form of a mixture of at least two of the stereoisomers, preferably enantiomers and/or diastereomers, in any mixing ratio, or a corresponding N-oxide thereof, or a corresponding salt thereof, or a corresponding solvate thereof.

Also preferred are substituted pyrazoline compounds of general formula I given above, wherein $R^6$ represents a linear or branched, saturated or unsaturated, optionally at least mono-substituted $C_{1-6}$ aliphatic group, a saturated or unsaturated, optionally at least mono-substituted, optionally at least one heteroatom as ring member containing $C_{3-8}$ cycloaliphatic group, which may be condensed with a mono-or polycyclic ring-system, or an optionally at least mono-substituted, 5- or 6-membered aryl or heteroaryl group, which may be condensed with a mono- or polycyclic ring system and/or bonded via a methylene (-$CH_2$-) or ethylene (-$CH_2$-$CH_2$)-group, preferably $R^6$ represents a $C_{1-6}$-alkyl group, a saturated, optionally at least mono-substituted cycloaliphatic group, which may be condensed with a mono-or polycyclic ring-system, or a phenyl group, which is optionally substituted with one or more $C_{1-6}$ alkyl groups, and $R^1$-$R^5$, $R^7$ and $R^8$ have the meaning given above, optionally in form of one of the stereoisomers, preferably enantiomers or diastereomers, a racemate or in form of a mixture of at least two of the stereoisomers, preferably enantiomers and/or diastereomers, in any mixing ratio, or a corresponding N-oxide thereof, or a corresponding salt thereof, or a corresponding solvate thereof.

Moreover substituted pyrazoline compounds of general formula I given above are preferred, wherein $R^7$ and $R^8$, identical or different, represent a hydrogen atom, an unbranched or branched, saturated or unsaturated, optionally at least mono-substituted $C_{1-6}$ aliphatic radical, a saturated or unsaturated, optionally at least mono-substituted, optionally at least one heteroatom as ring member containing $C_{3-8}$ cycloaliphatic group, which may be condensed with an optionally at least mono-substituted mono- or polycyclic ring system, or an optionally at least mono-substituted, 5- or 6 membered aryl or heteroaryl group, which may be condensed with an optionally at least mono-substituted mono- or polycyclic ring system and/or bonded via a methylene (-$CH_2$-) or ethylene (-$CH_2$-$CH_2$)-group, preferably represent a hydrogen atom or a $C_{1-6}$ alkyl radical, and $R^1$-$R^6$ have the meaning given above, optionally in form of one of the stereoisomers, preferably enantiomers or diastereomers, a racemate or in form of a mixture of at least two of the stereoisomers, preferably enantiomers and/or diastereomers, in any mixing ratio, or a corresponding N-oxide thereof, or a corresponding salt thereof, or a corresponding solvate thereof.

Particularly preferred are compounds of general formula I given below,

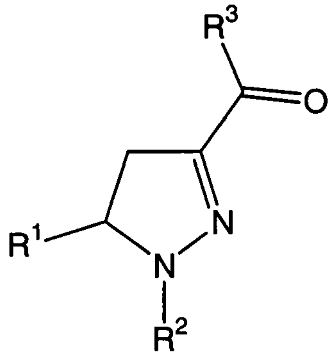

I

wherein

$R^1$ represents a phenyl ring, which is mono-substituted with a halogen atom, preferably a chlorine atom, in its 4-position,
$R^2$ represents a phenyl ring, which is di-substituted with two halogen atoms, preferably chlorine atoms, in its 2- and 4-position,
$R^3$ represents a pyrrolidinyl group, a piperidinyl group, a piperazinyl group, a homo-piperazinyl group, a morpholinyl group, or an —$NR^4R^5$-moiety,

$R^4$ represents a hydrogen atom or a linear or branched $C_{1-6}$-alkyl group,
$R^5$ represents a linear or branched $C_{1-6}$ alkyl group, a pyrrolidinyl group, a piperidinyl group, a piperazinyl group, a homopiperazinyl group, a morpholinyl group, a triazolyl group, whereby each of the heterocyclic rings may be substituted with one or more, identical or different, $C_{1-6}$-alkyl groups, or an -$SO_2$-$R^6$-moiety, and
$R^6$ represents a phenyl group, which is optionally substituted with one or more $C_{1-6}$ alkyl groups, which may be identical or different,
optionally in form of one of the stereoisomers, preferably enantiomers or diastereomers, a racemate or in form of a mixture of at least two of the stereoisomers, preferably enantiomers and/or diastereomers, in any mixing ratio, or a corresponding N-oxide thereof, or a corresponding salt thereof, or a corresponding solvate thereof.

Most particularly preferred are substituted pyrazoline compounds selected from the group consisting of:

N-piperidinyl-5-(4-chloro-phenyl)-1-(2,4-dichlorophenyl)-4,5-dihydro-1H-pyrazole-3-carboxamide,

5-(4-Chloro-phenyl)-1-(2,4-dichloro-phenyl)-4,5-dihydro-1H-pyrazole-3-carboxylic acid-[1,2,4]-triazole-4-yl-amide,

5-(4-Chloro-phenyl)-1-(2,4-dichloro-phenyl)-4,5-dihydro-1H-pyrazole-3-carboxylic acid-(4-methyl-piperazin-1-yl)-amide,

5-(4-Chloro-phenyl)-1-(2,4-dichloro-phenyl)-4,5-dihydro-1H-pyrazole-3-carboxylic acid diethylamide,

[5-(4-Chloro-phenyl)-1-(2,4-dichloro-phenyl)-4,5-dihydro-1H-pyrazole-3-yl]-piperidine-1-yl-methanone,

N-[5-(4-Chloro-phenyl)-1-(2,4-dichlorophenyl)-4,5-dihydro-1H-pyrazole-3-carbonyl]-4-methylphenylsufonamide,

optionally in the form of a corresponding N-oxide, or a corresponding salt, or a corresponding solvate.

In another aspect the present invention also provides a process for the preparation of substituted pyrazoline compounds of general formula I, according to which at least one benzaldehyde compound of general formula IV

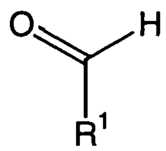

(IV)

wherein $R^1$ has the meaning given above, is reacted with a pyruvate compound of general formula (V)

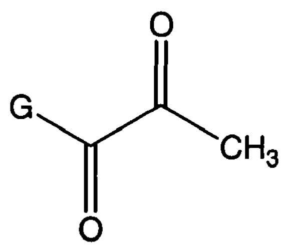

(V),

wherein G represents an OR group with R being a branched or unbranched $C_{1-6}$ alkyl radical, preferably an ethyl radical, or G represents an $O^-K$ group with K being a cation, preferably a monovalent cation, more preferably an alkali metal cation, even more preferably a sodium cation, to yield a compound of general formula (VI)

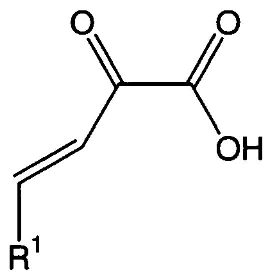

(VI)

wherein $R^1$ has the meaning given above, which is optionally isolated and/or optionally purified, and which is reacted with an optionally substituted phenyl hydrazine of general formula (VII)

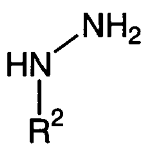

(VII)

or a corresponding salt thereof, wherein $R^2$ has the meaning given above, under an inert atmosphere, to yield a compound of general formula (VIIII)

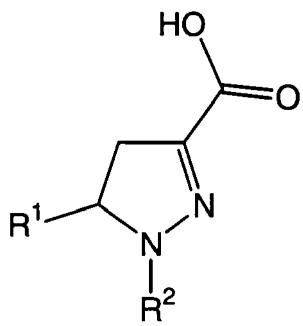

(VIII)

wherein $R^1$ and $R^2$ have the meaning as given above, which is optionally isolated and/or optionally purified, and optionally transferred under inert atmosphere to a compound of general formula (IX)

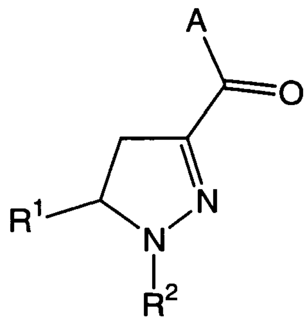

(IX)

wherein the substituents $R^1$ and $R^2$ have the meaning given above and A represents a leaving group, via the reaction with an activating agent, said compound being optionally isolated and/or optionally purified, and at least one compound of general formula (VI) is reacted with a compound of general formula $R^3H$, wherein $R^3$ represents an $-NR^4R^5$-moiety, wherein $R^4$ and $R^5$ have the meaning given above, to yield a substituted pyrazoline compound of general formula I, wherein $R^3$ represents an $-NR^4R^5$-moiety,
and/or at least one compound of general formula (IX) is reacted with a compound of the general formula $R^3H$, in which $R^3$ has the meaning given above to yield a compound of general formula (I) given above, which is optionally isolated and/or optionally purified.
The inventive process is also illustrated in scheme I given below:

**Scheme I:**

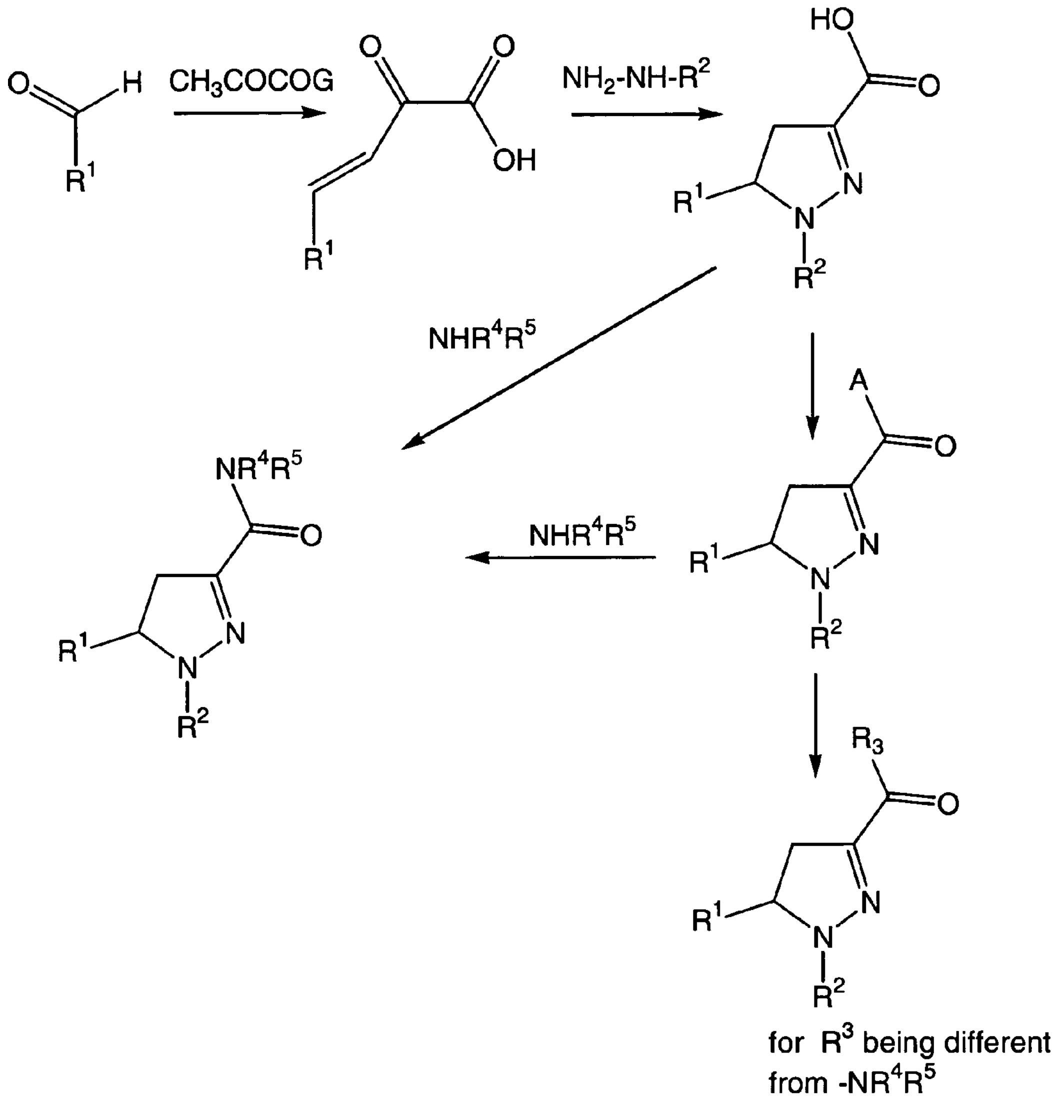


The reaction of the benzaldehyde compound of general formula IV with a pyruvate compound of general formula V is preferably carried out in the presence of at least one base, more preferably in the presence of an alkali metal hydroxide such as sodium hydroxide or potassium hydroxide or an alkali metal methoxide such as sodium methoxide, as described, for example, in Synthetic communications, 26(11), 2229-33, (1996). The respective description is hereby incorporated by reference and forms part of the disclosure. Preferably sodium pyruvate may be used as the pyruvate compound. Preferably said reaction is carried out in a protic reaction medium such as a $C_{1-4}$ alkyl alcohol or mixtures of these. Mixtures of such alcohols with water, e.g. ethanol/water may also be used.

Reaction temperature as well as the duration of the reaction may vary over a broad range. Preferred reaction temperatures range from -10 °C to the boiling point of the reaction medium. Suitable reaction times may vary for example from several minutes to several hours.

Also preferred the reaction of the benzaldehyde compound of general formula IV with a pyruvate compound of general formula V is carried out under acid catalysed conditions, more preferably by refluxing the mixture in dichloromethane in the presence of copper(II)trifluoromethanesulfonate as described, for example, in Synlett, (1), 147-149, 2001. The respective description is hereby incorporated by reference and forms part of the disclosure.

The reaction of the compound of general formula (VI) with an optionally substituted phenyl hydrazin of general formula (VII) is preferably carried out in a suitable reaction medium such as $C_{1-4}$-alcohols or ethers such as dioxane or tetrahydrofurane or mixtures of at least two of these afore mentioned compounds. Also preferably, said reaction may be carried

out in the presence of an acid, whereby the acid may be organic such as acetic acid and/or inorganic such as hydrochloric acid. Furthermore, the reaction may also be carried out in the presence of a base such as piperidine, piperazine, sodium hydroxide, potassium hydroxide, sodium methoxide or sodium ethoxide, or a mixture of at least two of these bases may also be used.

Reaction temperature as well as the duration of the reaction may vary over a broad range. Suitable reaction temperatures range from room temperature, i.e. approximately 25 °C to the boiling point of the reaction medium. Suitable reaction times may vary for example from several minutes to several hours.

The carboxylic group of the compound of general formula (VIII) may be activated for further reactions by the introduction of a suitable leaving group according to conventional methods well known to those skilled in the art. Preferably the compounds of general formula (VIII) are transferred into an acid chloride, an acid anhydride, a mixed anhydride, a $C_{1-4}$ alkyl ester, an activated ester such as p-nitrophenylester. Other well known methods for the activation of acids include the activation with N,N-dicyclohexylcarbodiimide or benzotriazol-N-oxotris(dimethylamino) phosphonium hexafluorophosphate (BOP)).

If said activated compound of general formula (IX) is an acid chloride, it is preferably prepared by reaction of the corresponding acid of general formula (VIII) with thionyl chloride or oxalyl chloride, whereby said chlorinating agent is also used as the solvent. Also preferably an additional solvent may be used. Suitable solvents include hydrocarbons such as benzene, toluene or xylene, halogenated hydrocarbons such as dichloromethane, chloroform or carbon tetrachloride, ethers such as diethyl ether, dioxane, tetrahydrofurane or dimethoxyethane. Mixtures of two or more solvents from one class or two or more solvents from different classes may also be used. Preferred reaction temperature range from 0º C to the boiling point of the solvent and reaction times from several minutes to several hours.

If said activated compound of general formula (IX) is a mixed anhydride, said anhydride may preferably be prepared, for example, by reaction of the corresponding acid of general formula (VIII) with ethyl chloroformiate in the presence of a base such as triethylamine or pyridine, in a suitable solvent.

The reaction of general formula (IX) with a compound of general formula $HR^3$ to yield compounds of general formula I, wherein $R^3$ represents an $-NR^4R^5$ moiety is preferably carried out in presence of a base such as triethylamine in a reaction medium such as methylenchloride. The temperature is preferably in the range from 0ºC to the boiling point of the reaction medium. The reaction time may vary over a broad range, e.g. from several hours to several days.

The reaction of general formula (IX) with a compound of general formula $HR^3$ to yield compounds of general formula I, wherein $R^3$ represents a saturated or unsaturated, optionally at least mono-substituted, optionally at least one heteroatom as ring member containing cycloaliphatic group, which may be condensed with an optionally at least mono-substituted mono- or polycyclic ring system, or an optionally at least mono-substituted aryl or heteroaryl group, which may be condensed with an optionally at least mono-substituted mono- or polycyclic ring system may be carried out according to conventional methods well known to those skilled in the art, e.g. from Pascual, A., J. Prakt Chem., 1999, 341(7), 695-700; Lin, S. et al., Heterocycles, 2001, 55(2), 265-277; Rao, P. et al., J. Org. Chem., 2000, 65(22), 7323-7344, Pearson D.E and Buehler, C.A., Synthesis, 1972, 533-542 and references cited therein. The respective descriptions are hereby incorporated by reference and form part of the present disclosure.

Preferably said reaction is carried out in the presence of a Lewis acid, which is preferably selected from the group consisting of $FeCl_3$, $ZnCl_2$ and $AlCl_3$, in a suitable reaction medium such as toluene, benzene, tetrahydrofurane or similar. The temperature is preferably in teh range from 0ºC to the boiling point of the reaction medium, more preferably from 15 to 25 °C. The reaction time may vary over a broad range, e.g. from several minutes to several hours.

The afore mentioned reactions involving the synthesis of the 4,5-dihydro-pyrazole ring or the reaction of a compound comprising said ring are carried out under an inert atmosphere, preferably nitrogen or argon, to avoid oxidation of the ring-system. During the processes described above the protection of sensitive groups or of reagents may be necessary and/or desirable. The introduction of conventional protective groups as well as their removal may be performed by methods well-known to those skilled in the art.

If the substituted pyrazoline compounds of general formula (I) themselves are obtained in form of a mixture of stereoisomers, particularly enantiomers or diastereomers, said mixtures may be separated by standard procedures known to those skilled in the art, e.g. chromatographic methods or fractunalized crystallization with chiral reagents. It is also possible to obtain pure stereoisomers via stereoselective synthesis.

In another aspect the present invention relates to the compound

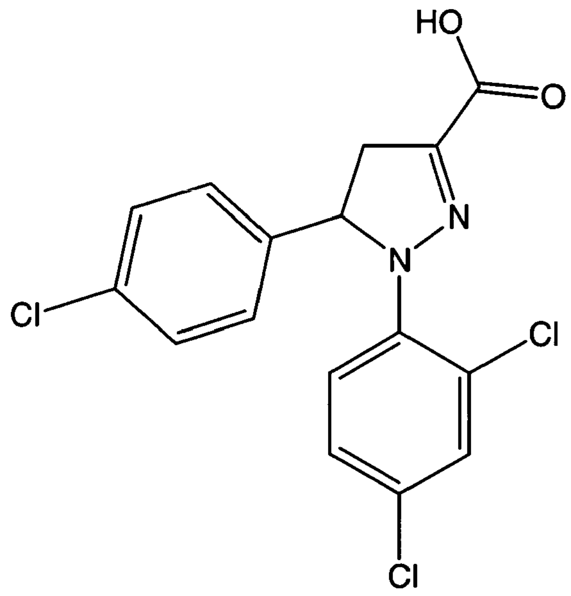

optionally in form of one of the stereoisomers, preferably enantiomers or diastereomers, a racemate or in form of a mixture of at least two of the stereoisomers, preferably enantiomers and/or diastereomers, in any mixing ratio, or a corresponding salt thereof, or a corresponding solvate thereof, in particular as an intermediate in a process for preparing substituted pyrazoline compounds of general formula (I).

In a further aspect the present invention also provides a process for the preparation of salts of substituted pyrazoline compounds of general formula (I) and stereoisomers thereof, wherein at least one compound of general formula (I) having at least one basic group is reacted with at least one inorganic and/or organic acid, preferably in the presence of a suitable reaction medium. Suitable reaction media include, for example, any of the ones given above. Suitable inorganic acids include hydrochloric acid, hydrobromic acid, phosphoric acid, sulfuric acid, nitric acid, suitable organic acids are e.g. citric acid, maleic acid, fumaric acid, tartaric acid, or derivatives thereof, p-toluenesulfonic acid, methanesulfonic acid or camphersulfonic acid.

In yet a further aspect the present invention also provides a process for the preparation of salts of substituted pyrazoline compounds of general formula (I) or stereoisomers thereof, wherein at least one compound of general formula (I) having at least one acidic group is reacted with one or more suitable bases, preferably in the presence of a suitable reaction medium. Suitable bases are e.g. hydroxides, carbonates or alkoxides, which include suitable cations, derived e.g. from alkaline metals, alkaline earth metals or organic cations, e.g. $[NH_nR_{4-n}]^+$, wherein n is 0, 1, 2, 3 or 4 and R represents a branched or unbranched $C_{1-4}$-alkyl-radical. Suitable reaction media are, for example, any of the ones given above.

Solvates, preferably hydrates, of the substituted pyrazoline compounds of general formula (I), of corresponding stereoisomers, of corresponding N-oxides or of corresponding salts thereof may also be obtained by standard procedures known to those skilled in the art.

Substituted pyrazoline compounds of general formula I, which comprise nitrogen-atom containing saturated, unsaturated or aromatic rings may also be obtained in the form of their N-oxides by methods well known to those skilled in the art.

Those skilled in the art understand that the term substituted pyrazoline compounds as used herein is to be understood as encompassing derivatives such as ethers, esters and complexes of these compounds as well. The term "derivatives" as used in this application is defined here as meaning a chemical compound having undergone a chemical derivation starting from an acting (active) compound to change (ameliorate for pharmaceutical use) any of its physico-chemical properties, especially a so-called prodrug, e.g. their esters and ethers. Examples of well known methods of producing a prodrug of a given acting compound are known to those skilled in the art and can be found e.g. in Krogsgaard-Larsen et al., Textbook of Drugdesign and Discovery, Taylor & Francis (April 2002). The respective description is hereby incorporated by reference and forms part of the disclosure.

The purification and isolation of the inventive substituted pyrazoline compounds of general formula (I), of a corresponding stereoisomer, or salt, or N-oxide, or solvate or any intermediate thereof may, if required, be carried out by conventional methods known to those skilled in the art, e.g. chromatographic methods or recrystallization. The substituted pyrazoline compounds of general formula (I) given below, their stereoisomers, corresponding N-oxides, corresponding salts thereof and corresponding solvates are toxicologically acceptable and are therefore suitable as pharmaceutical active substances for the preparation of medicaments.

It has been found that the substituted pyrazoline compounds of general formula I given below, stereoisomers thereof,

N-oxides thereof, corresponding salts and corresponding solvates have a high affinity to cannabinoid receptors, particularly cannabinoid 1 ($CB_1$)-receptors, i.e. they are selective ligands for the ($CB_1$)-receptor and act as modulators, e.g. antagonists, inverse agonists or agonists, on these receptors. In particular, these pyrazoline compounds show little or no development of tolerance during treatment, particularly with respect to food intake, i.e. if the treatment is interrupted for a given period of time and then continued afterwards, the inventively used pyrazoline compounds will again show the desired effect. After ending the treatment with the pyrazoline compounds, the positive influence on the body weight is found to continue.

Furthermore, these pyrazoline compounds show relatively weak Herg channel affinity, thus a low risk of prolongation of the QT-interval is to be expected for these compounds.

In summary, the inventively used pyrazoline compounds are distinguished by a broad spectrum of beneficial effects, while at the same time showing relatively little undesired effects, i.e. effects which do not positively contribute to or even interfere with the well being of the patient.

In another aspect of the invention, the invention relates to substituted pyrazoline compounds of the general formula II

II

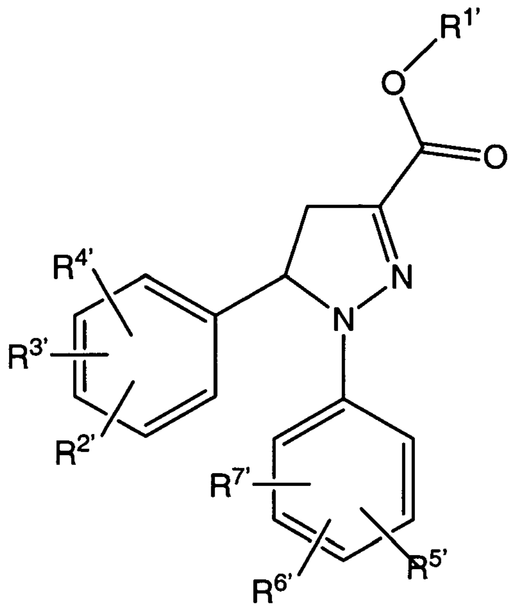

wherein

$R^{1'}$ represents hydrogen or a linear or branched $C_{1-4}$-alkyl group,

$R^{2'}$, $R^{3'}$ and $R^{4'}$ independently of each other represent hydrogen, a linear or branched $C_{1-6}$-alkyl group, a linear or branched $C_{1-6}$-alkoxy group, a halogen atom, $CH_2F$, $CHF_2$, $CF_3$, CN, OH, $NO_2$, -(C=O)-$R^{8'}$, SH, $SR^{8'}$, $SOR^{8'}$, $SO_2R^{8'}$, $NH_2$, $NHR^{8'}$, $NR^{8'}R^{9'}$, -(C=O)-$NH_2$, -(C=O)-$NHR^{8'}$ or -(C=O)-$NR^{8'}R^{9'}$ whereby $R^{8'}$ and $R^{9'}$ for each substituent independently represent linear or branched $C_{1-6}$ alkyl,

$R^{5'}$ and $R^{6'}$ independently of each other represent a linear or branched $C_{1-6}$-alkyl group, a linear or branched $C_{1-6}$-alkoxy group, a halogen atom, $CH_2F$, $CHF_2$, $CF_3$, CN, OH, $NO_2$, -(C=O)-$R^{10'}$, SH, $SR^{10'}$, $SOR^{10'}$, $NH_2$, $NHR^{10'}$, $NR^{10'}R^{11'}$, -(C=O)-$NH_2$, -(C=O)-$NHR^{10'}$ and -(C=O)-$NR^{10'}R^{11'}$, whereby $R^{10'}$ and optionally $R^{11'}$ for each substituent independently represent linear or branched $C_{1-6}$alkyl;

$R^{7'}$ represents hydrogen, a linear or branched $C_{1-6}$-alkyl group, a linear or branched $C_{1-6}$-alkoxy group, a halogen atom, $CH_2F$, $CHF_2$, $CF_3$, CN, OH, $NO_2$, -(C=O)-$R^{10'}$, SH, $SR^{10'}$, $SOR^{10'}$, $NH_2$, $NHR^{10'}$, $NR^{10'}R^{11'}$, -(C=O)-$NH_2$, -(C=O)-$NHR^{10'}$ and -(C=O)-$NR^{10'}R^{11'}$, whereby $R^{10'}$ and optionally $R^{11'}$ for each substituent independently represent linear or branched

$C_{1-6}$ alkyl;
with the proviso that
if $R^{1'}$ and $R^{7'}$ are H and $R^{5'}$ and $R^{6'}$ both represent Cl in the 3- and 4-position of the phenyl ring neither of $R^{2'}$, $R^{3'}$ and $R^{4'}$ may represent F in the 4-position of the phenyl ring if the other two of $R^{2'}$, $R^{3'}$ and $R^{4'}$ both represent H; optionally in form of one of the stereoisomers, preferably enantiomers or diastereomers, a racemate or in form of a mixture of at least two of the stereoisomers, preferably enantiomers and/or diastereomers, in any mixing ratio, or a corresponding N-oxide thereof, or a corresponding salt thereof, or a corresponding solvate thereof and nanoparticles for the production of a medicament for the prophylaxis and/or treatment of disorders related to the cannabinoid receptor system.

With respect to compounds of general formula II,
preferred are linear or branched, saturated or unsaturated aliphatic groups, which may be substituted by one or more substituents, may preferably be selected from the group consisting of methyl, ethyl, n-propyl, isopropyl, n-butyl, iso-butyl, sec-butyl, tert-butyl, n-pentyl, n-hexyl, n-heptyl, n-octyl, n-nonyl, n-decyl, vinyl, ethinyl, propenyl, propinyl, butenyl and butinyl.

In the context of this invention, alkyl and cycloalkyl radicals are understood as meaning saturated and unsaturated (but not aromatic), branched, unbranched and cyclic hydrocarbons, which can be unsubstituted or mono- or polysubstituted. In these radicals, $C_{1-2}$-alkyl represents C1- or C2-alkyl, $C_{1-3}$-alkyl represents C1-, C2- or C3-alkyl, $C_{1-4}$-alkyl represents C1-, C2-, C3- or C4-alkyl, $C_{1-5}$-alkyl represents C1-, C2-, C3-, C4-, or C5-alkyl, $C_{1-6}$-alkyl represents C1-, C2-, C3-, C4-, C5- or C6-alkyl, $C_{1-7}$-alkyl represents C1-, C2-, C3-, C4-, C5-, C6- or C7-alkyl, $C_{1-8}$-alkyl represents C1-, C2-, C3-, C4-, C5-, C6-, C7- or C8-alkyl, $C_{1-10}$-alkyl represents C1-, C2-, C3-, C4-, C5-, C6-, C7-, C8-, C9- or C10-alkyl and $C_{1-18}$-alkyl represents C1-, C2-, C3-, C4-, C5-, C6-, C7-, C8-, C9-, C10-, C11-, C12-, C13-, C14-, C15-, C16-, C17- or C18-alkyl. Furthermore, $C_{3-4}$-cycloalkyl represents C3- or C4-cycloalkyl, $C_{3-5}$-cycloalkyl represents C3-, C4- or C5-cycloalkyl, $C_{3-6}$-cycloalkyl represents C3-, C4-, C5- or C6-cycloalkyl, $C_{3-7}$-cycloalkyl represents C3-, C4-, C5-, C6- or C7-cycloalkyl, $C_{3-8}$-cycloalkyl represents C3-, C4-, C5-, C6-, C7- or C8-cycloalkyl, $C_{4-5}$-cycloalkyl represents C4- or C5-cycloalkyl, $C_{4-6}$-cycloalkyl represents C4-, C5- or C6-cycloalkyl, $C_{4-7}$-cycloalkyl represents C4-, C5-, C6- or C7-cycloalkyl, $C_{5-6}$-cycloalkyl represents C5- or C6-cycloalkyl and $C_{5-7}$-cycloalkyl represents C5-, C6- or C7-cycloalkyl. In respect of cycloalkyl, the term also includes saturated cycloalkyls in which one or 2 carbon atoms are replaced by a heteroatom, S, N or O. However, mono- or polyunsaturated, preferably monounsaturated, cycloalkyls without a heteroatom in the ring also in particular fall under the term cycloalkyl as long as the cycloalkyl is not an aromatic system. The alkyl and cycloalkyl radicals are preferably methyl, ethyl, vinyl (ethenyl), propyl, allyl (2-propenyl), 1-propinyl, methylethyl, butyl, 1-methylpropyl, 2-methylpropyl, 1,1-dimethylethyl, pentyl, 1,1-dimethylpropyl, 1,2-dimethylpropyl, 2,2-dimethylpropyl, hexyl, 1-methylpentyl, cyclopropyl, 2-methylcyclopropyl, cyclopropylmethyl, cyclobutyl, cyclopentyl, cyclopentylmethyl, cyclohexyl, cycloheptyl, cyclooctyl, and also adamantyl, (if substituted also $CHF_2$, $CF_3$ or $CH_2OH$) as well as pyrazolinone, oxopyrazolinone, [1,4]-dioxane or dioxolane. Here, in connection with alkyl and cycloalkyl - unless expressly defined otherwise - the term substituted in the context of this invention is understood as meaning replacement of at least one hydrogen radical by F, Cl, Br, I, $NH_2$, SH or OH, "polysubstituted" radicals being understood as meaning that the replacement takes effect both on different and on the same atoms several times with the same or different substituents, for example three times on the same C atom, as in the case of $CF_3$, or at different places, as in the case of $-CH(OH)-CH=CH-CHCl_2$. Particularly preferred substituents here are F, Cl and OH. In respect of cycloalkyl, the hydrogen radical can also be replaced by $OC_{1-3}$-alkyl or $C_{1-3}$-alkyl (in each case mono- or polysubstituted or unsubstituted), in particular methyl, ethyl, n-propyl, i-propyl, $CF_3$, methoxy or ethoxy.

The term $(CH_2)_{3-6}$ is to be understood as meaning $-CH_2-CH_2-CH_2-$, $-CH_2-CH_2-CH_2-CH_2-$, $-CH_2-CH_2-CH_2-CH_2-CH_2-$ and $-CH_2-CH_2-CH_2-CH_2-CH_2-CH_2-$, $(CH_2)_{1-4}$ is to be understood as meaning $-CH_2-$, $-CH_2-CH_2-$, $-CH_2-CH_2-CH_2-$ and $-CH_2-CH_2-CH_2-CH_2-$, $(CH_2)_{4-5}$ is to be understood as meaning $-CH_2-CH_2-CH_2-CH_2-$ and $-CH_2-CH_2-CH_2-CH_2-CH_2-$, etc.

An aryl radical is understood as meaning ring systems with at least one aromatic ring but without heteroatoms even in only one of the rings. Examples are phenyl, naphthyl, fluoranthenyl, fluorenyl, tetralinyl or indanyl, in particular 9H-fluorenyl or anthracenyl radicals, which can be unsubstituted or monosubstituted or polysubstituted.

A heteroaryl radical is understood as meaning heterocyclic ring systems which have at least one unsaturated ring and can contain one or more heteroatoms from the group consisting of nitrogen, oxygen and/or sulfur and can also be mono- or polysubstituted. Examples which may be mentioned from the group of heteroaryls are furan, benzofuran, thiophene, benzothiophene, pyrrole, pyridine, pyrimidine, pyrazine, quinoline, isoquinoline, phthalazine, benzo-1,2,5-thiadiazole, benzothiazole, indole, benzotriazole, benzodioxolane, benzodioxane, carbazole and quinazoline.

Here, in connection with aryl and heteroaryl, substituted is understood as meaning substitution of the aryl or heteroaryl by R, OR, a halogen, preferably F and/or Cl, a $CF_3$, a CN, an $NO_2$, an NRR, a $C_{1-6}$-alkyl (saturated), a $C_{1-6}$-alkoxy, a $C_{3-8}$-cycloalkoxy, a $C_{3-8}$-cycloalkyl or a $C_{2-6}$-alkylene.

In a preferred embodiment of the invention for a compound according to formula II at least one of $R^{2'}$, $R^{3'}$ or $R^{4'}$ represents hydrogen, while at least one of $R^{2'}$, $R^{3'}$ or $R^{4'}$ is different from hydrogen.

In a preferred embodiment of the invention for a compound according to formula II $R^{7'}$ represents hydrogen.

In a preferred embodiment of the invention for a compound according to formula II $R^{2'}$, $R^{3'}$ and $R^{4'}$ independently of

each other represent hydrogen, a linear or branched $C_{1-6}$-alkyl group, a halogen atom, or $CF_3$, preferably $R^{2'}$, $R^{3'}$ and $R^{4'}$ independently of each other represent hydrogen, methyl, ethyl, F, Cl, Br and $CF_3$.

In a preferred embodiment of the invention for a compound according to formula II $R^{5'}$ and $R^{6'}$ independently of each other represent a linear or branched $C_{1-6}$-alkyl group, a halogen atom, or $CF_3$, preferably $R^{5'}$ and $R^{6'}$ independently of each other represent methyl, ethyl, F, Cl, Br and $CF_3$.

In a preferred embodiment of the invention for a compound according to formula II $R^{2'}$ represents a chlorine atom in the 4-position of the phenyl ring, while $R^{3'}$ and $R^{4'}$ represent hydrogen.

In a preferred embodiment of the invention for a compound according to formula II $R^{5'}$ and $R^{6'}$ each represent a chlorine atoms in the 2- and 4-position of the phenyl ring, while $R^{7'}$ represents hydrogen.

In a preferred embodiment of the invention for a compound according to formula II $R^{1'}$ represents hydrogen, methyl or ethyl, preferably hydrogen.

In a highly preferred further aspect of the invention the compound of general formula II is represented by a compound of general formula III

wherein

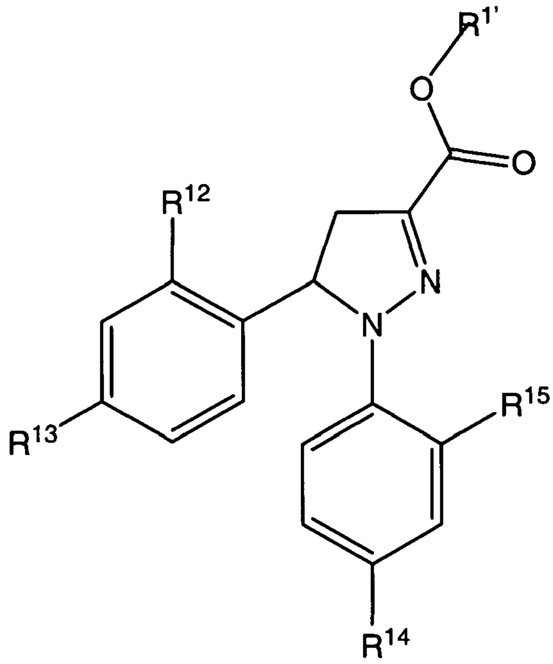

III

$R^{1'}$ represents hydrogen or a linear or branched $C_{1-4}$-alkyl group,

$R^{12}$ or $R^{13}$ independently of each other represent a linear or branched $C_{1-6}$-alkyl group, a linear or branched $C_{1-6}$-alkoxy group, a halogen atom, $CH_2F$, $CHF_2$, $CF_3$, CN, OH, $NO_2$, SH, $NH_2$, hydrogen, methyl, ethyl, F, Cl, Br and $CF_3$, $R^{14}$ or $R^{15}$ independently of each other represent a linear or branched $C_{1-6}$-alkyl group, a linear or branched $C_{1-6}$-alkoxy group, a halogen atom, $CH_2F$, $CHF_2$, $CF_3$, CN, OH, $NO_2$, SH, $NH_2$, methyl, ethyl, F, Cl, Br and $CF_3$, optionally in form of one of the stereoisomers, preferably enantiomers or diastereomers, a racemate or in form of a mixture of at least two of the stereoisomers, preferably enantiomers and/or diastereomers, in any mixing ratio, or a corresponding N-oxide thereof, or a corresponding salt thereof, or a corresponding solvate thereof.

In a preferred embodiment of the invention for a compound according to formula III $R^{12}$ and $R^{13}$ independently of each other represent hydrogen, a linear or branched $C_{1-6}$-alkyl group, a halogen atom, or $CF_3$, preferably $R^{12}$ and $R^{13}$ independently of each other represent hydrogen, methyl, ethyl, F, Cl, Br and $CF_3$.

In a preferred embodiment of the invention for a compound according to formula III $R^{14}$, and $R^{15}$ independently of each other represent a linear or branched $C_{1-6}$-alkyl group, a halogen atom, or $CF_3$, preferably $R^{14}$ and $R^{15}$ independently of each other represent methyl, ethyl, F, Cl, Br and $CF_3$.

In a preferred embodiment of the invention for a compound according to formula III $R^{13}$ represents Cl and $R^{12}$ represents hydrogen.

In a preferred embodiment of the invention for a compound according to formula III $R^{14}$ and $R^{15}$ each represent Cl.

In a preferred embodiment of the invention for a compound according to formula III $R^{1'}$ represents hydrogen, methyl or ethyl, preferably hydrogen.
In another preferred embodiment the compound according to formula II or III is selected from the group consisting of:

5-(4-chloro-phenyl)-1-(2,4-dichlorophenyl)-4,5-dihydro-1H-pyrazol-3-carboxylic acid,
optionally in the form of a corresponding N-oxide, a corresponding salt or a corresponding solvate.

Another preferred embodiment of the invention covers also any prodrug of the compounds of the invention described above as well as any medicament comprising this and any use thereof; especially including their esters and ethers. Examples of well known methods of producing a prodrug of a given acting compound are known to those skilled in the art and can be found e.g. in Krogsgaard-Larsen et al., Textbook of Drugdesign and Discovery, Taylor & Francis (April 2002).

In another aspect the present invention also provides a process for the preparation of substituted pyrazoline compounds of general formula II or III, wherein $R^{1'}$ is hydrogen, given above, in that at least one benzaldehyde compound of general formula IV'

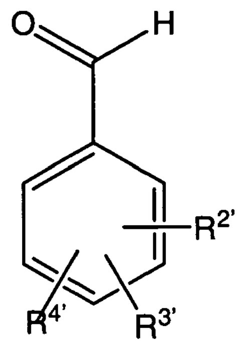

**(IV')**

wherein $R^{2'}$, $R^{3'}$ and $R^{4'}$ have the meaning mentioned above, is reacted with a pyruvate compound of general formula (V')

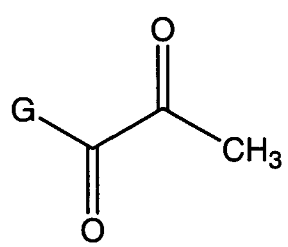

**(V'),**

wherein G represents an OR group with R being a branched or unbranched $C_{1-6}$ alkyl radical or G represents an $O^{-}K$ group with K being a cation, preferably an anorganic kation, more preferably an alkali metal kation, most preferably sodium, to yield a compound of general formula (VI')

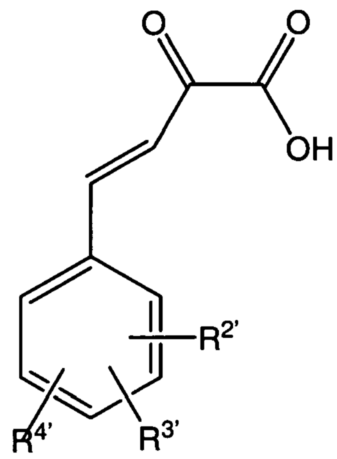

(VI')

which is optionally isolated and/or optionally purified, and which is reacted with an optionally substituted phenyl hydrazine of general formula (VII')

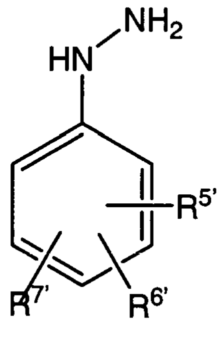

(VII')

or a corresponding salt thereof, wherein $R^{5'}$, $R^{6'}$ and $R^{7'}$ have the meaning mentioned above, under inert atmosphere, to yield a compound of general formula (VIII')

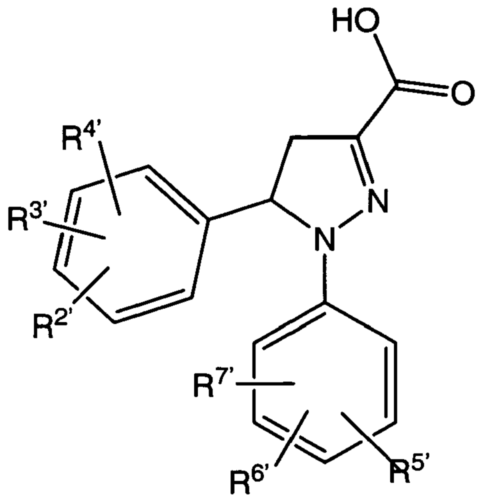

(VIII')

wherein $R^{2'}$, $R^{3'}$, $R^{4'}$, $R^{5'}$, $R^{6'}$ and $R^{7'}$ have the meaning as given above, which is optionally isolated and/or optionally purified, and optionally esterified to an alkyl-ester if in the substituted pyrazoline compound of general formula I or II according to the invention $R^{1'}$ is a linear or branched $C_{1-4}$-alkyl group.

The inventive process is also illustrated in scheme I given below:

**Scheme I:**

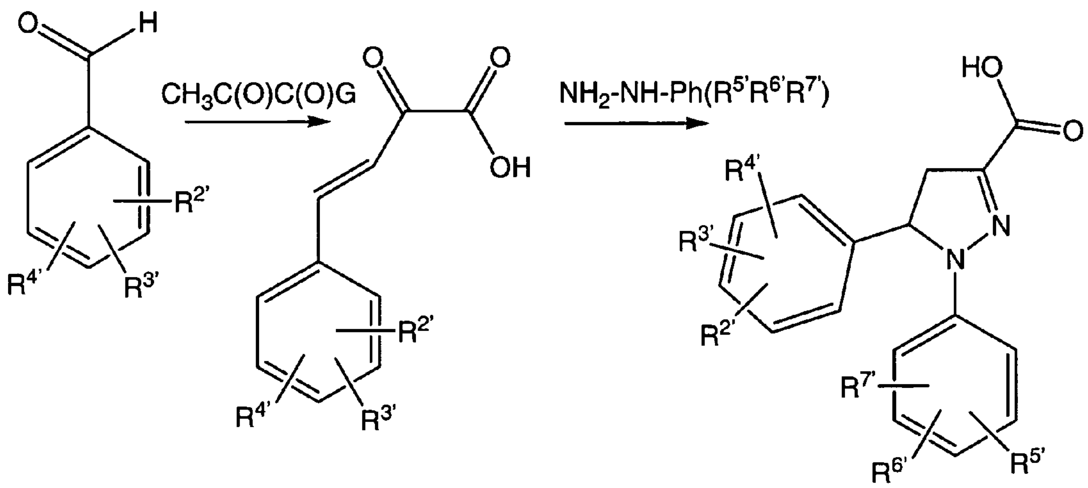


The reaction of the benzaldehyde compound of general formula III with a pyruvate compound of general formula V' is preferably carried out in the presence of at least one base, more preferably in the presence of an alkali metal hydroxide such as sodium hydroxide or potassium hydroxide or an alkali metal methoxide such as sodium methoxide, as described, for example, in Synthetic communications, 26(11), 2229-33, (1996). The respective description is hereby incorporated by reference and forms part of the disclosure. Preferably said reaction is carried out in a protic reaction medium such as a $C_{1-4}$ alkyl alcohol or mixtures of these.

Reaction temperature as well as the duration of the reaction may vary over a broad range. Preferred reaction temperatures range from -10°C to the boiling point of the reaction medium. Suitable reaction times may vary for example from several minutes to several hours.

Also preferred the reaction of the benzaldehyde compound of general formula III with a pyruvate compound of

general formula V' is carried out under acid catalysed conditions, more preferably by refluxing the mixture in dichloromethane in the presence of copper(II)trifluoromethanesulfonate as described, for example, in Synlett, (1), 147-149, 2001. The respective description is hereby incorporated by reference and forms part of the disclosure.

The reaction of the compound of general formula (VI') with an optionally substituted phenyl hydrazin of general formula (VII') is preferably carried out in a suitable reaction medium such as $C_{1-4}$-alcohols or ethers such as dioxane or tetrahydrofurane or mixtures of at least two of these afore mentioned compounds. Also preferably, said reaction may be carried out in the presence of an acid, whereby the acid may be organic such as acetic acid and/or inorganic such as hydrochloric acid. Furthermore, the reaction may also be carried out in the presence of a base such as piperidine, piperazine, sodium hydroxide, potassium hydroxide, sodium methoxide or sodium ethoxide, or a mixture of at least two of these bases may also be used.

Reaction temperature as well as the duration of the reaction may vary over a broad range. Suitable reaction temperatures range from room temperature, i.e. approximately 25 °C to the boiling point of the reaction medium. Suitable reaction times may vary for example from several minutes to several hours.

The carboxylic group of the compound of general formula (VIII') may be activated for further reactions by the introduction of a suitable leaving group according to conventional methods well known to those skilled in the art. Preferably the compounds of general formula (VIII') are transferred into an acid chloride, an acid anhydride, a mixed anhydride, a $C_{1-4}$ alkyl ester, an activated ester such as p-nitrophenylester. Other well known methods for the activation of acids include the activation with N,N-dicyclohexylcarbodiimide or benzotriazol-N-oxotris(dimethylamino) phosphonium hexafluorophosphate (BOP)).

If said activated compound of general formula (VIII') is an acid chloride, it is preferably prepared by reaction of the corresponding acid of general formula (Vlll') with thionyl chloride or oxalyl chloride, whereby said chlorinating agent is also used as the solvent. Also preferably an additional solvent may be used. Suitable solvents include hydrocarbons such as benzene, toluene or xylene, halogenated hydrocarbons such as dichloromethane, chloroform or carbon tetrachloride, ethers such as diethyl ether, dioxane, tetrahydrofurane or dimethoxyethane. Mixtures of two or more solvents from one class or two or more solvents from different classes may also be used. Preferred reaction temperature range from 0º C to the boiling point of the solvent and reaction times from several minutes to several hours.

If said activated compound of general formula (VIII') is a mixed anhydride, said anhydride may preferably be prepared, for example, by reaction of the corresponding acid of general formula (VIII') with ethyl chloroformiate in the presence of a base such as triethylamine or pyridine, in a suitable solvent.

Following that the activated compound can be reacted with an alkyl-alcohol to arrive at compounds according to general formulas II or III with $R^{1'}$ being a a linear or branched $C_{1-4}$-alkyl group.

During the processes described above the protection of sensitive groups or of reagents may be necessary and/or desirable. The introduction of conventional protective groups as well as their removal may be performed by methods well-known to those skilled in the art.

If the substituted pyrazoline compounds of general formula II or III themselves are obtained in form of a mixture of stereoisomers, particularly enantiomers or diastereomers, said mixtures may be separated by standard procedures known to those skilled in the art, e.g. chromatographic methods or fractionalized crystallization with chiral reagents. It is also possible to obtain pure stereoisomers via stereoselective synthesis.

In a further aspect the present invention also provides a process for the preparation of salts of substituted pyrazoline compounds of general formula II or III and stereoisomers thereof, wherein at least one compound of general formula II or III having at least one basic group is reacted with at least one inorganic and/or organic acid, preferably in the presence of a suitable reaction medium. Suitable reaction media include, for example, any of the ones given above. Suitable inorganic acids include hydrochloric acid, hydrobromic acid, phosphoric acid, sulfuric acid, nitric acid, suitable organic acids are e.g. citric acid, maleic acid, fumaric acid, tartaric acid, or derivatives thereof, p-toluenesulfonic acid, methanesulfonic acid or camphersulfonic acid.

In yet a further aspect the present invention also provides a process for the preparation of salts of substituted pyrazoline compounds of general formula II or III or stereoisomers thereof, wherein at least one compound of general formula II or III having at least one acidic group is reacted with one or more suitable bases, preferably in the presence of a suitable reaction medium. Suitable bases are e.g. hydroxides, carbonates or alkoxides, which include suitable cations, derived e.g. from alkaline metals, alkaline earth metals or organic cations, e.g. $[NH_nR_{4-n}]^+$, wherein n is 0, 1, 2, 3 or 4 and R represents a branched or unbranched $C_{1-4}$-alkyl-radical. Suitable reaction media are, for example, any of the ones given above.

Solvates, preferably hydrates, of the substituted pyrazoline compounds of general formula II or III, of corresponding stereoisomers, of corresponding N-oxides or of corresponding salts thereof may also be obtained by standard procedures known to those skilled in the art.

Substituted pyrazoline compounds of general formula II or III, which comprise nitrogen-atom containing saturated, unsaturated or aromatic rings may also be obtained in the form of their N-oxides by methods well known to those skilled in the art.

The purification and isolation of the inventive substituted pyrazoline compounds of general formula II or III, of a corresponding stereoisomer, or salt, or solvate or any intermediate thereof may, if required, be carried out by conventional methods known to those skilled in the art, e.g. chromatographic methods or recrystallization.

Thus, an other aspect of the present invention relates to a medicament comprising at least one substituted pyrazoline compound of general formula I,

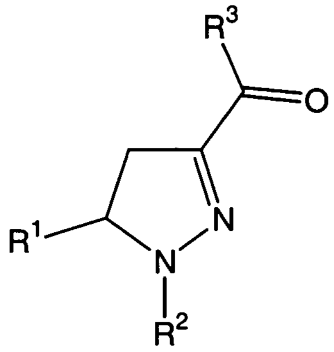

I

wherein

$R^1$ represents an optionally at least mono-substituted phenyl group,

$R^2$ represents an optionally at least mono-substituted phenyl group,

$R^3$ represents a saturated or unsaturated, optionally at least mono-substituted, optionally at least one heteroatom as ring member containing cycloaliphatic group, which may be condensed with an optionally at least mono-substituted mono- or polycyclic ring system, or $R^3$ represents an optionally at least mono-substituted aryl or heteroaryl group, which may be condensed with an optionally at least mono-substituted mono- or polycyclic ring system, or $R^3$ represents an $-NR^4R^5$-moiety,

$R^4$ and $R^5$, identical or different, represent a hydrogen atom, an unbranched or branched, saturated or unsaturated, optionally at least mono-substituted aliphatic radical, a saturated or unsaturated, optionally at least mono-substituted, optionally at least one heteroatom as ring member containing cycloaliphatic group, which may be condensed with an optionally at least mono-substituted mono- or polycyclic ring system, or an optionally at least mono-substituted aryl or heteroaryl group, which may be condensed with an optionally at least mono-substituted mono- or polycyclic ring system and/or bonded via a linear or branched alkylene group, an $-SO_2-R^6$-moiety, or an $-NR^7R^8$-moiety, with the proviso that $R^4$ and $R^5$ do not identically represent hydrogen,

$R^6$ represents a linear or branched, saturated or unsaturated, optionally at least mono-substituted aliphatic group, a saturated or unsaturated, optionally at least mono-substituted, optionally at least one heteroatom as ring member containing cycloaliphatic group, which may be condensed with a mono- or polycyclic ring-system, or an optionally at least mono-substituted aryl or heteroaryl group, which may be condensed with a mono- or polycyclic ring system and/or bonded via a linear or branched alkylene group,

$R^7$ and $R^8$, identical or different, represent a hydrogen atom, an unbranched or branched, saturated or unsaturated, optionally at least mono-substituted aliphatic radical, a saturated or unsaturated, optionally at least mono-substituted, optionally at least one heteroatom as ring member containing cycloaliphatic group, which may be condensed with an optionally at least mono-substituted mono- or polycyclic ring system, or an optionally at least mono-substituted aryl or heteroaryl group, which may be condensed with an optionally at least mono-substituted mono- or polycyclic ring system and/or bonded via a linear or branched alkylene group,

optionally in form of one of the stereoisomers, preferably enantiomers or diastereomers, a racemate or in form of a mixture of at least two of the stereoisomers, preferably enantiomers and/or diastereomers, in any mixing ratio, or a corresponding N-oxide thereof, or a corresponding salt thereof, or a corresponding solvate thereof, and optionally one or more pharmaceutically acceptable excipients.

A mono- or polycyclic ring-system according to the present invention means a mono-or polycyclic hydrocarbon ring-system that may be saturated, unsaturated or aromatic. If the ring system is polycyclic, each of its different rings may show a different degree of saturation, i.e. it may be saturated, unsaturated or aromatic. Optionally each of the rings of the mono- or polycyclic ring system may contain one or more, e.g. 1, 2 or 3, heteroatoms as ring members, which may be identical or different and which can preferably be selected from the group consisting of N, O, S and P, more preferably be selected from the group consisting of N, O and S.

Preferably the polycyclic ring-system may comprise two rings that are condensed. The rings of the mono- or polycyclic ring-sytem are preferably 5- or 6-membered. The term "condensed" according to the present invention means that a ring or ring-system is attached to another ring or ring-system, whereby the terms "annulated" or "annelated" are also used by those skilled in the art to designate this kind of attachment.

If one or more of the residues $R^3$-$R^8$ represents or comprises a saturated or unsaturated, optionally at least one heteroatom as ring member containing cycloaliphatic group, which is substituted by one or more, e.g. 1, 2, 3 or 4, substituents, unless defined otherwise, each of the substituents may be independently selected from the group consisting of hydroxy, fluorine, chlorine, bromine, branched or unbranched $C_{1-6}$-alkoxy, branched or unbranched $C_{1-6}$-alkyl, branched or unbranched $C_{1-4}$-perfluoroalkoxy, branched or unbranched $C_{1-4}$-perfluoroalkyl, oxo, amino, carboxy, amido, cyano, nitro, -$SO_2NH_2$, -CO-$C_{1-4}$-alkyl, - SO-$C_{1-4}$-alkyl, -$SO_2$-$C_{1-4}$-alkyl, -NH-$SO_2$-$C_{1-4}$-alkyl, wherein the $C_{1-4}$-alkyl may in each case be branched or unbranched, and a phenyl group, more preferably be selected from the group consisting of hydroxy, F, Cl, Br, methyl, ethyl, methoxy, ethoxy, oxo, $CF_3$ and a phenyl group.

If one or more of the residues $R^3$-$R^8$ represents or comprises a cycloaliphatic group, which contains one or more heteroatoms as ring members, unless defined otherwise, each of these heteroatoms may preferably be selected from the group consisting of of N, O and S. Preferably a cycloaliphatic group may contain 1, 2 or 3 heteroatoms independently selected from the group consisting of N, O and S as ring members.

Suitable saturated or unsaturated, optionally at least one heteroatom as ring member containing, optionally at least mono-substituted cycloaliphatic groups may preferably be selected from the group consisting of Cyclopropyl, Cyclobutyl, Cyclopentyl, Cyclohexyl, Cycloheptyl, Cyclooctyl, Cyclopentenyl, Cyclohexenyl, Cycloheptenyl, Cyclooctenyl, Pyrrolidinyl, Piperidinyl, Piperazinyl, homo-Piperazinyl and Morpholinyl.

If one or more of the residues $R^3$-$R^8$ comprises a mono- or polycyclic ring system, which is substituted by one or more, e.g. 1, 2, 3, 4 or 5 substituents, unless defined otherwise, each of the substituents may be independently selected from the group consisting of hydroxy, fluorine, chlorine, bromine, branched or unbranched $C_{1-6}$-alkoxy, branched or unbranched $C_{1-6}$-alkyl, branched or unbranched $C_{1-4}$-perfluoroalkoxy, branched or unbranched $C_{1-4}$-pertluoroalkyl, amino, carboxy, oxo, amido, cyano, nitro, -$SO_2NH_2$, -CO-$C_{1-4}$-alkyl, -SO-$C_{1-4}$-alkyl, -$SO_2$-$C_{1-4}$-alkyl, -NH-$SO_2$-$C_{1-4}$-alkyl , wherein the $C_{1-4}$-alkyl may in each case be branched or unbranched, and a phenyl group, more preferably be selected from the group consisting of hydroxy, F, Cl, Br, methyl, ethyl, methoxy, ethoxy, $CF_3$, oxo and a phenyl group.

If one or more of the residues $R^1$-$R^8$ represents or comprises an aryl group, including a phenyl group, which is substituted by one or more, e.g. 1, 2, 3, 4 or 5 substituents, unless defined otherwise, each of the substituents may be independently selected from the group consisting of a halogen atom (F, Cl, Br, I), a linear or branched $C_{1-6}$-alkyl group, a linear or branched $C_{1-6}$ alcoxy group, a formyl group, a hydroxy group, a trifluoromethyl group, a trifluoromethoxy group, a -CO-$C_{1-6}$-alkyl group, a cyano group, a nitro group, a carboxy group, a —CO-O-$C_{1-6}$-alkyl group, a —CO-$NR^AR^B$-moiety, a -CO-NH-$NR^CR^D$-moiety, an —SH, an -S-$C_{1-6}$-alkyl group, an -SO-$C_{1-6}$-alkyl group, an -$SO_2$-$C_{1-6}$-alkyl group, a -$C_{1-6}$-alkylene-S-$C_{1-6}$-alkyl group, a -$C_{1-6}$-alkylene-SO-$C_{1-6}$-alkyl group, a -$C_{1-6}$-alkylene-$SO_2$-$C_{1-6}$-alkyl group, an —$NH_2$-moiety, an NHR'-moiety or an NR'R"-moiety, wherein R' and R" independently represent a linear or branched $C_{1-6}$-alkyl group, a $C_{1-6}$-alkyl group substituted by one or more hydroxy groups and a —$C_{1-6}$-alkylene-$NR^ER^F$ group, whereby $R^A$, $R^B$, identical or different, represent hydrogen or a $C_{1-6}$-alkyl group, or $R^A$ and $R^B$ together with the bridging nitrogen atom form a saturated, mono- or bicyclic, 3-10 membered heterocyclic ring system, which may be at least mono-substituted by one or more, identical or different, $C_{1-6}$ alkyl groups and/or which may contain at least one further heteroatom selected from the group consisting of nitrogen, oxygen and sulphur as a ring member,

$R^C$, $R^D$, identical or different, represent a hydrogen atom, a $C_{1-6}$-alkyl group, a -CO-O-$C_{1-6}$-alkyl group, a $C_{3-8}$-cycloalkyl group, a $C_{1-6}$-alkylene-$C_{3-8}$-cycloalkyl group, $C_{1-6}$-alkylene-O-$C_{1-6}$-alkyl group or a $C_{1-6}$-alkyl group substituted with one or more hydroxy groups, or $R^C$, $R^D$ together with the bridging nitrogen atom form a saturated, mono- or bicyclic, 3-10 membered heterocyclic ring system, which may be at least mono-substituted by one or more substituents independently selected from the group consisting of $C_{1-6}$ alkyl group, a -CO-$C_{1-6}$-alkyl group, a —CO-O- $C_{1-6}$-alkyl group, a - CO-NH-$C_{1-6}$-alkyl group, a -CS-NH- $C_{1-6}$-alkyl group, an oxo group, a $C_{1-6}$-alkyl group substituted with one or more hydroxy groups, a $C_{1-6}$-alkylene-O-$C_{1-6}$-alkyl group and a —CO-$NH_2$ group and/or which may contain at least one further heteroatom selected from the group consisting of nitrogen, oxygen and sulphur as a ring member, and

wherein $R^E$, $R^F$, identical or different, represent hydrogen or a $C_{1-6}$-alkyl group, or $R^E$ and $R^F$ together with the bridging nitrogen atom form a saturated, mono- or bicyclic, 3-10 membered heterocyclic ring system, which may be at least mono-substituted by one or more, identical or different $C_{1-6}$ alkyl groups and/or which may contain at least one further heteroatom selected from the group consisting of nitrogen, oxygen and sulphur as a ring member.

Preferred aryl groups, which may optionally be at least mono-substituted, are phenyl and naphthyl.

If one or more of the residues $R^3$-$R^8$ represents or comprises a heteroaryl group, which is substituted by one or more, e.g. 1, 2, 3, 4 or 5 substituents, unless defined otherwise, each of the substituents may be independently selected from the group consisting of a halogen atom (e.g. F, Cl, Br, I), a linear or branched $C_{1-6}$-alkyl group, a linear or branched $C_{1-6}$ alcoxy group, a formyl group, a hydroxy group, a trifluoromethyl group, a trifluoromethoxy group, a -CO-$C_{1-6}$-alkyl group, a cyano group, a carboxy group, a —CO-O-$C_{1-6}$-alkyl group, a —CO-$NR^AR^B$- moiety, a -CO-NH-

$NR^CR^D$-moiety, an -S-$C_{1-6}$-alkyl group, an -SO-$C_{1-6}$-alkyl group, an -$SO_2$-$C_{1-6}$-alkyl group, a -$C_{1-6}$-alkylene-S-$C_{1-6}$-alkyl group, a -$C_{1-6}$-alkylene-SO-$C_{1-6}$-alkyl group, a -$C_{1-6}$-alkylene-$SO_2$-$C_{1-6}$-alkyl group, a $C_{1-6}$-alkyl group substituted by one or more hydroxy groups and a —$C_{1-6}$-alkylene-$NR^ER^F$ group,

whereby $R^A$, $R^B$, identical or different, represent hydrogen or a $C_{1-6}$-alkyl group, or $R^A$ and $R^B$ together with the bridging nitrogen atom form a saturated, mono- or bicyclic, 3-10 membered heterocyclic ring system, which may be at least mono-substituted by one or more, identical or different, $C_{1-6}$ alkyl groups and/or which may contain at least one further heteroatom selected from the group consisting of nitrogen, oxygen and sulphur as a ring member,

$R^C$, $R^D$, identical or different, represent a hydrogen atom, a $C_{1-6}$-alkyl group, a -CO-O-$C_{1-6}$-alkyl group, a $C_{3-8}$-cycloalkyl group, a $C_{1-6}$-alkylene-$C_{3-8}$-cycloalkyl group, $C_{1-6}$-alkylene-O-$C_{1-6}$-alkyl group or a $C_{1-6}$-alkyl group substituted with one or more hydroxy groups, or $R^C$, $R^D$ together with the bridging nitrogen atom form a saturated, mono- or bicyclic, 3-10 membered heterocyclic ring system, which may be at least mono-substituted by one or more substituents independently selected from the group consisting of $C_{1-6}$ alkyl group, a -CO-$C_{1-6}$-alkyl group, a -CO-O- $C_{1-6}$-alkyl group, a - CO-NH-$C_{1-6}$-alkyl group, a -CS-NH- $C_{1-6}$-alkyl group, an oxo group, a $C_{1-6}$-alkyl group substituted with one or more hydroxy groups, a $C_{1-6}$-alkylene-O-$C_{1-6}$-alkyl group and a -CO-$NH_2$ group and/or which may contain at least one further heteroatom selected from the group consisting of nitrogen, oxygen and sulphur as a ring member, and

wherein $R^E$, $R^F$, identical or different, represent hydrogen or a $C_{1-6}$-alkyl group, or $R^E$ and $R^F$ together with the bridging nitrogen atom form a saturated, mono- or bicyclic, 3-10 membered heterocyclic ring system, which may be at least mono-substituted by one or more, identical or different $C_{1-6}$ alkyl groups and/or which may contain at least one further heteroatom selected from the group consisting of nitrogen, oxygen and sulphur as a ring member,

The heteroatoms, which are present as ring members in the heteroaryl radical, may, unless defined otherwise, independently be selected from the group consisting of nitrogen, oxygen and sulphur. Preferably a heteroaryl radical may comprise 1, 2 or 3 heteroatoms independently selected from the group consisting of N, O and S as ring members.

Suitable heteroaryl groups, which may optionally be at least mono-substituted, may preferably be selected from the group consisting of thienyl, furyl, pyrrolyl, pyridinyl, imidazolyl, pyrimidinyl, pyrazinyl, indolyl, chinolinyl, isochinolinyl, benzo[1,2,5]-thiodiazolyl, benzo[b]thiophenyl, benzo[b]furanyl, imidazo[2,1-b]thiazolyl, triazolyl, and pyrazolyl, more preferably be selected from the group consisting of thienyl-, benzo[1,2,5]-thiodiazolyl, benzo[b]thiophenyl, imidazo [2,1-b]thiazolyl, triazolyl and pyrazolyl.

If one or more of the residues $R^4$-$R^8$ represents or comprises a linear or branched, saturated or unsaturated aliphatic group such as an alkyl group, which is substituted by one or more, e.g. 1, 2, 3, 4 or 5 substituents, unless defined otherwise, each of the substituents may be independently selected from the group consisting of hydroxy, fluorine, chlorine, bromine, branched or unbranched $C_{1-4}$-alkoxy, branched or unbranched $C_{1-4}$-perfluoroalkoxy, branched or unbranched $C_{1-4}$-perfluoroalkyl, amino, carboxy, amido, cyano, nitro, -$SO_2NH_2$, -CO-$C_{1-4}$-alkyl, -SO-$C_{1-4}$-alkyl, -$SO_2$-$C_{1-4}$-alkyl, -NH-$SO_2$-$C_{1-4}$-alkyl , wherein the $C_{1-4}$-alkyl may in each case be branched or unbranched, and a phenyl group, more preferably be selected from the group consisting of hydroxy, F, Cl, Br, methoxy, ethoxy, $CF_3$ and a phenyl group.

Preferred linear or branched, saturated or unsaturated aliphatic groups, which may be substituted by one or more substituents, may preferably be selected from the group consisting of methyl, ethyl, n-propyl, isopropyl, n-butyl, iso-butyl, sec-butyl, tert-butyl, n-pentyl, n-hexyl, n-heptyl, n-octyl, n-nonyl, n-decyl, vinyl, ethinyl, propenyl, propinyl, butenyl and butinyl.

If any of the residues $R^4$-$R^8$ represents or comprises a linear or branched alkylene group, said alkylene group may preferably be selected from the group consisting of — methylene -($CH_2$)-, ethylene -($CH_2$-$CH_2$)-, n-propylene -($CH_2$-$CH_2$-$CH_2$)- or iso-proylene —(-C($CH_3$)$_2$)-.

Particularly preferred is a medicament comprising at least one compound of general formula I

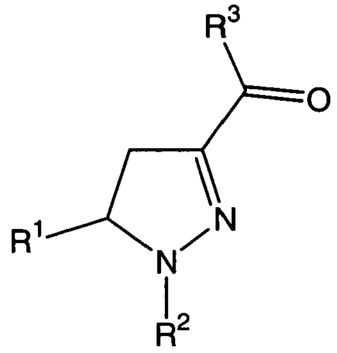

I

wherein

$R^1$ represents a phenyl group, which is optionally substituted with 1, 2, 3, 4 or 5 substituents independently selected from the group consisting of linear or branched $C_{1-6}$-alkyl, linear or branched $C_{1-6}$-alkoxy, F, Cl, Br, I, $CH_2F$, $CHF_2$, $CF_3$, CN, OH, $NO_2$, -(C=O)-R', SH, SR', SOR', $SO_2R'$ $NH_2$, NHR', NR'R", -(C=O)-$NH_2$, -(C=O)-NHR' and —(C=O)-NR'R", whereby R' and R" at each ocurrence independently represent a linear or branched $C_{1-6}$ alkyl group,

$R^2$ represents a phenyl group, which is optionally substituted with 1, 2, 3, 4 or 5 substituents independently selected from the group consisting of linear or branched $C_{1-6}$-alkyl, linear or branched $C_{1-6}$-alkoxy, F, Cl, Br, i, $CH_2F$, $CHF_2$, $CF_3$, CN, OH, $NO_2$, -(C=O)-R', SH, SR', SOR', $SO_2R'$, $NH_2$, NHR', NR'R", -(C=O)-$NH_2$, -(C=O)-NHR' and -(C=O)-NR'R", whereby R' and R" at each ocurrence independently represent a linear or branched $C_{1-6}$ alkyl group,

$R^3$ represents a saturated or unsaturated $C_{3-8}$ cycloaliphatic group, whereby said $C_{3-8}$ cycloaliphatic group is optionally substituted with 1, 2, 3 or 4 substituents independently selected from the group consisting of linear or branched $C_{1-6}$ alkyl, linear or branched $C_{1-6}$ alkoxy, OH, F, Cl, Br, I, CN, $CH_2F$, $CHF_2$, $CF_3$ and oxo (=O) and whereby said $C_{3-8}$ cycloaliphatic group may contain 1, 2 or 3 heteroatoms independently selected from the group consisting of N, O and S as ring members, or $R^3$ represents an —$NR^4R^5$-moiety,

$R^4$ represents a hydrogen atom or a linear or branched $C_{1-6}$-alkyl group,

$R^5$ represents a linear or branched $C_{1-6}$ alkyl group; an -$SO_2$-$R^6$-moiety; a saturated or unsaturated $C_{3-8}$ cycloaliphatic group, whereby said $C_{3-8}$ cycloaliphatic group is optionally substituted with 1, 2, 3 or 4 substituents independently selected from the group consisting of linear or branched $C_{1-6}$ alkyl group, a linear or branched $C_{1-6}$ alkoxy group, OH, F, Cl, Br, I, CN $CH_2F$, $CHF_2$, $CF_3$ and oxo (=O) and whereby said $C_{3-8}$ cycloaliphatic group may contain 1, 2 or 3 heteroatoms independently selected from the group consisting of N, O and S as ring members, and

$R^6$ represents a phenyl group, which is optionally substituted with 1, 2, 3, 4 or 5 substituents independently selected from the group consisting of a linear or branched $C_{1-6}$-alkyl group, a linear or branched $C_{1-6}$-alkoxy group, F, Cl, Br, I, $CH_2F$, $CHF_2$, $CF_3$, CN, OH, $NO_2$, -(C=O)-R', SH, SR', SOR', $SO_2R'$, $NH_2$, NHR', NR'R", -(C=O)-$NH_2$, -(C=O)-NHR' and -(C=O)-NR'R", whereby R' and R" at each ocurrence independently represent a linear or branched $C_{1-6}$ alkyl group,

optionally in form of one of the stereoisomers, preferably enantiomers or diastereomers, a racemate or in form of a mixture of at least two of the stereoisomers, preferably enantiomers and/or diastereomers, in any mixing ratio, or a corresponding N-oxide thereof, or a corresponding salt thereof, or a corresponding solvate thereof.

Furthermore a medicament is particularly preferred, which comprises at least one compound of general formula I given below,

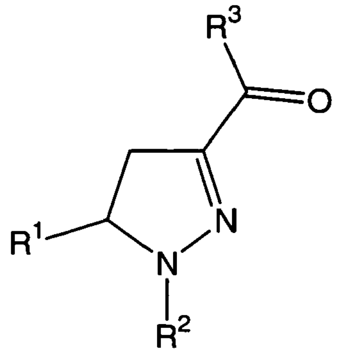

I

wherein

$R^1$ represents a phenyl ring, which is mono-substituted with a halogen atom, preferably a chlorine atom, in its 4-position,

$R^2$ represents a phenyl ring, which is di-substituted with two halogen atoms, preferably chlorine atoms, in its 2- and 4-position,

$R^3$ represents a pyrrolidinyl group, a piperidinyl group, a piperazinyl group, a homo-piperazinyl group, a morpholinyl group, or an $-NR^4R^5$-moiety,

$R^4$ represents a hydrogen atom or a linear or branched $C_{1-6}$-alkyl group,

$R^5$ represents a linear or branched $C_{1-6}$ alkyl group, a pyrrolidinyl group, a piperidinyl group, a piperazinyl group, a homo-piperazinyl group, a morpholinyl group, a triazolyl group, whereby each of the heterocyclic rings may be substituted with one or more, identical or different, $C_{1-6}$-alkyl groups, or an $-SO_2-R^6$-moiety, and

$R^6$ represents a phenyl group, which is optionally substituted with one or more $C_{1-6}$ alkyl groups, which may be identical or different,

optionally in form of one of the stereoisomers, preferably enantiomers or diastereomers, a racemate or in form of a mixture of at least two of the stereoisomers, preferably enantiomers and/or diastereomers, in any mixing ratio, or a corresponding N-oxide thereof, or a corresponding salt thereof, or a corresponding solvate thereof.

Most particularly preferred is a medicament comprising at least one substituted pyrazoline compound selected from the group consisting of:

N-piperidinyl-5-(4-chloro-phenyl)-1-(2,4-dichlorophenyl)-4,5-dihydro-1H-pyrazole-3-carboxamide,

5-(4-Chloro-phenyl)-1-(2,4-dichloro-phenyl)-4,5-dihydro-1H-pyrazole-3-carboxylic acid-[1,2,4]-triazole-4-yl-amide,

5-(4-Chloro-phenyl)-1-(2,4-dichloro-phenyl)-4,5-dihydro-1H-pyrazole-3-carboxylic acid-(4-methyl-piperazin-1-yl)-amide,

5-(4-Chloro-phenyl)-1-(2,4-dichloro-phenyl)-4,5-dihydro-1H-pyrazole-3-carboxylic acid diethylamide,

[5-(4-Chloro-phenyl)-1-(2,4-dichloro-phenyl)-4,5-dihydro-1H-pyrazole-3-yl]-piperidine-1-yl-methanone,

N-[5-(4-Chloro-phenyl)-1-(2,4-dichlorophenyl)-4,5-dihydro-1H-pyrazole-3-carbonyl]-4-methylphenylsufonamide,

optionally in the form of a corresponding N-oxide, or a corresponding salt, or a corresponding solvate.

The inventive medicament may preferably also comprise any of the inventive pyrazoline compounds or combinations of at least two of these pyrazoline compounds given above.

Said medicament may also comprise any combination of one or more of the substituted pyrazoline compounds of general formula I given above, stereoisomers thereof, corresponding N-oxides thereof, physiologically acceptable salts thereof or physiologically acceptable solvates thereof.

Preferably said medicament is suitable for the modulation (regulation) of cannabinoid-receptors, preferably cannabinoid 1 ($CB_1$) receptors, for the prophylaxis and/or treatment of disorders of the central nervous system, disorders of the immune system, disorders of the cardiovascular system, disorders of the endocrinous system, disorders of the

respiratory system, disorders of the gastrointestinal tract or reproductive disorders.

Particularly preferably said medicament is suitable for the prophylaxis and/or treatment of psychosis.

Also particularly preferably said medicament is suitable for the prophylaxis and/or treatment of food intake disorders, preferably bulimia, anorexia, cachexia, obesity and/or type II diabetus mellitus (non-insuline dependent diabetes mellitus), more preferably obesity. The inventive medicament also seems to be active in the prophylaxis and/or treatment of appetency disorders, e.g. the pyrazoline compounds of general formula I also reduce the desire for sweets.

Also particularly preferably said medicament is suitable for the prophylaxis and/or treatment of cancer, preferably for the prophylaxis and/or treatment of one or more types of cancer selected from the group consisting of brain cancer, bone cancer, lip cancer, mouth cancer, esophageal cancer, stomach cancer, liver cancer, bladder cancer, pancreas cancer, ovary cancer, cervical cancer, lung cancer, breast cancer, skin cancer, colon cancer, bowel cancer and prostate cancer, more preferably for the prophylaxis and/or treatment of one or more types of cancer selected from the group consisting of colon cancer, bowel cancer and prostate cancer.

Particularly preferably said medicament is suitable for the prophylaxis and/or treatment of alcohol abuse and/or alcohol addiction, nicotine abuse and/or nicotine addiction, drug abuse and/or drug addiction and/or medicament abuse and/or medicament addiction, preferably drug abuse and/or drug addiction and/or nicotine abuse and/or nicotine addiction.

Medicaments and/or drugs, which are frequently the subject of misuse include opioids, barbiturates, cannabis, cocaine, amphetamines, phencyclidine, hallucinogens and benzodiazepines.

The medicament is also suitable for the prophylaxis and/or treatment of one or more disorders selected from the group consisting of bone disorders, preferably osteoporosis (e.g. osteoporosis associated with a genetic predisposition, sex hormone deficiency, or ageing), cancer-associated bone disease or Paget's disease of bone; schizophrenia, anxiety, depression, epilepsy, neurodegenerative disorders, cerebellar disorders, spinocerebellar disorders, cognitive disorders, cranial trauma, head trauma, stroke, panic attacks, peripheric neuropathy, inflammation, glaucoma, migraine, Morbus Parkinson, Morbus Huntington, Morbus Alzheimer, Raynaud's disease, tremblement disorders, compulsive disorders, senile dementia, thymic disorders, tardive dyskinesia, bipolar disorders, medicament-induced movement disorders, dystonia, endotoxemic shock, hemorragic shock, hypotension, insomnia, immunologic disorders, sclerotic plaques, vomiting, diarrhea, asthma, memory disorders, pruritus, pain, or for potentiation of the analgesic effect of narcotic and non-narcotic analgesics, or for influencing intestinal transit.

Another aspect of the present invention is the use of at least one substituted pyrazoline compound of general formula I given above as suitable active substances, optionally in form of one of the stereoisomers, preferably enantiomers or diastereomers, a racemate or in form of a mixture of at least two of the stereoisomers, preferably enantiomers and/or diastereomers, in any mixing ratio, or a corresponding N-oxide thereof, or a corresponding salt thereof, or a corresponding solvate thereof, and optionally one or more pharmaceutically acceptable excipients, for the preparation of a medicament for the modulation of cannabinoid-receptors, preferably cannabinoid 1 ($CB_1$) receptors, for the prophylaxis and/or treatment of disorders of the central nervous system, disorders of the immune system, disorders of the cardiovascular system, disorders of the endocrinous system, disorders of the respiratory system, disorders of the gastrointestinal tract or reproductive disorders.

Particularly preferred is the use of at least one of the respective pyrazoline compounds, optionally in form of one of the stereoisomers, preferably enantiomers or diastereomers, a racemate or in form of a mixture of at least two of the stereoisomers, preferably enantiomers and/or diastereomers, in any mixing ratio, or a corresponding N-oxide thereof, or a corresponding salt thereof, or a corresponding solvate thereof, and optionally one or more pharmaceutically acceptable excipients, for the preparation of a medicament for the prophylaxis and/or treatment of psychosis.

Also particularly preferred is the use of at least one of the respective pyrazoline compounds, optionally in form of one of the stereoisomers, preferably enantiomers or diastereomers, a racemate or in form of a mixture of at least two of the stereoisomers, preferably enantiomers and/or diastereomers, in any mixing ratio, or a corresponding N-oxide thereof, or a corresponding salt thereof, or a corresponding solvate thereof, and optionally one or more pharmaceutically acceptable excipients, for the preparation of a medicament for the prophylaxis and/or treatment of food intake disorders, preferably bulimia, anorexia, cachexia, obesity and/or type II diabetus mellitus (non-insuline dependent diabetes mellitus), more preferably obesity.

Also particularly preferred is the use of at least one of the respective pyrazoline compounds, optionally in form of one of the stereoisomers, preferably enantiomers or diastereomers, a racemate or in form of a mixture of at least two of the stereoisomers, preferably enantiomers and/or diastereomers, in any mixing ratio, or a corresponding N-oxide thereof, or a corresponding salt thereof, or a corresponding solvate thereof, and optionally one or more pharmaceutically acceptable excipients, for the preparation of a medicament for the prophylaxis and/or treatment of cancer, preferably for the prophylaxis and/or treatment of one or more types of cancer selected from the group consisting of brain cancer, bone cancer, lip cancer, mouth cancer, esophageal cancer, stomach cancer, liver cancer, bladder cancer, pancreas cancer, ovary cancer, cervical cancer, lung cancer, breast cancer, skin cancer, colon cancer, bowel cancer and prostate cancer, more preferably for the prophylaxis and/or treatment of one or more types of cancer selected from the group

consisting of colon cancer, bowel cancer and prostate cancer.

Also particularly preferred is the use of at least one of the respective pyrazoline compounds, optionally in form of one of the stereoisomers, preferably enantiomers or diastereomers, a racemate or in form of a mixture of at least two of the stereoisomers, preferably enantiomers and/or diastereomers, in any mixing ratio, or a corresponding N-oxide thereof, or a corresponding salt thereof, or a corresponding solvate thereof, and optionally one or more pharmaceutically acceptable excipients, for the preparation of a medicament for the prophylaxis and/or treatment of alcohol abuse and/or alcohol addiction, nicotine abuse and/or nicotine addiction, drug abuse and/or drug addiction and/or medicament abuse and/or medicament addiction, preferably drug abuse and/or drug addiction and/or nicotine abuse and/or nicotine addiction.

Medicaments/drugs, which are frequently the subject of misuse include opioids, barbiturates, cannabis, cocaine, amphetamines, phencyclidine, hallucinogens and benzodiazepines.

Also preferred is the use of at least one of the respective pyrazoline compounds, optionally in form of one of the stereoisomers, preferably enantiomers or diastereomers, a racemate or in form of a mixture of at least two of the stereoisomers, preferably enantiomers and/or diastereomers, in any mixing ratio, or a corresponding N-oxide thereof, or a corresponding salt thereof, or a corresponding solvate thereof, and optionally one or more pharmaceutically acceptable excipients, for the preparation of a medicament for the prophylaxis and/or treatment of one or more disorders selected from the group consisting of bone disorders, preferably osteoporosis (e.g. osteoporosis associated with a genetic predisposition, sex hormone deficiency, or ageing), cancer-associated bone disease or Paget's disease of bone; schizophrenia, anxiety, depression, epilepsy, neurodegenerative disorders, cerebellar disorders, spinocerebellar disorders, cognitive disorders, cranial trauma, head trauma, stroke, panic attacks, peripheric neuropathy, inflammation, glaucoma, migraine, Morbus Parkinson, Morbus Huntington, Morbus Alzheimer, Raynaud's disease, tremblement disorders, compulsive disorders, senile dementia, thymic disorders, tardive dyskinesia, bipolar disorders, , medicament-induced movement disorders, dystonia, endotoxemic shock, hemorragic shock, hypotension, insomnia, immunologic disorders, sclerotic plaques, vomiting, diarrhea, asthma, memory disorders, pruritus, pain, or for potentiation of the analgesic effect of narcotic and non-narcotic analgesics, or for influencing intestinal transit.

The medicament according to the present invention may be in any form suitable for the application to humans and/or animals, preferably humans including infants, children and adults and can be produced by standard procedures known to those skilled in the art. The composition of the medicament may vary depending on the route of administration.

The medicament of the present invention may for example be administered parentally in combination with conventional injectable liquid carriers, such as water or suitable alcohols. Conventional pharmaceutical excipients for injection, such as stabilizing agents, solubilizing agents, and buffers, may be included in such injectable compositions. These medicaments may for example be injected intramuscularly, intraperitoneally, or intravenously.

Thus, another aspect of the present invention relates to a Medicament comprising at least one substituted pyrazoline compound of general formula II or III according to the invention and optionally one or more pharmaceutically acceptable excipients.

Thus, another aspect of the present invention relates to a Medicament comprising at least one substituted pyrazoline compound of general formula II

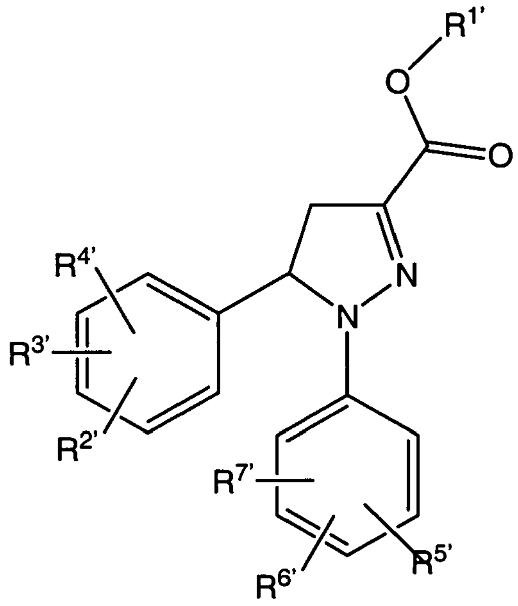

II

wherein
$R^1$ represents hydrogen or a linear or branched $C_{1-4}$-alkyl group,
$R^{2'}$, $R^{3'}$ and $R^{4'}$ independently of each other represent hydrogen, a linear or branched $C_{1-6}$-alkyl group, a linear or branched $C_{1-6}$-alkoxy group, a halogen
atom, $CH_2F$, $CHF_2$, $CF_3$, CN, OH, $NO_2$, -(C=O)-$R^{8'}$, SH, $SR^{8'}$, $SOR^{8'}$, $SO_2R^{8'}$, $NH_2$, $NHR^{8'}$, $NR^{8'}R^{9'}$, -(C=O)-$NH_2$, -(C=O)-$NHR^{8'}$ or -(C=O)-$NR^{8'}R^{9'}$ whereby $R^{8'}$ and $R^{9'}$ for each substituent independently represent linear or branched $C_{1-6}$ alkyl,
$R^{5'}$, $R^{6'}$ and $R^{7'}$ independently of each other represent hydrogen, a linear or branched $C_{1-6}$-alkyl group, a linear or branched $C_{1-6}$-alkoxy group, a halogen
atom, $CH_2F$, $CHF_2$, $CF_3$, CN, OH, $NO_2$, -(C=O)-$R^{10'}$, SH, $SR^{10'}$, $SOR^{10'}$, $NH_2$, $NHR^{10'}$, $NR^{10'}R^{11'}$, -(C=O)-$NH_2$, -(C=O)-$NHR^{10'}$ and -(C=O)-$NR^{10'}R^{11'}$, whereby $R^{10'}$ and optionally $R^{11'}$ for each substituent independently represent linear or branched $C_{1-6}$ alkyl;
optionally in form of one of the stereoisomers, preferably enantiomers or diastereomers, a racemate or in form of a mixture of at least two of the stereoisomers, preferably enantiomers and/or diastereomers, in any mixing ratio, or a corresponding N-oxide thereof, or a corresponding salt thereof, or a corresponding solvate thereof;
and optionally one or more pharmaceutically acceptable excipients.
In an embodiment of the medicament according to the invention for the compound according to formula II at least one of $R^{2'}$, $R^{3'}$ or $R^{4'}$ represents hydrogen, while at least one of $R^{2'}$, $R^{3'}$ or $R^{4'}$ is different from hydrogen.

In an embodiment of the medicament according to the invention for the compound according to formula II at least one of $R^{5'}$, $R^{6'}$ or $R^{7'}$ represents hydrogen, while at least one $R^{5'}$, $R^{6'}$ or $R^{7'}$ is different from hydrogen.

In an embodiment of the medicament according to the invention for the compound according to formula II $R^{2'}$, $R^{3'}$ and $R^{4'}$ independently of each other represent hydrogen, a linear or branched $C_{1-6}$-alkyl group, a halogen atom, or $CF_3$, preferably $R^{2'}$, $R^{3'}$ and $R^{4'}$ independently of each other represent hydrogen, methyl, ethyl, F, Cl, Br and $CF_3$.

In an embodiment of the medicament according to the invention for the compound according to formula II $R^{5'}$, $R^{6'}$ and $R^{7'}$ independently of each other represent hydrogen, a linear or branched $C_{1-6}$-alkyl group, a halogen atom, or $CF_3$, preferably $R^{5'}$, $R^{6'}$ and $R^{7'}$ independently of each other represent hydrogen, methyl, ethyl, F, Cl, Br and $CF_3$.

In an embodiment of the medicament according to the invention for the compound according to formula II $R^{2'}$ represents a chlorine atom in the 4-position of the phenyl ring, while $R^{3'}$ and $R^{4'}$ represent hydrogen.

In an embodiment of the medicament according to the invention for the compound according to formula II $R^{5'}$ and $R^{6'}$ each represent a chlorine atoms in the 2- and 4-position of the phenyl ring, while $R^{7'}$ represents hydrogen.

In an embodiment of the medicament according to the invention for the compound according to formula II $R^{1'}$ represents hydrogen, methyl or ethyl, preferably hydrogen.

In an embodiment of the medicament according to the invention the compound according to formula II is represented by a compound of general formula (III)

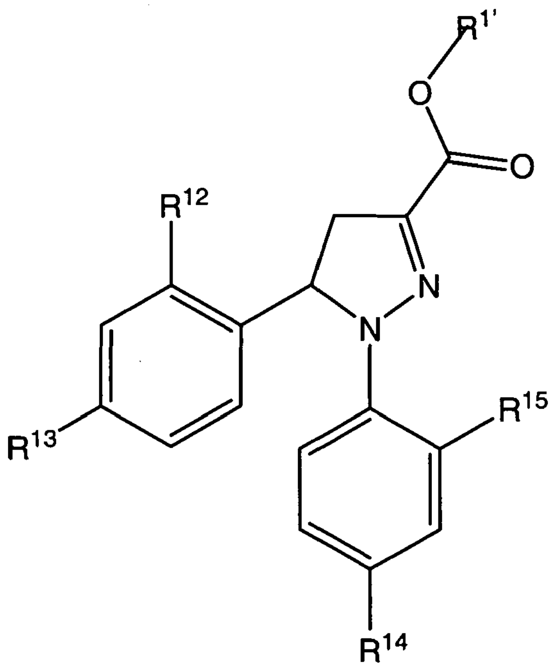

II

III

wherein

$R^{1'}$ represents hydrogen or a linear or branched $C_{1-4}$-alkyl group,

$R^{12}$, $R^{13}$, $R^{14}$ or $R^{15}$ independently of each other represent a linear or branched $C_{1-6}$-alkyl group, a linear or branched $C_{1-6}$-alkoxy group, a halogen atom, $CH_2F$, $CHF_2$, $CF_3$, CN, OH, $NO_2$, SH, $NH_2$, hydrogen, methyl, ethyl, F, Cl, Br and $CF_3$, optionally in form of one of the stereoisomers, preferably enantiomers or diastereomers, a racemate or in form of a mixture of at least two of the stereoisomers, preferably enantiomers and/or diastereomers, in any mixing ratio, or a corresponding N-oxide thereof, or a corresponding salt thereof, or a corresponding solvate thereof.

In an embodiment of the medicament according to the invention for the compound according to formula III $R^{12}$ and $R^{13}$ independently of each other represent hydrogen, a linear or branched$C_{1-6}$-alkyl group, a halogen atom, or $CF_3$, preferably $R^{12}$ and $R^{13}$ independently of each other represent hydrogen, methyl, ethyl, F, Cl, Br and $CF_3$.

In an embodiment of the medicament according to the invention for the compound according to formula III $R^{14}$, and $R^{15}$ independently of each other represent hydrogen, a linear or branched $C_{1-6}$-alkyl group, a halogen atom, or $CF_3$, preferably $R^{14}$ and $R^{15}$ independently of each other represent hydrogen, methyl, ethyl, F, Cl, Br and $CF_3$.

In an embodiment of the medicament according to the invention for the compound according to formula III $R^{13}$ represents Cl and $R^{12}$ represents hydrogen.

In an embodiment of the medicament according to the invention for the compound according to formula III $R^{14}$ and $R^{15}$ each represent Cl.

In an embodiment of the medicament according to the invention for the compound according to formula III $R^{1'}$ represents hydrogen, methyl or ethyl, preferably hydrogen.

In an embodiment of the medicament according to the invention the compound according to formulas II or III is selected from the group consisting of:

5-(4-chloro-phenyl)-1-(2,4-dichlorophenyl)-4,5-dihydro-1H-pyrazol-3-carboxylic acid,

optionally in the form of a corresponding N-oxide, a corresponding salt or a corresponding solvate.

Another aspect of the invention is a medicament comprising at least one combination of compounds according to the invention and optionally one or more pharmaceutically acceptable excipients.

In an embodiment of the medicaments according to the invention the medicament is for the regulation of triglyceride levels in the blood plasma and for the prophylaxis and/or treatment of disorders of the central nervous system,

especially stroke, of disorders of the cardiovascular system and of of food intake disorders, preferably bulimia, anorexia, cachexia, obesity, type II diabetus mellitus (non-insuline dependent diabetes mellitus), preferably obesity and diabetis.

In an embodiment of the medicament comprising the compound according to formula II or III according to the invention the medicament is for the prophylaxis and/or treatment of disorders of the central nervous system, disorders of the immune system, disorders of the cardiovascular system, disorders of the endocrinous system, disorders of the respiratory system, disorders of the gastrointestinal tract or reproductive disorders.

In an embodiment of the medicament comprising the combination according to the invention the medicament is for the modulation of cannabinoid-receptors, preferably cannabinoid 1 ($CB_1$) receptors, for the prophylaxis and/or treatment of disorders of the central nervous system, disorders of the immune system, disorders of the cardiovascular system, disorders of the endocrinous system, disorders of the respiratory system, disorders of the gastrointestinal tract or reproductive disorders.

In an embodiment of the medicaments according to the invention the medicament is for the prophylaxis and/or treatment of food intake disorders, preferably bulimia, anorexia, cachexia, obesity, type II diabetus mellitus (non-insuline dependent diabetes mellitus), preferably obesity.

In an embodiment of the medicaments according to the invention the medicament is for the prophylaxis and/or treatment of psychosis.

In an embodiment of the medicaments according to the invention the medicament is for the prophylaxis and/or treatment of alcohol abuse and/or addiction, nicotine abuse and/or addiction, drug abuse and/or addiction and/or medicament abuse and/or addiction, preferably drug abuse and/or addiction and/or nicotine abuse and/or addiction.

In an embodiment of the medicaments according to the invention the medicament is for the prophylaxis and/or treatment of one or more disorders selected from the group consisting of schizophrenia, anxiety, depression, epilepsy, neurodegenerative disorders, cerebellar disorders, spinocerebellar disorders, cognitive disorders, cranial trauma, panic attacks, peripheric neuropathy, glaucoma, migraine, Morbus Parkinson, Morbus Huntington, Morbus Alzheimer, Raynaud's disease, tremblement disorders, compulsive disorders, senile dementia, thymic disorders, tardive dyskinesia, bipolar disorders; bone disorders including osteoporosis or Paget's disease of bone; cancer, preferably for the prophylaxis and/or treatment of one or more types of cancer selected from the group consisting of brain cancer, bone cancer, lip cancer, mouth cancer, esophageal cancer, stomach cancer, liver cancer, bladder cancer, pancreas cancer, ovary cancer, cervical cancer, lung cancer, breast cancer, skin camcer, colon cancer, bowl cancer and prostate cancer, more preferably for the prophylaxis and/or treatment of one or more types of cancer selected from the group consisting of colcon cancer, bowel cancer and prostate cancer; medicament-induced movement disorders, dystonia, endotoxemic shock, hemorragic shock, hypotension, insomnia, immunologic disorders, sclerotic plaques, vomiting, diarrhea, asthma, memory disorders, pruritus, pain, or for potentiation of the analgesic effect of narcotic and non-narcotic analgesics, or for influencing intestinal transit.

Said medicaments may also comprise any combination of one or more of the substituted pyrazoline compounds of general formula I given above, stereoisomers thereof, corresponding N-oxides thereof, physiologically acceptable salts thereof or physiologically acceptable solvates thereof.

Particularly preferably said medicaments are suitable for the prophylaxis and/or treatment of alcohol abuse, drug abuse and/or medicament abuse, preferably drug abuse and the treatment of obesity.

Medicaments and/or drugs, which are frequently the subject of misuse include opioids, barbiturates, cannabis, cocaine, amphetamines, phencyclidine, hallucinogens and benzodiazepines.

The medicament according to the present invention may be in any form suitable for the application to humans and/or animals, preferably humans including infants, children and adults and can be produced by standard procedures known to those skilled in the art. The composition of the medicament may vary depending on the route of administration.

The medicament of the present invention may for example be administered parentally in combination with conventional injectable liquid carriers, such as water or suitable alcohols. Conventional pharmaceutical excipients for injection, such as stabilizing agents, solubilizing agents, and buffers, may be included in such injectable compositions. These medicaments may for example be injected intramuscularly, intraperitoneally, or intravenously.

Solid oral compositions (which are preferred as are liquid ones) may be prepared by conventional methods of blending, filling or tabletting. Repeated blending operations may be used to distribute the active agent throughout those compositions employing large quantities of fillers. Such operations are conventional in the art. The tablets may for example be prepared by wet or dry granulation and optionally coated according to the methods well known in normal pharmaceutical practice, in particular with an enteric coating.

The mentioned formulations will be prepared using standard methods such as those described or referred to in the Spanish and US Pharmacopeias and similar reference texts.

Medicaments according to the present invention may also be formulated into orally administrable compositions containing one or more physiologically compatible carriers or excipients, in solid or liquid form. These compositions may contain conventional ingredients such as binding agents, fillers, lubricants, and acceptable wetting agents. The compositions may take any convenient form, such as tablets, pellets, multiparticles, capsules, lozenges, caplets, aqueous or

oily solutions, suspensions, emulsions, gels, or dry powdered forms suitable for reconstitution with water or other suitable liquid medium before use, for immediate, controlled or retarded release.

The liquid oral forms for administration may also contain certain additives such as sweeteners, flavoring, preservatives, and emulsifying agents. Non-aqueous liquid compositions for oral administration may also be formulated, containing edible oils. Such liquid compositions may be conveniently encapsulated in e.g., gelatin capsules in a unit dosage amount.

The compositions of the present invention may also be administered topically or via a suppository.

The daily dosage for humans and animals may vary depending on factors that have their basis in the respective species or other factors, such as age, sex, weight or degree of illness and so forth. The daily dosage for humans may preferably be in the range from1 to 2000, preferably 1 to 1500, more preferably 1 to 1000 milligrams of active substance to be administered during one or several intakes per day.

Another preferred aspect of the invention is the use of at least one substituted pyrazoline compound of formula II or III according to the invention or at least one combination of compounds according to the invention (and optionally one or more pharmaceutically acceptable excipients), for the preparation of a medicament for the regulation of triglyceride levels in the blood plasma and for the prophylaxis and/or treatment of disorders of the central nervous system, especially stroke, of disorders of the cardiovascular system and of food intake disorders, especially bulimia, anorexia, cachexia, obesity, type II diabetus mellitus (non-insuline dependent diabetes mellitus), preferably obesity and diabetis.

Another preferred aspect of the invention is the use of at least one substituted pyrazoline compound of formaula II or III according to the invention (and optionally one or more pharmaceutically acceptable excipients,) for the preparation of a medicament for the prophylaxis and/or treatment of disorders of the central nervous system, disorders of the immune system, disorders of the cardiovascular system, disorders of the endocrinous system, disorders of the respiratory system, disorders of the gastrointestinal tract or reproductive disorders.

Another preferred aspect of the invention is the use of at least one combination of compounds according to the invention (and optionally one or more pharmaceutically acceptable excipients,) for the preparation of a medicament for the modulation of cannabinoid-receptors, preferably cannabinoid 1 ($CB_1$) receptors, for the prophylaxis and/or treatment of disorders of the central nervous system, disorders of the immune system, disorders of the cardiovascular system, disorders of the endocrinous system, disorders of the respiratory system, disorders of the gastrointestinal tract or reproductive disorders.

Another preferred aspect of the invention is the use of at least one substituted pyrazoline compound of formula II or III according to the invention or at least one combination of compounds according to the invention (and optionally one or more pharmaceutically acceptable excipients), for the preparation of a medicament for the prophylaxis and/or treatment of food intake disorders, preferably bulimia, anorexia, cachexia, obesity, type II diabetus mellitus (non-insuline dependent diabetes mellitus), preferably obesity.

Another preferred aspect of the invention is the use of at least one substituted pyrazoline compound of formula II or III according to the invention or at least one combination of compounds according to the invention (and optionally one or more pharmaceutically acceptable excipients), for the preparation of a medicament for the prophylaxis and/or treatment of psychosis.

Another preferred aspect of the invention is the use of at least one substituted pyrazoline compound of formula II or III according to the invention or at least one combination of compounds according to the invention (and optionally one or more pharmaceutically acceptable excipients), for the preparation of a medicament for the prophylaxis and/or treatment of alcohol abuse and/or addiction, nicotine abuse and/or addiction, medicament abuse and/or addiction and/or drug abuse and/or addiction, preferably drug abuse and/or addiction or nicotine abuse and/or addiction.

Another preferred aspect of the invention is the use of at least one substituted pyrazoline compound of formula II or III according to the invention or at least one combination of compounds according to the invention (and optionally one or more pharmaceutically acceptable excipients), for the preparation of a medicament for the prophylaxis and/or treatment of one or more disorders selected from the group consisting of schizophrenia, anxiety, depression, epilepsy, neurodegenerative disorders, cerebellar disorders, spinocerebellar disorders, cognitive disorders, cranial trauma, panic attacks, peripheric neuropathy, glaucoma, migraine, Morbus Parkinson, Morbus Huntington, Morbus Alzheimer, Raynaud's disease, tremblement disorders, compulsive disorders, senile dementia, thymic disorders, tardive dyskinesia, bipolar disorders; bone disorders including osteoporosis or Paget's disease of bone; cancer, preferably for the prophylaxis and/or treatment of one or more types of cancer selected from the group consisting of brain cancer, bone cancer, lip cancer, mouth cancer, esophageal cancer, stomach cancer, liver cancer, bladder cancer, pancreas cancer, ovary cancer, cervical cancer, lung cancer, breast cancer, skin camcer, colon cancer, bowl cancer and prostate cancer, more preferably for the prophylaxis and/or treatment of one or more types of cancer selected from the group consisting of colcon cancer, bowel cancer and prostate cancer; medicament-induced movement disorders, dystonia, endotoxemic shock, hemorragic shock, hypotension, insomnia, immunologic disorders, sclerotic plaques, vomiting, diarrhea, asthma, memory disorders, pruritus, pain, or for potentiation of the analgesic effect of narcotic and non-narcotic analgesics, or for influencing intestinal transit.

Medicaments according to the present invention may also be formulated into orally administrable compositions containing one or more physiologically compatible carriers or excipients, in solid or liquid form. These compositions may contain conventional ingredients such as binding agents, fillers, lubricants, and acceptable wetting agents. The compositions may take any convenient form, such as tablets, pellets, capsules, lozenges, aqueous or oily solutions, suspensions, emulsions, or dry powdered forms suitable for reconstitution with water or other suitable liquid medium before use, for immediate or retarded release.

The pharmaceutical excipients which can be used for the pharmaceutical composition according to the present invention include especially a diluent, a binder and a lubricant. A flowing agent, an antiadhesive and, optionally, a coloring agent and/or a flavoring agent can also be added.

A study of the effects of incorporating wetting agents showed that a low concentration of surfactant like a sodium alkylsulfate considerably increases the wettability. It was found that by incorporating a surfactant in the formulation, the formulation dissolves rapidly and completely and with a good reproducibility of the results. According to the present invention, sodium alkylsulfate is understood as meaning a sodium (C8-C12)alkylsulfate, for example sodium octylsulfate or, preferably, sodium laurylsulfate.

The diluent used in the composition of the present invention can be one or more compounds which are capable of densifying the active principle to give the desired mass. The preferred diluents are inorganic phosphates such as calcium phosphates; sugars such as hydrated or anhydrous lactose, or mannitol; and cellulose or cellulose derivatives such as, for example, microcrystalline cellulose, starch, corn starch or pregelatinized starch. Lactose monohydrate, mannitol, microcrystalline cellulose and corn starch, used by themselves or in a mixture, for example a mixture of lactose monohydrate and corn starch, are very particularly preferred.

The binder employed in the composition of the present invention can be one or more compounds which are capable of densifying a compound of formula (I) by converting it to larger and denser particles with better flow properties. The preferred binders are alginic acid or sodium alginate; cellulose and cellulose derivatives such as sodium carboxymethyl cellulose, ethyl cellulose, hydroxyethyl cellulose, hydroxypropyl cellulose, hydroxypropyl methyl cellulose or methyl cellulose; gelatin; acrylic acid polymers; and povidone, for example povidone K 30, which is a very particularly preferred binder. The binder is present in a proportion of 1% to 10% by weight in the pharmaceutical composition according to the invention.

The lubricant employed in the composition of the present invention can be one or more compounds which are capable of preventing the problems associated with the preparation of dry forms, such as the sticking and/or seizing problems which occur in the machines during compression or filling. The preferred lubricants are fatty acids or fatty acid derivatives such as calcium stearate, glyceryl monostearate, glyceryl palmitostearate, magnesium stearate, sodium laurylsulfate, sodium stearylfumarate, zinc stearate or stearic acid; hydrogenated vegetable oils, for example hydrogenated castor oil; polyalkylene glycols, especially polyethylene glycol; sodium benzoate; or talcum. Magnesium stearate is preferred according to the present invention. The lubricant is present in a proportion of 0.2% to 5% by weight in the pharmaceutical composition according to the invention.

The antiadhesive which may be employed in the composition of the present invention can be one or more compounds which are capable of reducing the sticky character of the formulation, for example of preventing adhesion to metal surfaces. The preferred antiadhesives are compounds containing silicon, for example silica or talcum. The antiadhesive can be present in a proportion of 0 to 5% by weight in the pharmaceutical composition according to the invention.

The flowing agent which may be employed in the composition of the present invention can be one or more compounds which are capable of facilitating the flow of the prepared formulation. The preferred flowing agents are compounds containing silicon, for example anhydrous colloidal silica or precipitated silica. The flowing agent can be present in a proportion of 0 to 15% by weight in the pharmaceutical composition according to the invention.

The disintegrating agent is understood as meaning cellulose or cellulose derivatives such as sodium carboxymethyl cellulose or crosslinked sodium carboxymethyl cellulose, crospovidone, pregelatinized starch or sodium carboxymethyl starch, crosslinked sodium carboxymethyl cellulose being a preferred disintegrating agent.

When not explicitely mentioned otherwise, the percentage in weight of excipients and additives is always related to the active principle.

According to the present invention, the pharmaceutical compositions may be prepared by a wet granulation or by a layering process.

Thus, for the wet granulation process, the internal phase, the active principle, the diluent, the binder, the disintegrating agent, the sodium alkylsulfate and, optionally, the coloring agent are mixed at room temperature and then wetted with the granulating liquid. The wet mass obtained is dried and then graded. The ingredient or ingredients of the external phase, namely the lubricant, possibly the antiadhesive, the flowing agent and, if appropriate, the coloring agent and/or the flavoring agent, are then added to the graded dry grains.

The layering process is characterized in that to an inert sugar/starch spherical core, a first layer is applied containing a mixture of the active ingredient, the diluent, the binder, the disintegrating agent, the sodium alkylsulfate

and, optionally, the coloring agent, optionally followed by a second isolation layer formed by water soluble polymers and compatible excipients. Finally, a layer consisting of an enteric coating is applied.

As a result of extensive studies on the sustained-release of a drug, the inventors of the present invention also discovered that the release of a drug in the colon, which is low in water content, can be achieved by providing a preparation adapted to absorb water into its core to undergo substantially complete gelation during its stay in the upper digestive tract such as stomach and small intestine, and then move in the form of the gel down to the lower digestive tract. The present invention was achieved based on the above finding.

Thus, another aspect of the present invention relates to a hydrogel-type sustained-release preparation comprising (1) at least one drug, (2) an additive providing for a penetration of water into the core of the preparation, and (3) a hydrogel-forming polymer, which preparation undergoes a substantially complete gelation during its stay in the upper digestive tract such as stomach and small intestine and is capable of releasing a drug in the colon.

The term "substantially complete gelation" of the preparation as used in this specification refers to the state in which not less than about 70%, preferably not less than about 80%, of the preparation is gelled.

Since even the colon can be utilized as a site of absorption, the sustained-release preparation of the present invention prolongs the absorption period of the drug to a remarkable extent and, hence, insures a steady blood level of the drug. Thus, the preparation of the present invention absorbs water during its stay in the upper digestive tract to undergo a substantially complete gelation and then moves down into the lower digestive tract with its surface being constantly eroded, and maintains drug release by further erosion in the lower digestive tract, with the result that a sustained and sufficient absorption of the drug is achieved even in the colon where little water is available.

The sustained-release preparation of the present invention is described in further detail hereinafter.

The drug or drugs which can be used in the preparation according to the present invention are not particularly limited in kind, provided that they are used for sustained-release system.

In order that these drugs may be readily absorbed in the colon which is low in water content, it is preferable to improve their solubilities in advance. Known techniques for improving the solubility of a drug which can be applied to hydrogel preparation can be employed. Among such techniques (solubilizing treatment) can be mentioned the method comprising adding a surfactant (e.g. polyoxyethylene-hydrogenated castor oils, polyoxy-ethylene-sorbitan higher fatty acid esters, polyoxyethylene polyoxypropylene glycols, sucrose fatty acid esters, etc.) and the method comprising preparing a solid dispersion of the drug and a solubilizer such as a polymer (e.g., a water-soluble polymer such as hydroxypropylmethylcellulose (HPMC), polyvinylpyrrolidone (PVP), polyethylene glycol (PEG), etc. or an enteric polymer such as carboxymethylethylcellulose (CMEC), hydroxypropylmethylcellulose phthalate (HPMCP), methyl methacrylate-methacrylic acid copolymer (Eudragit L and S; the trade name of Rhom & Haas Co.), etc.). When the drug is a basic substance, the method comprising adding an organic acid such as citric acid, tartaric acid or the like can be employed. If necessary, the method involving the formation of a soluble salt or the method comprising forming a clathrate using cyclodextrin or the like can also be employed. These procedures for solubilization can be modified as necessary according to the particular drug.

The additive for allowing water to penetrate into the core of the preparation according to the present invention (this additive for insuring a penetration of water into the preparation core will hereinafter be referred to as "hydrophilic base") is such that the amount of water required to dissolve 1 g of the hydrophilic base is not more than 5 ml and preferably not more than 4 ml at the temperature of 20 +/- 5 DEG C. The higher the solubility of the hydrophilic base in water, the more effective is the base in allowing water into the core of the preparation. The hydrophilic base includes, inter alia, highly hydrophilic polymers such as polyethylene glycol (PEG; e.g. PEG400, PEG1500, PEG4000, PEG6000 and PEG20000, produced by Nippon Oils and Fats Co.) and polyvinylpyrrolidone (PVP; e.g.PVP K30, the trade name of BASF), sugar alcohols such as D-sorbitol, xylitol, etc., sugars such as sucrose, anhydrous maltose, D-fructose, dextran (e.g. dextran 40), glucose, etc., surfactants such as polyoxyethylene-hydrogenated castor oil (HCO; e.g. Cremophor RH40 produced by BASF, HCO-40 and HCO-60 produced by Nikko Chemicals Co.), polyoxyethylene-polyoxypropylene glycol (e.g. Pluronic F68 produced by Asahi Denka Kogyo K.K.), polyoxyethylene-sorbitan high molecular fatty acid ester (Tween; e.g. Tween 80 produced by Kanto Kagaku K.K.), etc.; salts such as sodium chloride, magnesium chloride, etc., organic acids such as citric acid, tartaric acid, etc.; amino acids such as glycine, beta -alanine, lysine hydrochloride, etc.; and amino sugars such as meglumine.

Preferred ones are PEG6000, PVP, D-sorbitol, etc.

The proportion of such hydrophilic base depends on the characteristics of the drug (solubility, therapeutic efficacy, etc.) and content of the drug, solubility of the hydrophilic base itself, characteristics of the hydrogel-forming polymer used, the patient's condition at the time of administration and other factors. However, the proportion may preferably be a sufficient level to achieve a substantially complete gelation during the stay of the preparation in the upper digestive tract. The preparation stays in the upper digestive tract in a different period depending on the species and the individual but in about 2 hours after administration in the case of dogs and in about 4 to 5 hours after administration in the case of human (Br. J. clin. Pharmac, (1988) 26, 435-443).For administration to human, the proportion may preferably be a sufficient level to achieve a substantially complete gelation in about 4 to 5 hours after administration. The proportion

of the hydrophilic base is, therefore, generally about 5-80% by weight and preferably about 5-60% by weight based on the total weight of the preparation.

When the content of the hydrophilic base is too small, the necessary gelation into the core of the preparation does not proceed so that the release of the drug in the colon becomes insufficient. On the other hand, when the content of the hydrophilic base is excessive, the gelation proceeds in a shorter time but the resulting gel becomes so fragile that the release of the drug is too fast, thus failing to insure a sufficient sustained release. Moreover, because the amount of the base is large, the product becomes bulky.

The hydrogel-forming polymer mentioned above should have the physical characteristics, inclusive of viscosity in the gelled state, which permit the preparation of the present invention to retain its shape more or less during its travel down to the lower digestive tract, namely the colon, by withstanding the contractile forces of the digestive tract associated with the digestion of food.

The hydrogel-forming polymer which can be used in the preparation of the present invention is preferably a polymer showing a high viscosity on gelation. For example, a polymer showing a viscosity of not less than 1000 cps in 1% aqueous solution (at 25 DEG C) is particularly preferred.

The properties of the polymer depend on its molecular weight. The hydrogel-forming polymer which can be used in the present invention is preferably a substance of comparatively high molecular weight, viz. a polymer having an average molecular weight of not less than 2 x 10<6> and more preferably not less than 4 x 10<6>.

Among such polymers are polyethylene oxide (PEO) having a molecular weight of not less than 2 x 10<6> [e.g., Polyox WSR-303 (average mol. wt.: 7 x 10<6>; viscosity: 7500-10000 cps, 1% in H2O, 25 DEG C), Polyox WSR Coagulant (average mol. wt.: 5 x 10<6>; viscosity: 5500-7500 cps, under the same condition above), Polyox WSR-301 (average mol. wt.: 4 x 10<6>; viscosity: 1650-5500 cps, under the same condition above), Polyox WSR-N-60K (average mol. wt.: 2 x 10<6>; viscosity: 2000-4000 cps, 2% in H2O, 25 DEG C), all of which are trade names of Union Carbide Co.]; hydroxypropylmethylcellulose (HPMC) [e.g., Metolose 90SH100000 (viscosity: 4100-5600 cps., 1% in H2O, 20 DEG C), Metolose 90SH50000 (viscosity: 2900-3900 cps, under the same condition above), Metolose 90SH30000 (viscosity: 25000-35000 cps, 2% in H2O, 20 DEG C), all of which are trade names of Shin-Etsu Chemicals Co.]; sodium carboxymethylcellulose (CMC-Na) [e.g., Sanlose F-150MC (average mol. wt.: 2 x 10<5>; viscosity: 1200-1800 cps, 1% in H20, 25 DEG C), Sanlose F-1000MC (average mol. wt.: 42 x 10<4>; viscosity: 8000-12000 cps, under the same condition above), Sanlose F-300MC (average mol. wt.: 3 x 10<5>; viscosity: 2500-3000 cps, under the same condition above), all of which are trade names of Nippon Seishi Co., Ltd.]; hydroxyethylcellulose (HEC) [e.g., HEC Daicel SE850 (average mol. wt.: 148 x 10<4>; viscosity: 2400-3000 cps, 1% in H2O, 25 DEG C), HEC Daicel SE900 (average mol. wt.: 156 x 10<4>; viscosity: 4000-5000 cps, under the same condition above), all of which are trade names of Daicel Chemical Industries]; carboxyvinyl polymers [e.g., Carbopol 940 (average mol. wt.: ca. 25 x 10<5>; B.F.Goodrich Chemical Co.) and so on.

The preferred is a PEO having an average molecular weight of not less than 2 x 10<6>. Where a continuous release of the drug over a long time, for example more than 12 hours, is required, a polymer having a higher molecular weight, preferably an average molecular weight of not less than 4 x 10<6>, or a higher viscosity, preferably a viscosity of not less than 3000 cps at a concentration of 1% in water at 25 DEG C, is preferable.

The above hydrogel-forming polymer may be used singly, or two or more kind(s) of the above hydrogel-forming polymers in mixture may be used. Or, the mixture of two or more kinds of any polymers, which mixture has characteristics suitable for the present invention, may be suitably used for the present invention.

In order to insure a release of the drug in the human colon, it is necessary that a portion of the preparation having undergone gelation still remain in the colon even as late as at least 6-8 hours, preferably at least 12 hours, after administration.

In order to provide a hydrogel-type preparation having such properties, although it depends on the volume of the preparation, the kind of polymer and the properties and amount of the drug and of the additive for insuring a penetration of water into the preparation core, it is generally preferable that the preparation contains 10-95 weight % (preferably, 15-90 weight %) of the hydrogel-forming polymer based upon the preparation weighing less than 600 mg, and one preparation contains not less than 70 mg per preparation and preferably not less than 100 mg per preparation of the hydrogel-forming polymer. If the amount of this polymer is less than the above-mentioned level, the preparation will not tolerate erosion in the digestive tract for a sufficiently long time and a sufficient sustained release may not be achieved.

Regarding the types and proportions of the hydrophilic base and hydrogel-forming polymer (the latter is hereinafter referred to as hydrogel-forming base), their usefulness has been established by the following experiments.

The liquid oral forms for administration may also contain certain additives such as sweeteners, flavoring, preservatives, and emulsifying agents. Non-aqueous liquid compositions for oral administration may also be formulated, containing edible oils. Such liquid compositions may be conveniently encapsulated in e.g., gelatin capsules in a unit dosage amount.

The compositions of the present invention may also be administered topically or via a suppository.
The daily dosage for humans and animals may vary depending on factors that have their basis in the respective species

or other factors, such as age, sex, weight or degree of illness and so forth. The daily dosage for humans may preferably be in the range from1 to 2000, preferably 1 to 1500, more preferably 1 to 1000 milligrams of active substance to be administered during one or several intakes per day.

**Pharmacological Methods**

**I. In-vitro determination of affinity to CB1/CB2-Receptors**

The in-vitro determination of the affinity of the inventive substituted pyrazoline compounds to $CB_1$/$CB_2$-Rezeptors is carried out as described in the publication of Ruth A. Ross, Heather C. Brockie et al., "Agonist-inverse agonist characterization at $CB_1$ and $CB_2$ cannabinoid receptors of L-759633, L759656 and AM630", British Journal of Pharmacology, 126, 665-672, (1999), whereby the transfected human $CB_1$ and $CB_2$ receptors of Receptor Biology, Inc. are used. The radioligand used for both receptors is [$^3$H]-CP55940. The respective parts of the description is hereby incorporated by reference and forms part of the present disclosure.

**II. In-vivo bioassay system for determination of cannabinoid activity**

Mouse tetrad model

Substances with affinity for cannabinoid receptors are known to produce a wide range of pharmacological effects. It is also known that intravenous administration of a substance with affinity for cannabinoid receptors in mice produces analgesia , hypothermia, sedation and catalepsy. Individually, none of these effects can be considered as proof that a tested substance has affinity for cannabinoid-receptors, since all of these effects are common for various classes of centrally active agents. However, substances, which show all of these effects, i.e. substances that are active in this so-called tetrad model are considered to have affinity for the cannabinoid receptors. It has further been shown that cannabinoid receptor antagonists are higly effective in blocking the effects of a cannabinoid agonist in the mouse tetrad model.

The tetrad model is described, for example, in the publication of A. C. Howlett et al, International Union of Pharmacology XXVII. Classification of Cannabinoid Receptors, Pharmacol Rev 54, 161-202 , 2002 and David R. Compton et al., "In-vivo Characterization of a Specific Cannabinoid Receptor Antagonist (SR141716A) :Inhibition of Tetrahydrocannbinol- induced Responses and Apparent Agonist Activity", J. Pharmacol. Exp. Ther. 277 , 2, 586-594, 1996. The corresponding parts of the description are hereby incorporated by reference.

**Material and Methods**

Male NMRI mice with a weight of 20-30 g (Harlan, Barcelona, Spain) are used in all of the following experiments.

Before testing in the behavioral procedures given below, mice are acclimatized to the experimental setting. Pre-Treatment control values are determined for analgesia hot plate latency (in seconds), rectal temperature, sedation and catalepsy.

In order to determine the agonistic activty of the substance to be tested, the mice are injected intravenously with the substance to be tested or the vehicle alone. 15 minutes after injection, latency in hot plate analgesia is measured. Rectal temperature, sedation and catalepsy are measured 20 minutes after injection.

In order to determine the antagonistic activity the identical procedure is used as for the determination of the agonistic effects, but with the difference that the substance to be evaluated for its antagonistic activity is injectected 5 minutes before the intravenous injection of 1.25 mg/kg Win-55,212 a known cannabinoid-receptor agonist.

**Hot plate analgesia**

The hot plate analgesia is determined according to the method described in Woolfe D. et al. "The evaluation of analgesic action of pethidine hydrochloride (Demerol)", J. Pharmacol. Exp. Ther. 80, 300-307,1944. The respective description is hereby incorporated by reference and forms part of the present disclosure.

The mice are placed on a hot plate (Harvard Analgesimeter) at 55 $\pm$ 0.5 °C until they show a painful sensation by licking their paws or jumping and the time for these sensations to occur is recorded. This reading is considered the basal value (B). The maximum time limit the mice are allowed to remain on the hot plate in absence of any painful response is 40 seconds in order to prevent skin damage. This period is called the cut-off time (PC).

Fifteen minuts after the administration of the substance to be tested, the mice are again placed on the hot plate and the afore described procedure is repeated. This period is called the post-treatment reading (PT).

The degree of analgesia is calculated from the formula :

$$\% \text{ MPE of Analgesia} = (\text{PT- B}) / (\text{PC-B}) \times 100$$

MPE = Maximum possible effect.

**Determination of sedation and ataxia**

Sedation and ataxia is determined according to the method described in Desmet L. K. C. et al. "Anticonvulsive properties of Cinarizine and Flunarizine in Rats and Mice", Arzneim. -Forsch. (Frug Res) 25, 9, 1975. The respective description is hereby incorporated by reference and forms part of the present disclosure.

The chosen scoring system is

**0:** no ataxia;
**1:** doubful;
**2:** obvious calmness and quiet;
**3** pronounced ataxia;

prior to as well as after treatment.

The percentage of sedation is determined according to the formula:

$$\% \text{ of sedation} = \text{arithmetic mean} / 3 \text{ X } 100$$

**Hypothermia:**

Hypothermia is determined according to the method described in David R. Compton et al. "In-vivo Characterization of a Specific Cannabinoid Receptor Antagonist (SR141716A) Inhibition of Tetrahydrocannbinol- induced Responses and Apparent Agonist Activity", J. Pharmacol Exp Ther. 277 , 2, 586-594, 1996. The respective description is hereby incorporated by reference and forms part of the present disclosure.

The base-line rectal temperatures are determined with a thermometer (Yello Springs Instruments Co., Panlabs) and a thermistor probe inserted to 25mm before the administration of the substance to be tested. Rectal temperature is again measured 20 minutes after the administration of the substances to be tested. The temperature difference is calculated for each animal, whereby differences of ≥ -2 ºC are considered to represent activity.

**Catalepsy:**

Catalepsy is determined according to the method described in Alpermann H. G. et al. "Pharmacological effets of Hoe 249: A new potential antidepressant", Drugs Dev. Res. 25, 267-282. 1992. The respective description is hereby incorporated by reference and forms part of the present disclosure.

The cataleptic effect of the substance to be tested is evaluated according to the duration of catalepsy, whereby the animals are placed head downwards with their kinlegs upon the top of the wooden block.

The chosen scoring system is:

Catalepsy for:
more than 60 seconds = 6; 50 -60 seconds = 5, 40-50 seconds = 4, 30-40 seconds = 3, 20-30 seconds = 2, 5-10 seconds = 1, and less than 5 seconds =0.

The percentage of catalepsy is determined according ot the following formula:

$$\% \text{ Catalepsy} = \text{arithmetic mean} / 6 \text{ X } 100$$

**III. In vivo testing for antiobesic activity**

The in-vivo testing for antiobesic activity of the inventive pyrazoline compounds is carried out as described in the publication of G. Colombo et al., "Appetite Suppression and Weight Loss after the Cannabinoid Antagonist SR 141716"; Life Sciences, 63 (8), 113-117, (1998). The respective part of the description is hereby incorporated by reference and forms part of the present disclosure.

**IV. In vivo testing for antidepressant activity**

The in-vivo testing for antidepressant activity of the inventive pyrazoline compounds in the water despair test is carried out as described in the publication of E.T. Tzavara et al., "The CB1 receptor antagonist SR141716A selectively increases monoaminergic neurotransmission in the medial prefrontal cortex: implications for therapeutic actions"; Br. J. Pharmacol. 2003, 138(4):544:53. The respective part of the description is hereby incorporated by reference and forms part of the present disclosure.

The present invention is illustrated below with the aid of examples. These illustrations are given solely by way of example and do not limit the general spirit of the present invention.

**Examples:**

**Example 1:**

**1 mg Gelatin Capsule**

A gelatin capsule prepared by wet granulation and having the following composition:

*Internal phase*

N-piperidinyl-5-(4-chloro-phenyl)-1-(2,4-dichlorophenyl)-4,5-dihydro-1H-pyrazole-3-carboxamide 1 mg
corn starch 51 mg
200 mesh lactose monohydrate 103.33mg
povidone K 30 4.3mg
crosslinked sodium carboxymethyl cellulose 8.5mg

*Granulation*

sodium laurylsulfate 0.17mg
purified water QS
***External phase***
magnesium stearate 1.7mg
For a size 3 white-opaque gelatin capsule completed up to 170mg

**Example 2:**

**10 mg Gelatin Capsule**

A gelatin capsule prepared by wet granulation and having the following composition: compound N-[5-(4-Chloro-phenyl)-1-(2,4-dichloro-phenyl)-4,5-dihydro-1H-pyrazole-3-carbonyl]-4-methyl-phenylsulfonamide 10 mg
corn starch 51 mg
200 mesh lactose monohydrate 94.33 mg
povidone K 30 4.3 mg
crosslinked sodium carboxymethyl cellulose 8.5 mg

*Granulation*

sodium laurylsulfate 0.17 mg
purified water QS

***External phase***

magnesium stearate 1.7 mg
For a size 3 white-opaque gelatin capsule completed up to 170 mg

**Example 3:**

**30 mg Gelatin Capsule**

A gelatin capsule prepared by wet granulation and having the following composition: compound 5-(4-Chloro-phenyl)-1-(2,4-dichloro-phenyl)-4,5-dihydro-1H-pyrazole-3-carboxylic acid diethylamide 30 mg
corn starch 51 mg
200 mesh lactose monohydrate 74.33 mg
povidone K 30 4.3 mg
crosslinked sodium carboxymethyl cellulose 8.5 mg

***Granulation***

sodium laurylsulfate 0.17 mg
purified water QS

***External phase***

magnesium stearate 1.7 mg
For a size 3 white-opaque gelatin capsule completed up to 170 mg

**Example 4:**

**30 mg Gelatin Capsule**

A gelatin capsule prepared by wet granulation and having the following composition: compound [5-(4-Chloro-phenyl)-1-(2,4-dichloro-phenyl)-4,5-dihydro-1H-pyrazol-3-yl]-piperidin-1-yl-methanone 30 mg
corn starch 51 mg
200 mesh lactose monohydrate 73.65 mg
povidone K 30 4.3 mg
crosslinked sodium carboxymethyl cellulose 8.5 mg

***Granulation***

sodium laurylsulfate 0.85 mg
purified water QS

***External phase***

magnesium stearate 1.7 mg
For a size 3 white-opaque gelatin capsule completed up to 170 mg

**Example 5:**

**1 mg tablet**

compound 5-(4-Chloro-phenyl)-1-(2,4-dichioro-phenyl)-4,5-dihydro-1H-pyrazole-3-carboxylic acid diethylamide 1 mg
corn starch 50 mg
200 mesh lactose monohydrate 130 mg
6 cP hydroxypropyl methyl cellulose 6 mg
crosslinked sodium carboxymethyl cellulose 10 mg

***Granulation***

sodium laurylsulfate 1 mg
purified water QS

*External phase*

magnesium stearate 2 mg
For a tablet completed up to 200 mg

**Example 6:**

**10 mg tablet**

***Internal phase***

compound 5-(4-Chloro-phenyl)-1-(2,4-dichloro-phenyl)-4,5-dihydro-1H-pyrazole-3-carboxylic acid diethylamide 10 mg
corn starch 50mg
200 mesh lactose monohydrate 211.5 mg
6 cP hydroxypropyl methyl cellulose 9 mg
sodium carboxymethyl starch 15 mg
sodium laurylsulfate 1.5 mg
***Granulation***
purified waterQS

***External phase***

magnesium stearate 3 mg
For a tablet completed up to 300 mg

**Example 7:**

**30 mg tablet**

***Internal phase***

compound N-oxide of N-piperidinyl-5-(4-chlorophenyl)-1-(2,4-dichlorophenyl)-4,5-dihydropyrazole-3-carboxamide 30 mg
corn starch 80 mg
200 mesh lactose monohydrate 252 mg
povidone K 30 12 mg
crosslinked sodium carboxymethyl cellulose 20 mg
sodium laurylsulfate 2 mg

***Granulation***

purified water QS
***External phase***
magnesium stearate 4 mg
For a tablet completed up to 400 mg

**Example 8**

**Pellets**

In 580 g of deionized water, 75 g of 5-(4-Chloro-phenyl)-1-(2,4-dichloro-phenyl)-4,5-dihydro-1H-pyrazole-3-carboxylic acid-[1,2,4]triazol-4-yl amide (10.6%) and 70 g of hydroxypropylmethylcellulose, 20 mg crosslinked sodium

carboxymethyl cellulose (2.8%) and 1 mg sodium laurylsulfate (0.14%) are dispersed. 490 g of inert uniform sugar/starch spheres (composition according to US pharmacopoeia) are introduced into a fluidized bed apparatus and the previous obtained dispersion is sprayed on the spheres. After spraying, the spheres are dried before applying the second layer. In 350 g of deionized water, 52 g of hydroxypropylmethylcellulose and 7 g of titanium dioxide are dispersed and the resulting aqueous dispersion is sprayed on the spheres obtained in the previous step. After spraying, the spheres are dried before applying the third enteric coating layer.

In 280 g of deionized water, 290 g of a USP methacrylic acid copolymer (type C aqueous suspension), 13 g of triethylcitrate and 38,5 g of talc are dispersed, and the resulting aqueous dispersion is sprayed on the spheres obtained in the previous step. After applying this final enteric coating layer the spheres (pellets) are dried.

**Example 9**

In a solvent mixture (dichloromethane-methanol) were dissolved 5-(4-Chloro-phenyl)-1-(2,4-dichloro-phenyl)-4,5-dihydro-1H-pyrazole-3-carboxylic acid-[1,2,4]triazol-4-yl amide (CPTA), Tween 80 and CMEC and the solution was spray-dried using a spray dryer. The dried mixture was blended with POLYOX303 and the resulting composition was compression-molded using an oil press at a compression pressure of 0.8 ton/punch to provide tablets (SR) each measuring 8.0 mm in diameter and weighing 171.6 mg (CPTA content: 65 mg). Separately, CPTA and TC-5E were dissolved in a solvent mixture (dichloromethane-methanol) and using a Hi-Coater, this immediate-release component (QR; CPTA: 15 mg) was coated on the SR (CPTA: 65 mg) component to provide tablets each weighing 194.1 mg (CPTA: 80 mg).

**Example 10**

30 mg tablet

***Internal phase***

5-(4-chloro-phenyl)-1-(2,4-dichlorophenyl)-4,5-dihydro-1H-pyrazol-3-carboxylic acid 30 mg
corn starch 80 mg
200 mesh lactose monohydrate 252 mg
povidone K 30 12 mg
crosslinked sodium carboxymethyl cellulose 20 mg
sodium laurylsulfate 2 mg

*Granulation*

purified water QS

*External phase*

magnesium stearate 4 mg
For a tablet completed up to 400 mg

Example 11: N-piperidinyl-5-(4-chlorophenyl)-1-(2,4-dichlorophenyl)-4,5-dihydro-1H-pyrazole-3-carboxamide

a) 4-(4-chlorophenyl)-2-oxo-3-butenoic acid

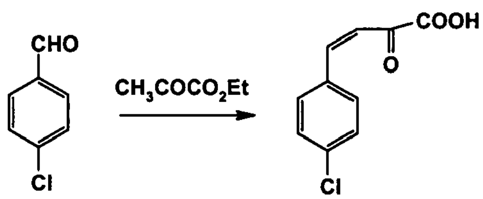


In a three neck flask p-chlorobenzaldehyde (13,3 g, 95 mmoles) and ethyl pyruvate (10 g, 86 mmoles) were dissolved in 150 ml of absolute ethanol.The solution was ice-cooled to 0°C and an aqueous solution of NaOH (3.8 g in 45 mL

water) was added dropwise keeping the temperature below or equal to 10°C, whereby a yellow-orange colored precipitate was formed. The reaction mixture was stirred for 1 hour at 0°C and an additional 1.5 hours at room temperature (approximately 25 °C). Afterwards the reaction mixture was cooled down to approximately 5°C and the insoluble sodium salt of 4-(4-chlorophenyl)-2-oxo-3-butenoic acid was isolated by filtration. The filtrate was left in the refrigerator overnight, whereby more precipitate is formed, which was filtered off, combined with the first fraction of the salt and washed with diethyl ether. The sodium salt of 4-(4-chlorophenyl)-2-oxo-3-butenoic acid was then treated with a solution of 2N HCl, stirred for some minutes and solid 4-(4-chlorophenyl)-2-oxo-3-butenoic acid was separated via filtration and dried to give 12.7 g of the desired product (70% of theoretical yield).
IR (KBr, $cm^{-1}$) : 3500-2500, 1719,3, 1686,5, 1603,4, 1587,8, 1081,9.
$^1H$ NMR($CDCl_3$, □) : 7,4 (d, J=8,4Hz, 2H), 7,5 (d, J=16,1Hz, 1H), 7,6 (d, J=8,4Hz, 2H), 8,1 (d, J=16,1 Hz, 1 H).

b) 5-(4-chlorophenyl)-1-(2,4-dichlorophenyl)-4,5-dihydro-pyrazole-3-carboxylic acid

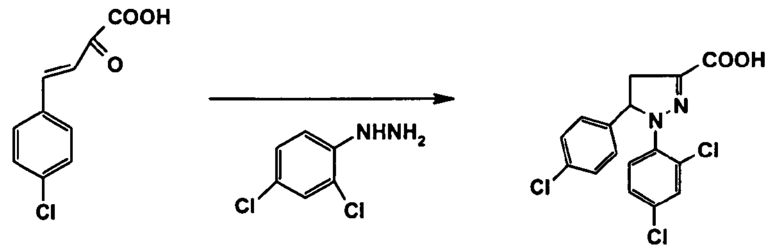

4-(4-chlorophenyl)-2-oxo-3-butenoic acid obtained according to step a) (12.6 g, 60 mmoles), 2,4-dichlorophenylhydrazine hydrochloride (12.8 g, 60 mmoles) and glacial acetic acid (200 mL) were mixed under a nitrogen atmosphere and heated to reflux for 4 hours, cooled down to room temperature (approximately 25 °C) and given into ice-water, whereby a sticky mass was obtained, which was extracted with methylene chloride. The combined methylene chloride fractions were washed with water, dried with sodium sulfate, filtered and evaporated to dryness to give a pale yellow solid (12.7 g, 57% of theoretical yield).
IR (KBr, $cm^{-1}$) : 3200-2200, 1668,4, 1458, 1251,4, 1104,8.
$^1H$ NMR ($CDCl_3$, □) : 3,3 (dd, 1 H), 3,7 (dd, 1 H), 5,9 (dd, 1 H), 7,09-7,25 (m, 7H).

(c) 5-(4-chlorophenyl)-1-(2,4-dichtorophenyl)-4,5-dihydro-pyrazole-3-carboxylic acid chloride

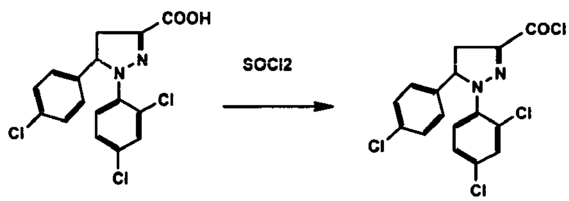


Under nitrogen atmosphere 5-(4-chlorophenyl)-1-(2,4-dichlorophenyl)-4,5-dihydro-pyrazole-3-carboxylic acid (2.5 g, 6.8 mmols) obtained according to step (b) was dissolved in 4 mL of in thionyl chloride and heated to reflux for 2.5 hours. The excess thionyl chloride is removed from the reaction mixture under reduced pressure and the resulting crude residue (2.6 g) is used without any further purification.
IR (KBr, $cm^{-1}$) : 1732,3, 1700, 1533,3, 1478,1, 1212,9, 826,6.

d) N-piperidinyl-5-(4-chlorophenyl)-1-(2,4-dichlorophenyl)-4,5-dihydropyrazole-3-carboxamide [this compound may also be referred to as 5-(4-Chloro-phenyl)-1-(2,4-dichloro-phenyl)-4,5-dihydro-1H-pyrazole-3-carboxylic acid piperidin-1-ylamide or as 1-(2,4-dichlorophenyl)-5-(4-chlorophenyl)-4,5-dihydro-N-(piperidin-1-yl)-1H-pyrazole-3-carboxamide]

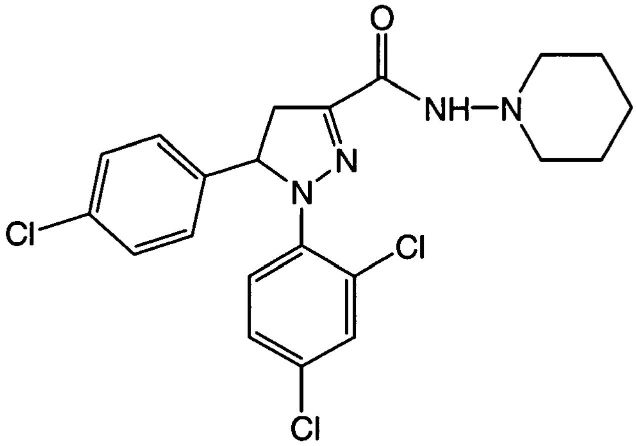

Under nitrogen atmosphere N-aminopiperidine (0.6 mL, 5.6 mmoles) and triethylamine (4 mL) were dissolved in methylene chloride (25 mL). The resulting mixture was ice-cooled down to 0°C and a solution of 5-(4-chlorophenyl)-1-(2,4-dichlorophenyl)-4,5-dihydro-pyrazole-3-carboxylic acid chloride obtained in step (c) in methylene chloride (15 mL) was added dropwise. The resulting reaction mixture was stirred at room temperature (approximately 25 °C) overnight. Afterwards the reaction mixture was washed with water, followed by a saturated aqueous solution of sodium bicarbonate, then again with water, dried over sodium sulfate, filtered and evaporated to dryness in a rotavapor. The resulting crude solid was crystallized from ethanol. The crystallized solid was removed via filtration and the mother liquors were concentrated to yield a second fraction of crystallized product. The two fractions were combined to give a total amount of 1.7 g (57% of theoretical yield) of N-piperidinyl-5-(4-chlorophenyl)-1-(2,4-dichlorophenyl)-4,5-dihydropyrazole-3-carboxamide having a melting point of 183-186°C.
IR (KBr, $cm^{-1}$) : 3222,9, 2934,9, 1647,4, 1474,7, 1268,3, 815,6.
$^1$H NMR ($CDCl_3$, □) : 1,4 (m, 2H), 1,7 (m, 4H), 2,8 (m, 4H), 3,3 (dd, J=6,1 y 18,3Hz, 1 H), 3,7 (dd, J=12,5 and 18,3 Hz, 1 H), 5,7 (dd, J=6,1 and 12,5 Hz, 1 H), 7,0-7,2 (m, 6H), 7,4 (s, 1 H).
The compounds according to the following examples 2-6 have been prepared analogously to the process described in Example 1.

**Example 12:** 5-(4-Chloro-phenyl)-1-(2,4-dichloro-phenyl)-4,5-dihydro-1H-pyrazole-3-carboxylic acid-[1,2,4]triazol-4-yl amide

Melting point: 134-138 ºC.
IR (KBr, $cm^{-1}$): 3448, 1686, 1477, 1243, 1091, 821.
$^1$H NMR($CDCl_3$, □): 3,1 (dd, J=6,2 and 17,9Hz, 1H), 3,7 (dd, J=12,3 and 17,9Hz, 1 H), 5,9 (dd, J=6,2 and 12,3 Hz, 1 H), 7,2-7,5 (m, 7H), 8,7 (s, 2H), 12,0 (bs, 1 H).

**Example 13:** 5-(4-Chloro-phenyl)-1-(2,4-dichloro-phenyl)-4,5-dihydro-1H-pyrazole-3-carboxylic acid-(4-methyl-piperazin-1-yl)-amide hydrochloride

Melting point: 150-155°C.
IR (KBr, $cm^{-1}$) : 3433, 1685, 1477, 1296, 1246, 1088, 1014, 825.
$^1$H NMR ($CDCl_3$, □): 2,7 (d, J=4,2Hz, 3H), 3,0-3,4 (m, 9H), 3,6 (dd, J=11,9 and 17,9 Hz, 1 H), 5,8 (dd, J=5,5 and 11,9 Hz, 1 H), 7,1 (d, J=8,4Hz, 2H), 7,25 (2d, J= 8,4 and 8,7 Hz, 3H), 7,4 (d, J=2,2Hz, 1 H), 7,5 (d, J=8,7Hz, 1 H), 9,8 (s, 1 H), 11,2 (bs).

**Example 14:** 5-(4-Chloro-phenyl)-1-(2,4-dichloro-phenyl)-4,5-dihydro-1H-pyrazole-3-carboxylic acid diethylamide

This compound was obtained in form of an oil.
IR (film, $cm^{-1}$) : 2974, 1621, 1471, 1274, 1092, 820.
$^1$H NMR ($CDCl_3$, □): 1,2 (m, 6H), 3,3-3,9 (m, 6H), 5,6 (dd, J=5,8 and 11,7 Hz, 1 H), 7-7,25 (m, 7H).

**Example 15:** [5-(4-Chloro-phenyl)-1-(2,4-dichloro-phenyl)-4,5-dihydro-1H-pyrazol-3-yl]-piperidin-1-yl-methanone

Melting point: 105-110ºC.
IR (KBr, $cm^{-1}$) : 2934, 1622, 1470, 1446, 1266, 1010, 817.
$^1H$ NMR ($CDCl_3$, □): 1,7 (m, 6H), 3,4 (dd, J=5,7 and 17,9Hz, 1H), 3,7 (m, 3H), 3,9 (m, 2H), 5,6 (dd, J=6,1 y 11,9 Hz, 1 H), 7-7,25 (m, 7H).

**Example 16:** N-[5-(4-Chloro-phenyl)-1-(2,4-dichloro-phenyl)-4,5-dihydro-1H-pyrazole-3-carbonyl]-4-methyl-phenylsulfonamide

This compound was obtained in form of an amorph solid.
IR (KBr, $cm^{-1}$) : 1697, 1481, 1436, 1340, 1169, 1074, 853.
$^1H$ NMR ($CDCl_3$, □): 2,4 (s, 3H), 3,2 (dd, J=6,6 and 18,3Hz, 1H), 3,6 (dd, J=12,8 and 18,3Hz, 1 H), 5,8 (dd, J=6,6 and 12,8Hz, 1 H), 7 (d, J=8,2Hz, 2H), 7,2 (s, 1 H), 7,3-7,4 (m, 6H), 8 (d, J=8,1 Hz, 2H), 9 (s, 1H).

**Example 17:** N-oxide of N-piperidinyl-5-(4-chlorophenyl)-1-(2,4-dichlorophenyl)-4,5-dihydropyrazole-3-carboxamide

Under nitrogen gas as an inert atmosphere N-piperidinyl-5-(4-chlorophenyl)-1-(2,4-dichlorophenyl)-4,5-dihydropyrazole-3-carboxamide (0,15 g, 332 mmoles) was dissolved in 7 ml of dichloromethane. The resulting solution was ice-cooled to 0 °C and m-chloroperbenzoic acid (0,204 g, 0,83 mmoles) added in several portions. After stirring for 15 minutes a control via thin layer chromatography showed that no starting material was remaining. A saturated solution of sodium bicarbonate was then slowly added, the organic phase separated, washed with water, dried over sodium sulfate and filtered. The filtered solution was evaporated to dryness and the crude product was purified via column chromatography yielding 78 mg (50 % of theoretical yield) of the N-oxide of N-piperidinyl-5-(4-chlorophenyl)-1-(2,4-dichlorophenyl)-4,5-dihydropyrazole-3-carboxamide in form of a white solid having a melting point of 115-120°C.
IR (KBr, $cm^{-1}$): 3202, 1678, 1654, 1474, 1309, 1107.
$^1H$-NMR ($CDCl_3$, □): 1.6 (m, 2H), 1.8-2.0 (m, 4H), 2.55 (m, 2H), 3.3 (dd, J = 6.3 Hz and 18.2 Hz, 1 H), 3.7 (m, 3H), 5.8 (dd, J = 6.3 Hz and 12.5 Hz, 1 H), 7.0-7.3 (m, 7H), 8.5 (s, 1 H.)

**Pharmacological Data:**

**I. In-vitro determination of affinity to $CB_1$/$CB_2$-Receptors**

The affinity of the inventive substituted pyrazoline compounds to $CB_1$/$CB_2$ receptors was determined as described above. Some of the values obtained are given in the following table I:

**Table I:**

| Compound according to Example | $CB_1$-Receptor Radiologand:[$^3H$]-CP55940 | | $CB_2$-Receptor Radiologand:[$^3H$]-CP55940 | |
|---|---|---|---|---|
| | % Inhibition $10^{-6}$ M | $K_i$(nM) | % Inhibition $10^{-6}$ M | $K_i$(nM) |
| 1 | 93 % | < 25 | 33 % | > 1000 |
| 5 | 79 % | 111 | 54 % | ≈ 1000 |

As can be seen from the values given in table 1 the inventive pyrazoline compounds are particularly suitable for regulating the $CB_1$-Receptor.

**II. In-vivo bioassay system for determination of cannabinoid activity**

The determinination of cannabinoid activity in-vivo was determined as described above. Some of the values obtained are given in the following table II:

Table II:

| Compound according to example: | dosis administered: 5 mg/kg i.v. Agonistic effect | | | | dosis administered 5 mg/kg i.v. prior to Win 55212-2 in a dose of 1,25mg/kg i.v. Antagonistic Effect | | | |
|---|---|---|---|---|---|---|---|---|
| | A | B | C. | D | A | B. | C | D |
| 1 | 0 | 0 | 0 | 0 | 74 | 100 | 100 | 100 |
| 5 | 0 | 50 | 0 | 0 | 50 | 40 | 20 | 20 |
| i.v. intravenous<br>A: Hot-Plate test<br>B: Hypothermia<br>C: Catalepsy<br>D: Sedation | | | | | | | | |

As can be seen from the values given in table II the inventive pyrazoline compounds show an antagonistic effect.

**III. In-vivo testing for antiobesic activity**

The in-vivo testing for antiobesic activity was carried out as described above, whereby four different groups of 10 rats each were treated as follows:

**Group I:**

Group was treated with vehicle, namely arabic gum (5 wt.-%) in water.

**Group II:**

The second group of rats was treated with the inventive compound N-piperidinyl-5-(4-chlorophenyl)-1-(2,4-dichlorophenyl)-4,5-dihydropyrazole-3-carboxamide according to Example 1. Said compound was administered intraperitoneally to the rats over a period of 14 days in a daily dosis of (10 mg/kg body weight).

**Group III:**

The third group of rats was treated with Amphetamine, an active ingredient known to reduce appetite. Said compound was administered intraperitoneally to the rats over a period of 14 days in a daily dosis of (5 mg/kg body weight).
As can be seen from Figure 1 the body weight is lowered due to the administration of the inventive compound according to example 11 and this effect is also observed after the treatment is ended.
**Figure 2** shows the reduction of food intake due to the administration of the inventive compound according to example 11.

**IV. In vivo testing for antidepressant activity**

The in-vivo testing for antidepressant activity of the inventive pyrazoline compounds in the water despair test was carried out as described above. In particular, the compound according to example 1 displayed positive effects with respect to immobility time and struggling time.

## Claims

**1.** A pharmaceutical composition for the oral administration comprising at least one compound of formula (I) :

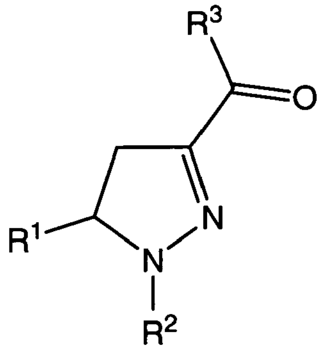

(I)

wherein,
$R^1$ represents a phenyl ring, which is mono-substituted with a halogen atom, preferably a chlorine atom, in its 4-position,
$R^2$ represents a phenyl ring, which is di-substituted with two halogen atoms, preferably chlorine atoms, in its 2- and 4-position,
$R^3$ represents a pyrrolidinyl group, a piperidinyl group, a piperazinyl group, a homo-piperazinyl group, a morpholinyl group, or an —$NR^4R^5$-moiety,
$R^4$ represents a hydrogen atom or a linear or branched $C_{1-6}$-alkyl group,
$R^5$ represents a linear or branched $C_{1-6}$ alkyl group, a pyrrolidinyl group, a piperidinyl group, a piperazinyl group, a homo-piperazinyl group, a morpholinyl group, a triazolyl group, whereby each of the heterocyclic rings may be substituted with one or more, identical or different, $C_{1-6}$-alkyl groups, or an -$SO_2$-$R^6$-moiety, and
$R^6$ represents a phenyl group, which is optionally substituted with one or more $C_{1-6}$ alkyl groups, which may be identical or different, optionally in form of one of the stereoisomers, preferably enantiomers or diastereomers, a racemate or in form of a mixture of at least two of the stereoisomers, preferably enantiomers and/or diastereomers, in any mixing ratio, or a corresponding N-oxide thereof, or a corresponding salt thereof, or a corresponding solvate thereof, and 0.05% to 0.5% by weight of the active principle of at least one surfactant and optionally one or more additional pharmaceutical excipients.

**2.** A pharmaceutical composition for the oral administration comprising at least one compound of formula (II),

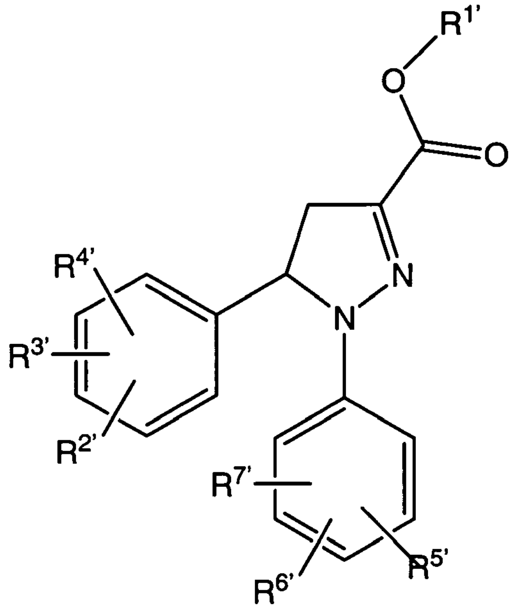

(II)

wherein

$R^{1'}$ represents hydrogen or a linear or branched $C_{1-4}$-alkyl group,

$R^{2'}$, $R^{3'}$ and $R^{4'}$ independently of each other represent hydrogen, a linear or branched $C_{1-6}$-alkyl group, a linear or branched $C_{1-6}$-alkoxy group, a halogen atom, $CH_2F$, $CHF_2$, $CF_3$, CN, OH, $NO_2$, -(C=O)-$R^{8'}$, SH, $SR^{8'}$, $SOR^{8'}$, $SO_2R^{8'}$, $NH_2$, $NHR^{8'}$, $NR^{8'}R^{9'}$, -(C=O)-$NH_2$, -(C=O)-$NHR^{8'}$ or -(C=O)-$NR^{8'}R^{9'}$ whereby $R^{8'}$ and $R^{9'}$ for each substituent independently represent linear or branched $C_{1-6}$ alkyl, $R^{5'}$ and $R^{6'}$ independently of each other represent a linear or branched $C_{1-6}$-alkyl group, a linear or branched $C_{1-6}$-alkoxy group, a halogen atom, $CH_2F$, $CHF_2$, $CF_3$, CN, OH, $NO_2$, -(C=O)-$R^{10'}$, SH, $SR^{10'}$, $SOR^{10'}$, $NH_2$, $NHR^{10'}$, $NR^{10'}R^{11'}$, - (C=O)-$NH_2$, -(C=O)-$NHR^{10'}$ and -(C=O)-$NR^{10'}R^{11'}$, whereby $R^{10'}$ and optionally $R^{11'}$ for each substituent independently represent linear or branched $C_{1-6}$ alkyl;

$R^{7'}$ represents hydrogen, a linear or branched $C_{1-6}$-alkyl group, a linear or branched $C_{1-6}$-alkoxy group, a halogen atom, $CH_2F$, $CHF_2$, $CF_3$, CN, OH, $NO_2$, - (C=O)-$R^{10'}$, SH, $SR^{10'}$, $SOR^{10'}$, $NH_2$, $NHR^{10'}$, $NR^{10'}R^{11'}$, -(C=O)-$NH_2$, -(C=O)-$NHR^{10'}$ and -(C=O)-$NR^{10'}R^{11'}$, whereby $R^{10'}$ and optionally $R^{11'}$ for each substituent independently represent linear or branched $C_{1-6}$ alkyl;

with the proviso that

if $R^{1'}$ and $R^{7'}$ are H and $R^{5'}$ and $R^{6'}$ both represent Cl in the 3- and 4-position of the phenyl ring neither of $R^{2'}$, $R^{3'}$ and $R^{4'}$ may represent F in the 4-position of the phenyl ring if the other two of $R^{2'}$, $R^{3'}$ and $R^{4'}$ both represent H;

optionally in form of one of the stereoisomers, preferably enantiomers or diastereomers, a racemate or in form of a mixture of at least two of the stereoisomers, preferably enantiomers and/or diastereomers, in any mixing ratio, or a corresponding N-oxide thereof, or a corresponding salt thereof, or a corresponding solvate thereof, and 0.05% to 0.5% by weight of the active principle of a surfactant and optionally one or more additional pharmaceutical excipients.

**3.** A pharmaceutical composition for the oral administration comprising at least one compound of formula (III)

III

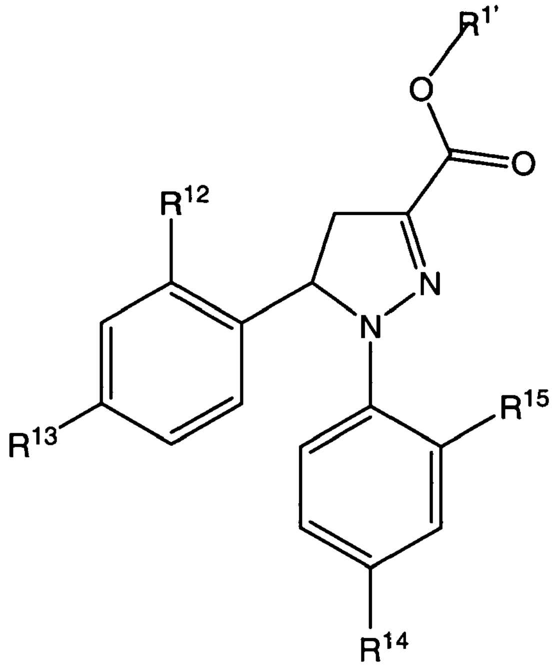

II

wherein
$R^{1'}$ represents hydrogen or a linear or branched $C_{1-4}$-alkyl group,
$R^{12}$, $R^{13}$, $R^{14}$ or $R^{15}$ independently of each other represent a linear or branched $C_{1-6}$-alkyl group, a linear or branched $C_{1-6}$-alkoxy group, a halogen atom, $CH_2F$, $CHF_2$, $CF_3$, CN, OH, $NO_2$, SH, $NH_2$, hydrogen, methyl, ethyl, F, Cl, Br and $CF_3$, and 0.05% to 0.5% by weight of the active principle of a surfactant and optionally one or more additional pharmaceutical excipients.

**4.** A pharmaceutical composition according to claims 1 to 3 which comprises:

0.5% to 40% by weight of compound of formula (I) , (II) or (III)
0.05% to 0.5% by weight of sodium alkylsulfate, and
2.5% to 10% by weight of a disintegrant.

**5.** A pharmaceutical composition according to claims 1 to 3 which contains:

0.5% to 40% by weight of compound of formula (I), (II) or (III)
0.1 % to 0.4% by weight of sodium laurylsulfate,
5% to 8% by weight of crosslinked sodium carboxymethyl cellulose,
1% to 10% by weight of binder,
0.2% to 5% by weight of lubricant,
and a diluent in a sufficient amount for 100%.

**6.** A pharmaceutical composition according to any one of claims 1 to 5 wherein the surfactant is added to the purified water in the process.

**7.** A pharmaceutical composition according to any one of claims 1 to 6 in the form of capsules, pellets, granules, tablets, sachets, powders, caplets, gels, multiparticulates such as granules, pellets, multipaticles, and the like, optionally compressed into tablets or filled into capsules.

**8.** A pharmaceutical composition according to claims 1 to 7 in the form of a gelatin capsule and having the following formulation, expressed in percentages by weight:

*Internal phase*
compound of formula (I), (II) or (III)
corn starch 30%
200 mesh lactose monohydrate 60.78%
povidone K 30 2.53%
crosslinked sodium carboxymethyl cellulose 5%
*Granulation*
sodium laurylsulfate 0.1 %
water QS
*External phase*
magnesium stearate 1 %

**9.** A pharmaceutical composition according to claims 1 to 7 in the form of a gelatin capsule and having the following formulation, expressed in percentages by weight:

*Internal phase*
compound of formula (I), (II), (III)
corn starch 30%
200 mesh lactose monohydrate 55.49%
povidone K 30 2.53%
crosslinked sodium carboxymethyl cellulose 5%
*Granulation*
sodium laurylsulfate 0.1%
water QS
*External phase*
magnesium stearate 1%

**10.** A pharmaceutical composition according to claim 1 to 7 in the form of a gelatin capsule and having the following formulation, expressed in percentages by weight:

*Internal phase*
compound of formula (I), (II) or (III)
corn starch 30%
200 mesh lactose monohydrate 43.73%
povidone K 30 2.53%
crosslinked sodium carboxymethyl cellulose 5%
*Granulation*
sodium laurylsulfate 0.1%
water QS
*External phase*
magnesium stearate 1%

**11.** A process for the preparation of a pharmaceutical composition according to any one of claims 1 to 10, **characterised in that**:

a) the active principle and the surfactant are mixed at room temperature optionally with a diluent, a binder and/or a coloring agent;
b) the mixture is wetted with purified water;
c) the resulting wet mass is dried and graded; and optionally
d) a lubricant and, an antiadhesive, a flowing agent, a coloring agent and/or a flavoring agent are added to the resulting graded dry grains.

**12.** A process according to claim 11 for the preparation of a pharmaceutical composition according to any one of claims 1 to 10, **characterised in that** the surfactant is incorporated in step b) instead of in step a).

**13.** A controlled release formulation comprising (1) at least one of the drugs defined in claims 1 to 3, (2) an additive which insures a penetration of water into the core of the preparation and (3) a controlled release polymer.

**14.** A controlled release formulation according to claim 13 wherein the controlled release polymer is a sustained release polymer.

**15.** A controlled release formulation according to claim 14 wherein the sustained release polymer is based on a soluble polymer.

**16.** A controlled release formulation according to claim 14 wherein the sustained release polymer is based on an insoluble polymer.

**17.** A controlled release formulation according to claim 15 or 16 wherein the sustained release polymer is based on a matrix-forming polymer.

**18.** A controlled release formulation according to claim 16 wherein the insoluble polymer is based on alkylcellulose, preferably ethylcellulose.

**19.** A controlled release formulation according to claim 15 wherein the soluble polymer is based on a cellulose derivative.

**20.** A controlled release formulation according to claim 19 wherein the cellulose derivative is hydroxypropylmethylcellulose or hydroxypropylcellulose.

**21.** A controlled release formulation according to claim 17 wherein the matrix-forming polymer is based on a hydrogel-forming polymer.

**22.** A hydrogel-type sustained-release preparation comprising (1) at least one of the drugs defined in claims 1 to 3, (2) at least one additive which insures a penetration of water into the core of the preparation and (3) at least one hydrogel forming polymer.

**23.** A hydrogel-type sustained-release preparation according to claim 22 comprising (1) at least one of the drugs defined in claims 1 to 3, (2) at least one additive which insures a penetration of water into the core of the preparation and (3) at least one hydrogel forming polymer, wherein said preparation is capable of undergoing substantially complete gelation during its stay in the upper digestive tract including stomach and small intestine and is capable of releasing the drug in the lower digestive tract including colon.

**24.** The hydrogel-type sustained-release preparation according to claim 23, wherein said additive which insures a penetration of water into the core of the preparation is at least one additive having a solubility that the volume of water required for dissolving 1 gram of said additive is not more than 5 ml.

**25.** The hydrogel-type sustained-release preparation according to claim 24, wherein said additive which insures a penetration of water into the core of the preparation is at least one additive having a solubility that the volume of water required for dissolving 1 gram of said additive is not more than 4 ml.

**26.** The hydrogel-type sustained-release preparation according to claim 22, wherein said hydrogel-forming polymer is either a polymer having an average molecular weight of not less than 2,000,000 or a polymer having a viscosity of not less than 1000 cps as measured at 1% concentration in water at 25 DEG C, or a mixture of two or more of these polymers.

**27.** The hydrogel-type sustained-release preparation according to claim 22, wherein said hydrogel-forming polymer includes at least one polyethylene oxide.

**28.** The hydrogel-type sustained-release preparation according to any one of claims 22 to 27, which comprises (1) at least one drug in an amount of not more than 85% by weight based on the total preparation, (2) at least one additive which insures a penetration of water into the core of the preparation in an amount of from 5 to 80% by weight based on the total preparation, and (3) at least one hydrogel-forming polymer in an amount of from 10 to 95% by weight based on the total preparation.

**29.** The hydrogel-type sustained-release preparation according to any one of claims 22 to 28, which comprises (1) at least one drug in an amount of not more than 80% by weight based on the total preparation, (2) at least one additive which insures a penetration of water into the core of the preparation in an amount of from 5 to 60% by weight based

on the total preparation, and (3) at least one hydrogel-forming polymer in an amount of from 15 to 90% by weight based on the total preparation.

**30.** A controlled release formulation according to claim 13 wherein the release of the active principle is controlled by pH.

**31.** A controlled release formulation according to claim 30 wherein the release of the active substance is controlled by a polymer whose dissolution is pH dependent.

**32.** A controlled release formulation according to claim 31 wherein the polymer is a gastric resistant polymer such as a co-polymerized methacrylic acid / methacrylic acid methyl esters.

**33.** An oral pharmaceutical preparation containing a nucleus formed by an inert core, a compound of formula (I), (II), or (III) as defined in claims 1 to 3, or a mixture of at least two of said compounds, an inert water soluble polymer and optionally pharmaceutical acceptable excipients;

**34.** A preparation according to claim 33 wherein the water soluble polymer comprises hydroxypropylmethylcellulose or hydroxypropylcellulose.

**35.** A preparation according to claim 33 which is enteric coated.

**36.** An oral pharmaceutical preparation which comprises:

(a) an inert core,
(b) a first layer containing a compound of formula (I), (II), or (III) as defined in claims 1 to 3, or a mixture thereof, and optionally an inert water soluble polymer and optionally one or more pharmaceutical acceptable excipients;
(c) a second layer containing a compound of formula (I), (II), or (III) as defined in claims 1 to 3, or a mixture thereof, and optionally an inert water soluble polymer and optionally one or more pharmaceutical acceptable excipients;

**37.** A formulation according to claim 36 wherein the release of the active substance is controlled.

**38.** A formulation according to claim 37 wherein the controlled release is pH-dependent.

**39.** A formulation according to claim 38 wherein the pH-dependent controlled release is insured by an enteric polymer.

**40.** A formulation according to claim 39 wherein the enteric polymer is a gastric resistant polymer such as co-polymerized methacrylic acid / methacrylic acid methyl esters.

**41.** A formulation according to claim 37 wherein the controlled release formulation is a sustained release formulation.

**42.** A pharmaceutical composition for oral administration of a compound of formula (I), (II) or (III) as defined in claims 1 to 3, or a mixture thereof, for adminsitration in form of caplets, gels, multiparticulates such as granules, pellets, multipaticles, and the like, optionally compressed into tablets or filled into capsules.

Figure 1

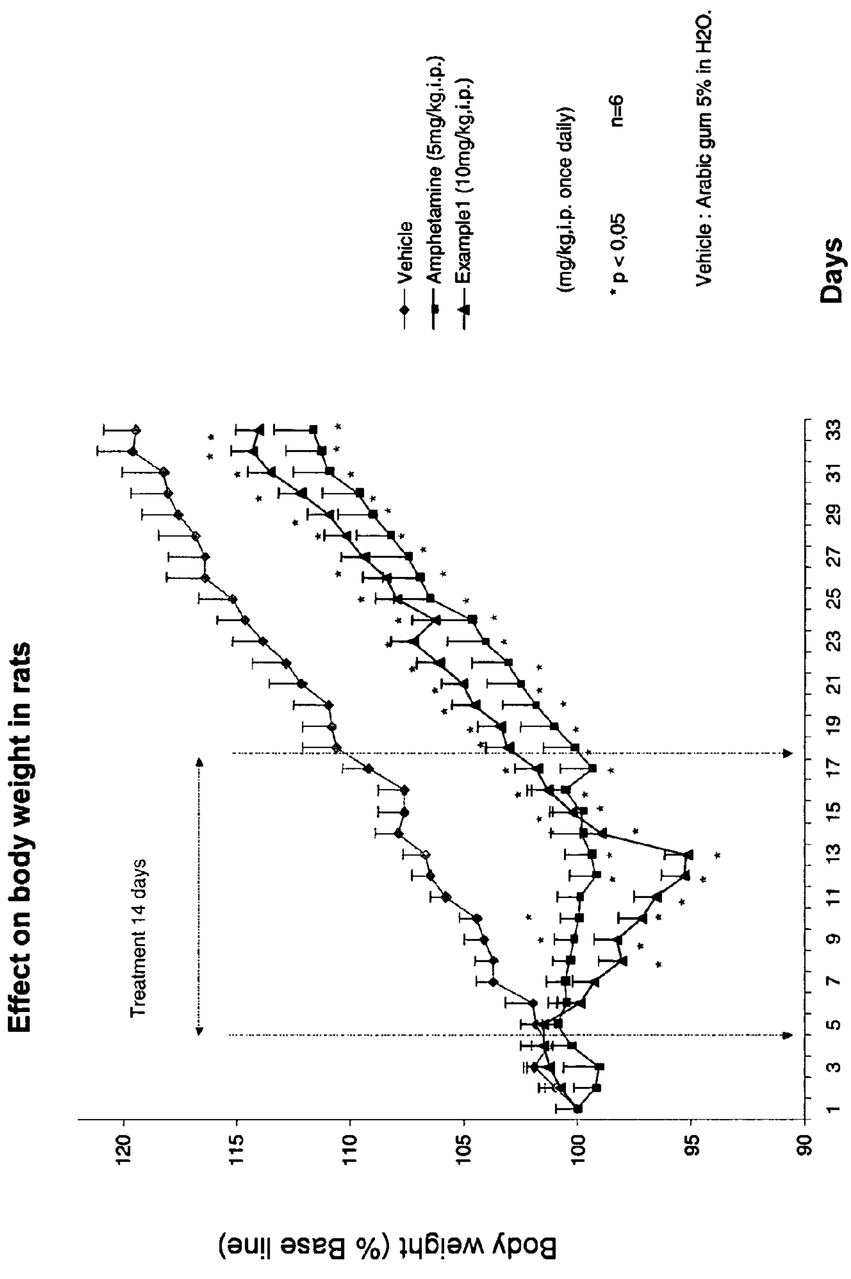
Effect on body weight in rats
Treatment 14 days
Body weight (% Base line)
120
115
110
105
100
95
90
1 3 5 7 9 11 13 15 17 19 21 23 25 27 29 31 33
Days
Vehicle
Amphetamine (5mg/kg,i.p.)
Example1 (10mg/kg,i.p.)
(mg/kg,i.p. once daily)
* p < 0,05
n=6
Vehicle : Arabic gum 5% in H2O.

Figure 2:

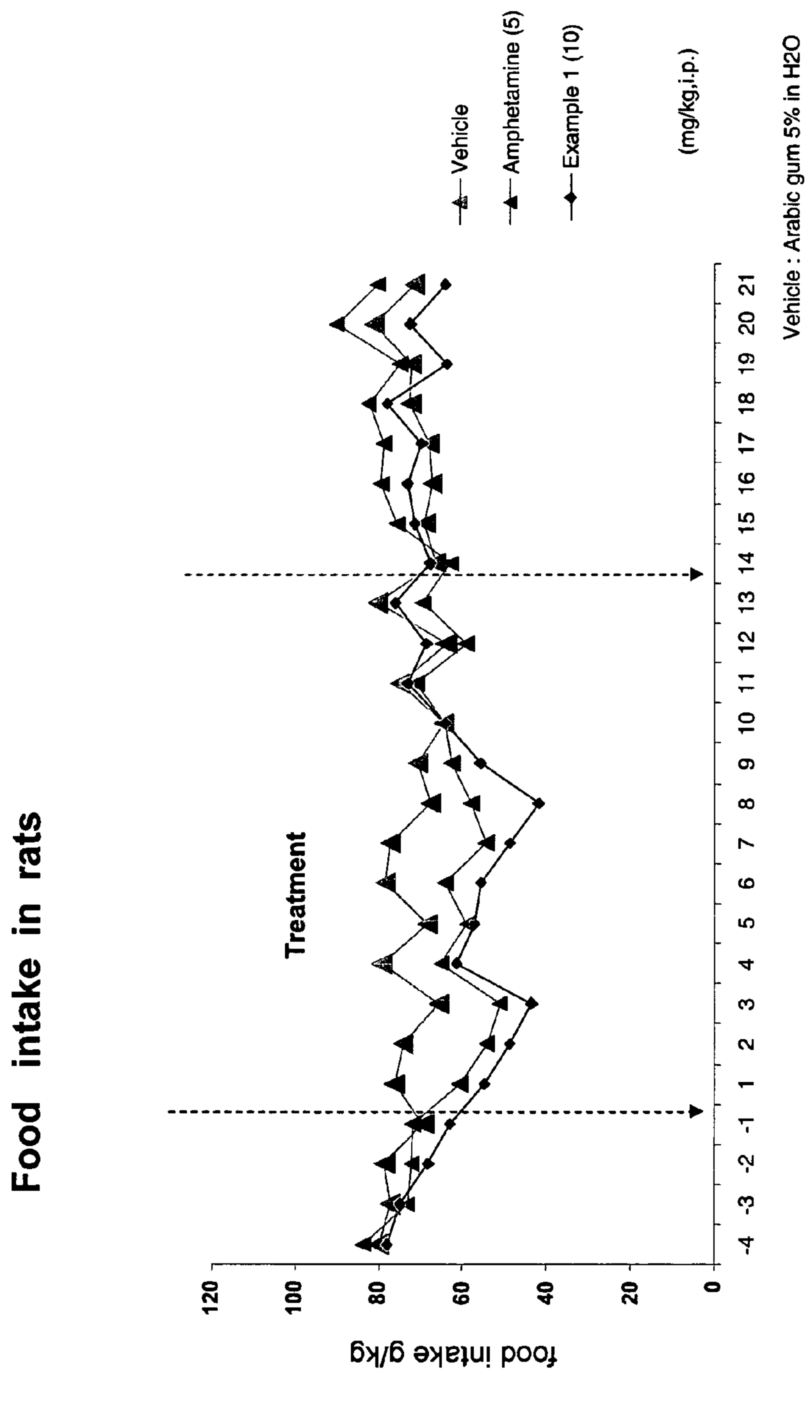
Food intake in rats
Treatment
food intake g/kg
120
100
80
60
40
20
0
-4 -3 -2 -1 1 2 3 4 5 6 7 8 9 10 11 12 13 14 15 16 17 18 19 20 21
Days
Vehicle
Amphetamine (5)
Example 1 (10)
(mg/kg,i.p.)
Vehicle : Arabic gum 5% in H2O

European Patent Office

# EUROPEAN SEARCH REPORT

Application Number
EP 05 38 4028

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2003/003145 A1 (ABRAMOVICI BERNARD ET AL) 2 January 2003 (2003-01-02)<br>* examples 1-7 *<br>----- | 1-42 | A61K31/4155<br>A61K31/496<br>A61K31/454 |
| E | WO 2005/074920 A (SOLVAY PHARMACEUTICALS B.V; LANGE, JOSEPHUS H.M; KRUSE, CORNELIS G; VA) 18 August 2005 (2005-08-18)<br>see compounds<br>* page 13, line 23 - page 14, line 10; claim 8 *<br>* page 5, line 40 - page 6, line 26 *<br>* example 2 *<br>----- | 1-42 | |
| A | FOR THE RIO-EUROPE STUDY GROUP VAN GAAL L F ET AL: "Effects of the cannabinoid-1 receptor blocker rimonabant on weight reduction and cardiovascular risk factors in overweight patients: 1-year experience from the RIO-Europe study"<br>LANCET THE, LANCET LIMITED. LONDON, GB, vol. 365, no. 9468,<br>16 April 2005 (2005-04-16), pages 1389-1397, XP004849986<br>ISSN: 0140-6736<br>* figure 2 *<br>----- | 1-42 | TECHNICAL FIELDS SEARCHED (IPC)<br>A61K<br>A61P |

The present search report has been drawn up for all claims

| Place of search | Date of completion of the search | Examiner |
|---|---|---|
| Munich | 4 November 2005 | Loher, F |

2

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO FORM 1503 03.82 (P04C01)

## ANNEX TO THE EUROPEAN SEARCH REPORT ON EUROPEAN PATENT APPLICATION NO. EP 05 38 4028

This annex lists the patent family members relating to the patent documents cited in the above-mentioned European search report.
The members are as contained in the European Patent Office EDP file on
The European Patent Office is in no way liable for these particulars which are merely given for the purpose of information.

04-11-2005

| Patent document cited in search report | Publication date | Patent family member(s) | Publication date |
|---|---|---|---|
| US 2003003145 A1 | 02-01-2003 | NONE | |
| WO 2005074920 A | 18-08-2005 | NONE | |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Non-patent literature cited in the description


- **HOLLISTER.** *Pharm. Rev.,* 1986, vol. 38, 1-20
- **RENY ; SINGHA.** *Prog. Drug. Res.,* 1991, vol. 36, 71-114
- **CONSROE ; SANDYK.** Marijuana/Cannabinoids, Neurobiology and Neurophysiology. CRC Press, 1992, vol. 459
- *Synthetic communications,* 1996, vol. 26 (11), 2229-33
- *Synlett,* 2001, 147-149
- **PASCUAL, A.** *J. Prakt Chem.,* 1999, vol. 341 (7), 695-700
- **LIN, S. et al.** *Heterocycles,* 2001, vol. 55 (2), 265-277
- **RAO, P. et al.** *J. Org. Chem.,* 2000, vol. 65 (22), 7323-7344
- **PEARSON D.E ; BUEHLER, C.A.** *Synthesis,* 1972, 533-542
- **KROGSGAARD-LARSEN et al.** Textbook of Drugdesign and Discovery. Taylor & Francis, April 2002
- *Br. J. clin. Pharmac,* 1988, vol. 26, 435-443
- *British Journal of Pharmacology,* 1999, vol. 126, 665-672
- **A. C. HOWLETT et al.** International Union of Pharmacology XXVII. *Classification of Cannabinoid Receptors, Pharmacol Rev,* 2002, vol. 54, 161-202
- **DAVID R. COMPTON et al.** In-vivo Characterization of a Specific Cannabinoid Receptor Antagonist (SR141716A) :Inhibition of Tetrahydrocannbinol- induced Responses and Apparent Agonist Activity. *J. Pharmacol. Exp. Ther.,* 1996, vol. 277 (2), 586-594
- **WOOLFE D. et al.** The evaluation of analgesic action of pethidine hydrochloride (Demerol. *J. Pharmacol. Exp. Ther.,* 1944, vol. 80, 300-307
- **DESMET L. K. C. et al.** Anticonvulsive properties of Cinarizine and Flunarizine in Rats and Mice. *Arzneim. -Forsch. (Frug Res,* 1975, vol. 25, 9
- **DAVID R. COMPTON et al.** In-vivo Characterization of a Specific Cannabinoid Receptor Antagonist (SR141716A) Inhibition of Tetrahydrocannbinol- induced Responses and Apparent Agonist Activity. *J. Pharmacol Exp Ther.,* 1996, vol. 277 (2), 586-594
- **ALPERMANN H. G. et al.** Pharmacological effets of Hoe 249: A new potential antidepressant. *Drugs Dev. Res.,* 1992, vol. 25, 267-282
- **G. COLOMBO et al.** Appetite Suppression and Weight Loss after the Cannabinoid Antagonist SR 141716. *Life Sciences,* 1998, vol. 63 (8), 113-117
- **E.T. TZAVARA et al.** The CB1 receptor antagonist SR141716A selectively increases monoaminergic neurotransmission in the medial prefrontal cortex: implications for therapeutic actions. *Br. J. Pharmacol.,* 2003, vol. 138 (4), 544-553